(19) Europäisches Patentamt
European Patent Office
Office européen des brevets

(11) EP 4 768 477 A1

(12) **EUROPEAN PATENT APPLICATION**
published in accordance with Art. 153(4) EPC

(43) Date of publication:
**01.07.2026 Bulletin 2026/27**

(21) Application number: **25736432.3**

(22) Date of filing: **06.01.2025**

(51) International Patent Classification (IPC):
***C07D 401/04*** *(2006.01)* ***A61K 31/454*** *(2006.01)*
***A61P 25/04*** *(2006.01)*

(52) Cooperative Patent Classification (CPC):
**A61K 31/454; A61P 25/04; C07D 401/04**

(86) International application number:
**PCT/CN2025/070679**

(87) International publication number:
**WO 2025/146193 (10.07.2025 Gazette 2025/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.01.2024 CN 202410019096**

(71) Applicant: **Yichang Humanwell Pharmaceutical Co., Ltd.**
**Yichang, Hubei 443005 (CN)**

(72) Inventors:
- **LI, Lie**
  **Yichang, Hubei 443005 (CN)**
- **SHENG, Xijun**
  **Yichang, Hubei 443005 (CN)**
- **TIAN, Luanyuan**
  **Yichang, Hubei 443005 (CN)**
- **CHENG, Peng**
  **Yichang, Hubei 443005 (CN)**
- **DENG, Yanfang**
  **Yichang, Hubei 443005 (CN)**
- **LIU, Zewen**
  **Yichang, Hubei 443005 (CN)**
- **LIANG, Dali**
  **Yichang, Hubei 443005 (CN)**
- **PAN, Xinyu**
  **Yichang, Hubei 443005 (CN)**
- **ZHU, Lijuan**
  **Yichang, Hubei 443005 (CN)**
- **SUN, Jun**
  **Yichang, Hubei 443005 (CN)**
- **SHU, Xuelian**
  **Yichang, Hubei 443005 (CN)**
- **YANG, Xiaoqing**
  **Yichang, Hubei 443005 (CN)**

(74) Representative: **Arnold & Siedsma**
**Bezuidenhoutseweg 57**
**2594 AC The Hague (NL)**

# (54) CYCLOHEXYL-SUBSTITUTED PIPERIDINE DERIVATIVE, PHARMACEUTICAL COMPOSITION COMPRISING SAME, AND USE THEREOF

(57) Disclosed in the present invention are a cyclohexyl-substituted piperidine derivative, a pharmaceutical composition comprising same, and a use thereof. The cyclohexyl-substituted piperidine derivative is a compound as represented by general formula (I), a stereoisomer thereof or a pharmaceutically acceptable salt thereof. The definition of substituents in general formula (I) is stated in detail in the description. The compound and the composition of the present invention can be used for treating a variety of diseases or disorders associated with an NOP receptor.

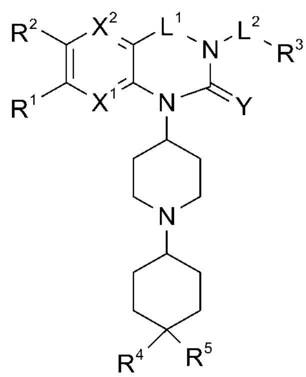

(I)

EP 4 768 477 A1

## Description

The present application claims priority to Chinese patent application filed on January 5, 2024, with Application No. CN202410019096X and invention title "Cyclohexyl-substituted Piperidine Derivative, Pharmaceutical Composition Comprising Same, and Use Thereof", the contents of which should be understood to be incorporated herein by reference.

Technical Field

The present application relates to, but is not limited to, the technical field of medical technology, in particular to a cyclohexyl-substituted piperidine derivative, a pharmaceutical composition comprising the same and use thereof.

Background

Pain, which is a complex physiological and psychological activity, is one of the most common clinical symptoms, posing a non-negligible impact on residents' health and quality of life. The pathogenesis of pain is diverse, and the pain accompanies almost all diseases. There is a huge demand for the analgesic drugs. At present, the most widely used analgesic drugs are Non-Steroidal Anti-Inflammatory Drugs (NSAIDs) and opioids, which are extensively applied in fields such as postoperative analgesia, pain relief in advanced cancer, and long-term chronic pain. Non-Steroidal Anti-Inflammatory Drugs have anti-inflammatory and analgesic effects, can relieve mild to moderate pain, but have adverse effects such as gastrointestinal irritation, liver and kidney function damage, neurological discomfort. Opioid analgesics, owing to their rapid onset and potent analgesic efficacy, serve as the primary therapeutic medicine for moderate to severe pain in clinical practice. However, their utility is limited by side effects such as addiction, respiratory depression, constipation, tolerance and pruritus, and these side effects also cause social problems such as drug dependence and fatalities resulting from abuse. The research and development of a safe and effective analgesic drug devoid of adverse effects represents an urgent social need, and at the current stage, research on opioid receptor agonists primarily focuses on eliminating or mitigating opioid-like side effects.

The NOP (Nociceptin/orphanin FQ peptide, Nociceptin/orphanin FQ) receptor, also known as ORL-1 (opioid receptor-like receptor 1) discovered in 1994, is the fourth class of opioid receptor, following $\mu$, $\kappa$ and $\delta$, and the NOP receptor shares approximately 60% homology with other opioid receptors. The NOP receptor does not bind to classical opioid ligands (such as naloxone), nor do their endogenous ligand orphanin FQ (Nociceptin/orphanin FQ, N/O FQ) bind to other opioid receptors, NOP-mediated analgesia has fewer adverse effects as compared to other opioid receptors. The nociceptin/orphanin FQ-NOP receptor system can not only inhibit opioid receptor-mediated analgesia effects, but also mediate analgesic effects by reducing hyperalgesia. Studies have shown that NOP receptor agonists have analgesic effect and effects of modulating the addiction induced by MOP agonists. In non-human primate experiments, it was found that selective NOP agonists do not cause respiratory depression and potential addiction (ACS Chem Neurosci., 2013, 4(2), 214-224). In addition, preclinical research results suggest that NOP ligands may have potential for pain relief, anxiolysis, antidepressant effect, treatment of drug addiction, and being as diuretics.

Peptide NOP receptor ligands have failed to advance in clinical trials due to poor pharmacokinetics and adverse effects, and non-peptide small molecule NOP receptor ligands may be more suitable as therapeutic agents. Ro 64-6198 is the first reported small molecule NOP receptor selective agonist, developed by Roche, and it has shown good analgesic effects in both non-human primate models and rodent model experiments without exhibiting the side effects mediated by the classic opioids. Mitsubishi Tanabe Pharmaceutical disclosed a class of benzimidazole compounds in WO2003082333A1, which are selective NOP receptor (ORL-1 receptor) agonists, among them, MT-7716 can dose-dependently reduce voluntary alcohol intake in rats, it has entered Phase I clinical trials for the treatment of alcohol dependence. Schering-Plough disclosed a class of piperidinyl derivative compounds as NOP receptor agonists for the treatment of cough in WO2001007050A1.

Previous reports show that NOP agonists exhibit their unique pharmacological effects, providing good analgesic effects while effectively reduce the side effects of the traditional opioids like morphine, such as respiratory depression, constipation, tolerance, addiction, etc. Drugs that selectively agonized NOP are expected to overcome the adverse effects of traditional opioid analgesics, however, current research on NOP receptor agonists is in its early stage, no NOP agonists have been approved for market as drugs yet. Therefore, the research on highly selective, safe and effective NOP agonists have important clinical significance and social significance.

Summary

The present invention provides a cyclohexyl-substituted piperidine derivative and a pharmaceutical composition comprising the compound and use thereof, which have better agonistic activity and selectivity for NOP receptors, and have better pharmacodynamics and/or pharmacokinetic properties, good safety, and can be used to treat or prevent diseases

related to NOP receptors.

In this regard, the present invention adopts the following technical solutions.

In one aspect, the present invention provides a compound having general formula (I), a stereoisomer thereof or a pharmaceutically acceptable salt thereof;

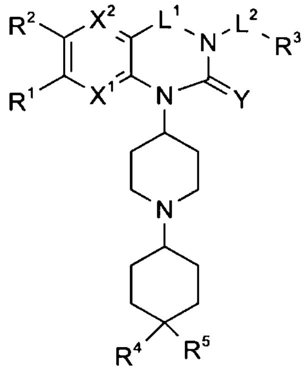

(I)

wherein,

$R^1$ and $R^2$ are each independently selected from hydrogen, halogen, $C_{1-6}$ alkyl, halogenated $C_{1-6}$ alkyl, $C_{1-6}$ alkoxy, halogenated $C_{1-6}$ alkoxy, and provided that: $R^1$ and $R^2$ are not simultaneously hydrogen;

$R^3$ is selected from $-C(O)NR^aR^b$, $-S(O)_2NR^aR^b$, $-S(O)(NR^a)R^b$, $-S(O)R^c$, $-S(O)_2R^c$, $-NHS(O)_2R^c$, $-NHC(O)R^d$, $-C(O)OR^d$, 5- to 10-membered heteroaryl, and said 5- to 10-membered heteroaryl is optionally substituted with one or more $R^e$;

$R^4$ and $R^5$ are each independently selected from hydrogen, halogen, $C_{1-6}$ alkyl, $C_{1-6}$ alkoxy; or $R^4$ and $R^5$ together with a carbon atom to which they are attached form $C_{3-8}$ cycloalkyl; or $R^4$ and $R^5$ together with a carbon atom to which they are attached form a 3- to 8-membered heterocyclyl, $R^4$ and $R^5$ can be the same or different, and provided that: $R^4$ and $R^5$ are not simultaneously hydrogen, wherein said $C_{1-6}$ alkyl, $C_{1-6}$ alkoxy, $C_{3-8}$ cycloalkyl and 3- to 8-membered heterocyclyl are each optionally substituted with one or more R';

$X^1$ and $X^2$ are each independently selected from N and $CR^f$;

$L^1$ is selected from a single bond and $-CR^gR^h-$;

$L^2$ is selected from $C_{1-6}$ alkylene group, and said $C_{1-6}$ alkylene group is optionally substituted with one or more $R^m$;

Y is selected from O and S;

$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', and $R^a$ and $R^b$ may be the same or different;

each $R^c$ is independently selected from $C_{1-6}$ alkyl, $C_{3-6}$ cycloalkyl, $C_{6-10}$ aryl, $-(CH_2)_n-C_{3-6}$ cycloalkane, $-(CH_2)_n-C_{6-10}$ aromatic hydrocarbon, wherein said $C_{1-6}$ alkyl, $C_{3-6}$ cycloalkyl, $C_{6-10}$ aryl, $-(CH_2)_n-C_{3-6}$ cycloalkane, $-(CH_2)_n-C_{6-10}$ aromatic hydrocarbon are each optionally substituted with one or more R';

each $R^d$ is independently selected from $C_{1-6}$ alkyl, $C_{3-6}$ cycloalkyl, $-(CH_2)_n-C_{3-6}$ cycloalkane, wherein said $C_{1-6}$ alkyl, $C_{3-6}$ cycloalkyl, $-(CH_2)_n-C_{3-6}$ cycloalkane are each optionally substituted with one or more R';

$R^e$, $R^f$ are each independently selected from hydrogen, halogen, $C_{1-3}$ alkyl, $-O-C_{1-3}$ alkane, wherein said $C_{1-3}$ alkyl, $-O-C_{1-3}$ alkane are each optionally substituted with one or more R';

$R^g$, $R^h$ and $R^m$ are each independently selected from hydrogen, halogen, $C_{1-3}$ alkyl, wherein said $C_{1-3}$ alkyl is optionally substituted with one or more R';

each R' is independently selected from H, F, Cl, Br, and I; and

n is an integer of 1, 2 or 3.

In one embodiment, $R^1$ and $R^2$ are each independently selected from hydrogen, halogen, $C_{1-3}$ alkyl, halogenated $C_{1-3}$ alkyl, $C_{1-3}$ alkoxy, halogenated $C_{1-3}$ alkoxy, and provided that: $R^1$ and $R^2$ are not simultaneously hydrogen. In another embodiment, $R^1$ and $R^2$ are each independently selected from hydrogen, halogen, $C_{1-3}$ alkyl, and provided that: $R^1$ and $R^2$ are not simultaneously hydrogen. In another embodiment, $R^1$ and $R^2$ are each independently halogen. In another embodiment, $R^1$ and $R^2$ are both fluorine or chlorine, or $R^1$ is chlorine and $R^2$ is fluorine; or $R^1$ is fluorine and $R^2$ is chlorine; or $R^1$ is chlorine and $R^2$ is methoxyl.

In one embodiment, $R^3$ is selected from $-C(O)NR^aR^b$, $-S(O)_2NR^aR^b$, $-S(O)(NR^a)R^b$, $-S(O)R^c$, $-S(O)_2R^c$,

$-NHS(O)_2R^c$, $-NHC(O)R^d$, $-C(O)OR^d$, and 5- to 6-membered heteroaryl, and said 5- to 6-membered heteroaryl is optionally substituted with one or more $R^e$; or $R^3$ is $-C(O)NR^aR^b$; or $R^3$ is $-S(O)_2NR^aR^b$; or $R^3$ is $-S(O)(NR^a)R^b$; or $R^3$ is $-S(O)R^c$; or $R^3$ is $-S(O)_2R^c$; or $R^3$ is $-NHS(O)_2R^c$; or $R^3$ is $-NHC(O)R^d$; or $R^3$ is-$C(O)OR^d$; or $R^3$ is 5- to 6-membered heteroaryl, and said 5- to 6-membered heteroaryl is optionally substituted with one or more $R^e$.

In another embodiment, $R^3$ is selected from $-C(O)N(CH_3)_2$, $-C(O)NH_2$, $-C(O)NHOH$, $-S(O)_2NH_2$, $-S(O)_2N(CH_3)_2$, $-S(O)(NH)CH_3$, $-S(O)CH_3$, $-S(O)CH_2CH_3$, $-S(O)_2CH_3$, $-S(O)_2CH_2CH_3$, $-C(O)NHCH_2CH_2OH$, $-C(O)NHCH_3$, $-C(O)NHCH_2CH_3$, $-NHC(O)CH_3$, $-S(O)_2CH(CH_3)_2$, $-NHC(O)CH_2CH_3$, $-C(O)OCH_2CH_3$, $-S(O)_2$-Ph, $-S(O)_2CH_2$-Ph,

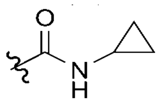
,

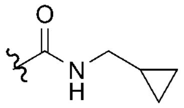
,
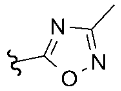
,

$-NHS(O)_2CH_3$, $-NHS(O)_2CH_2CH_3$; or, $R^3$ is selected from $C(O)NHCH_3$, $-S(O)_2N(CH_3)_2$, $-S(O)CH_3$, $-S(O)_2CH_3$, $-S(O)_2CH_2CH_3$, $-NHS(O)_2CH_3$, $-NHS(O)_2CH_2CH_3$, $-NHC(O)CH_3$, $-NHC(O)CH_2CH_3$.

In one embodiment, $R^4$ and $R^5$ are each independently selected from hydrogen, halogen, $C_{1-6}$ alkyl, $C_{1-6}$ alkoxy; or $R^4$ and $R^5$ together with the carbon atom to which they are attached form $C_{3-6}$ cycloalkyl; or $R^4$ and $R^5$ together with the carbon atom to which they are attached form a 3- to 6-membered heterocyclyl, $R^4$ and $R^5$ can be the same or different, and provided that: $R^4$ and $R^5$ are not simultaneously hydrogen, wherein said $C_{1-6}$ alkyl, $C_{1-6}$ alkoxy, $C_{3-6}$ cycloalkyl and 3- to 6-membered heterocyclyl are each optionally substituted with one or more R'. In another embodiment, $R^4$ is hydrogen, $R^5$ is selected from halogen, $C_{1-6}$ alkyl, $C_{1-6}$ alkoxy, wherein said $C_{1-6}$ alkyl, $C_{1-6}$ alkoxy are each optionally substituted with one or more R'. In another embodiment, $R^5$ is hydrogen, $R^4$ is selected from halogen, $C_{1-6}$ alkyl and $C_{1-6}$ alkoxy, wherein said $C_{1-6}$ alkyl, $C_{1-6}$ alkoxy are each optionally substituted with one or more R'. In another embodiment, $R^4$ and $R^5$ are each independently halogen. In another embodiment, $R^4$ and $R^5$ are each independently $C_{1-6}$ alkyl, wherein said $C_{1-6}$ alkyl is optionally substituted with one or more R'. In another embodiment, $R^4$, $R^5$ are each independently $C_{1-6}$ alkoxy, wherein said $C_{1-6}$ alkoxy is each optionally substituted with one or more R'. In another embodiment, $R^4$ and $R^5$ together with the carbon atom to which they are attached form $C_{3-6}$ cycloalkyl, wherein said $C_{3-6}$ cycloalkyl is optionally substituted with one or more R'. In another embodiment, $R^4$ and $R^5$ together with the carbon atom to which they are attached form a 3- to 6-membered heterocyclyl, wherein said 3- to 6-membered heterocyclyl is optionally substituted with one or more R'.

In another embodiment, $R^4$ and $R^5$ are each independently selected from hydrogen, halogen, $C_{1-6}$ alkyl, or $R^4$ and $R^5$ together with the carbon atom to which they are attached form $C_{3-6}$ cycloalkyl, or $R^4$ and $R^5$ together with the carbon atom to which they are attached form a 3-to 6-membered heterocyclyl, $R^4$ and $R^5$ can be the same or different, and provided that $R^4$ and $R^5$ are not simultaneously hydrogen. In another embodiment, $R^4$ and $R^5$ are each independently selected from hydrogen, halogen, $C_{1-3}$ alkyl, or $R^4$ and $R^5$ together with the carbon atom to which they are attached form $C_{3-4}$ cycloalkyl, or $R^4$ and $R^5$ together with the carbon atom to which they are attached form a 3-4 membered heterocyclyl, $R^4$ and $R^5$ can be the same or different, and provided that $R^4$ and $R^5$ are not simultaneously hydrogen. In another embodiment, both $R^4$ and $R^5$ are methyl, or one of $R^4$ and $R^5$ is isopropyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl.

In one embodiment, $X^1$ and $X^2$ are each independently selected from N and CH. In another embodiment, both of $X^1$ and $X^2$ are N. In another embodiment, $X^1$ is N, $X^2$ is CH. In another embodiment, $X^2$ is N, $X^1$ is CH. In another embodiment, both of $X^1$ and $X^2$ are CH.

In one embodiment, $L^1$ is selected from a single bond and $-CR^gR^h-$. In another embodiment, $L^1$ is selected from a single bond. In another embodiment, $L^1$ is selected from $-CR^gR^h-$. In another embodiment, $L^1$ is selected from $-CH_2-$.

In another embodiment, $L^1$ is selected from a single bond and $-CH_2-$.

In one embodiment, $L^2$ is selected from $C_{1-3}$ alkylene group, and said $C_{1-3}$ alkylene group is optionally substituted with one or more $R^m$. In another embodiment, $L^2$ is selected from $-CH_2-$, $-CH_2CH_2-$, and $-CH(CH_3)-$.

In one embodiment, Y is selected from O and S. In another embodiment, Y is O. In another embodiment, Y is S.

In one embodiment, $R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane and $-(CH_2)_n-OH$, wherein said $C_{1-6}$ alkyl, $C_{3-6}$ cycloalkyl, $-(CH_2)_n-C_{3-6}$ cycloalkane and $-(CH_2)_n-OH$ are each optionally substituted with one or more R', $R^a$ and $R^b$ may be the same or different. In another embodiment, one of $R^a$ and $R^b$ is selected from hydrogen and the other is selected from hydroxyl, $C_{1-3}$ alkyl, $C_{3-6}$ cycloalkyl, $-(CH_2)_n-C_{3-6}$ cycloalkane, $-(CH_2)_n-OH$, wherein said $C_{1-3}$ alkyl, $C_{3-6}$ cycloalkyl, $-(CH_2)_n-C_{3-6}$ cycloalkane,

$-(CH_2)_n$-OH are each optionally substituted with one or more R'. In another embodiment, one of $R^a$ and $R^b$ is selected from hydrogen and the other is selected from methyl, ethyl, cyclopropyl, cyclopropylmethyl, hydroxyl and hydroxyethyl. In another embodiment, both of $R^a$ and $R^b$ are $C_{1-3}$ alkyl. In another embodiment, both of $R^a$ and $R^b$ are hydrogen.

In one embodiment, each $R^c$ is independently selected from $C_{1-3}$ alkyl, $C_{3-6}$ cycloalkyl, phenyl, $-(CH_2)_n-C_{3-6}$ cycloalkane, $-(CH_2)_n$-benzene, wherein said $C_{1-3}$ alkyl, $C_{3-6}$ cycloalkyl, phenyl, $-(CH_2)_n-C_{3-6}$ cycloalkane, $-(CH_2)_n$-benzene are each optionally substituted with one or more R'. In another embodiment, each $R^c$ is independently selected from methyl, ethyl, propyl, isopropyl, cyclopropyl, cyclopropylmethyl, benzyl and phenyl.

In one embodiment, each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'. In another embodiment, each $R^d$ is independently selected from methyl, ethyl, propyl, isopropyl, cyclopropyl and cyclopropylmethyl.

In one embodiment, $R^e$, $R^f$ are each independently selected from hydrogen, halogen, $C_{1-3}$ alkyl, $-O-C_{1-3}$ alkane, wherein said $C_{1-3}$ alkyl, $-O-C_{1-3}$ alkane are each optionally substituted with one or more R'. In another embodiment, $R^e$ and $R^f$ are each independently hydrogen. In another embodiment, $R^e$ and $R^f$ are each independently halogen. In another embodiment, $R^e$ and $R^f$ are each independently $C_{1-3}$ alkyl, and said $C_{1-3}$ alkyl is optionally substituted with one or more R'. In another embodiment, $R^e$ and $R^f$ are each independently $-O-C_{1-3}$ alkane, and said $-O-C_{1-3}$ alkane is optionally substituted with one or more R'.

In another embodiment, $R^e$ and $R^f$ are each independently selected from hydrogen, fluorine, chlorine, bromine, methyl, methoxyl, trifluoromethyl and trifluoromethoxyl.

In one embodiment, $R^g$, $R^h$ and $R^m$ are each independently selected from hydrogen, fluorine and methyl. In another embodiment, $R^g$, $R^h$ and $R^m$ are each independently hydrogen. In another embodiment, $R^g$, $R^h$ and $R^m$ are each independently fluorine. In another embodiment, $R^g$, $R^h$ and $R^m$ are each independently methyl.

In one embodiment, each R' is independently selected from H, F, Cl, and Br. In another embodiment, each R' is independently selected from H, F and Cl.

In one embodiment, n is an integer of 1, 2 or 3. In another embodiment, n is 1. In another embodiment, n is 2. In another embodiment, n is 3.

In a preferred embodiment of the present invention, said compound represented by general formula (I), a stereoisomer thereof or a pharmaceutically acceptable salt thereof, is further represented by general formula (II):

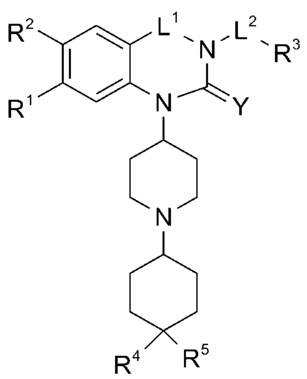

(II)

wherein:

$R^1$~ $R^5$, $L^1$, $L^2$, Y in general formula (II) are as defined hereinabove.

In a preferred embodiment of the present invention, in the general formula (II),

$R^1$ and $R^2$ are each independently selected from hydrogen, halogen, $C_{1-3}$ alkyl, and provided that: $R^1$ and $R^2$ are not simultaneously hydrogen; preferably, both of $R^1$ and $R^2$ are halogen, further preferably, $R^1$ and $R^2$ are both fluorine or chlorine, or $R^1$ is chlorine and $R^2$ is fluorine, or $R^1$ is fluorine and $R^2$ is chlorine; or, $R^1$ is chlorine and $R^2$ is methoxyl;

$R^3$ is selected from $-C(O)NR^aR^b$, $-S(O)_2NR^aR^b$, $-S(O)(NR^a)R^b$, $-S(O)R^c$, $-S(O)_2R^c$, $-NHS(O)_2R^c$, $-NHC(O)R^d$, $-C(O)OR^d$, and 5- to 6-membered heteroaryl, and said 5- to 6-membered heteroaryl is optionally substituted with one or more $R^e$; preferably, $R^3$ is selected from $-C(O)NR^aR^b$, $-S(O)_2NR^aR^b$, $-S(O)(NR^a)R^b$, $-S(O)R^c$, $-NHS(O)_2R^c$, $-S(O)_2R^c$;

$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are

attached form a 3- to 6-membered heterocyclyl, $R^4$ and $R^5$ may be the same or different, and provided that: $R^4$ and $R^5$ are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;

$L^1$ is selected from a single bond and $-CH_2-$;

$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;

Y is selected from O;

$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;

each $R^c$ is independently selected from $C_{1-3}$ alkyl, $C_{3-6}$ cycloalkyl, phenyl, $-(CH_2)_n-C_{3-6}$ cycloalkane, $-(CH_2)_n-$benzene, wherein said $C_{1-3}$ alkyl, $C_{3-6}$ cycloalkyl, phenyl, $-(CH_2)_n-C_{3-6}$ cycloalkane, $-(CH_2)_n-$benzene are each optionally substituted with one or more R'; preferably, each $R^c$ is independently selected from methyl, ethyl, propyl, isopropyl, cyclopropyl, cyclopropylmethyl, benzyl and phenyl, further preferably, each $R^c$ is independently selected from methyl and ethyl;

each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'; in another embodiment, each $R^d$ is independently selected from methyl, ethyl, propyl, isopropyl, cyclopropyl and cyclopropylmethyl;

$R^e$ is selected from hydrogen, fluorine, chlorine, bromine, methyl, methoxyl, trifluoromethyl and trifluoromethoxyl;

$R^m$ is selected from hydrogen, fluoro and methyl;

each R' is independently selected from H, F, Cl, and Br; preferably, each R' is independently selected from H, F and Cl; and

n is an integer of 1, 2 or 3, preferably, n is an integer of 1 or 2.

In a preferred embodiment of the present invention, said compound represented by general formula (I), a stereoisomer thereof or a pharmaceutically acceptable salt thereof, is further represented by general formula (III):

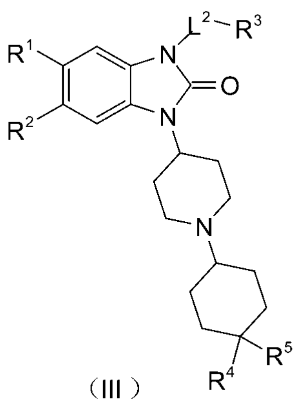

(III)

wherein:

$R^1$~ $R^5$, $L^2$ in general formula (III) are as defined hereinabove.

In a preferred embodiment of the present invention, in the general formula (III),

$R^1$ and $R^2$ are each independently selected from hydrogen, halogen, $C_{1-3}$ alkyl, and provided that: $R^1$ and $R^2$ are not simultaneously hydrogen; preferably, both of $R^1$ and $R^2$ are halogen, further preferably $R^1$ and $R^2$ are both fluoro or chloro, or $R^1$ is chloro and $R^2$ is fluoro, or $R^1$ is fluoro and $R^2$ is chloro; or, $R^1$ is chloro and $R^2$ is methoxyl;

$R^3$ is selected from $-C(O)NR^aR^b$, $-S(O)_2NR^aR^b$, $-S(O)(NR^a)R^b$, $-S(O)R^c$, $-S(O)_2R^c$, $-NHS(O)_2R^c$, $-NHC(O)R^d$, $-C(O)OR^d$, and 5- to 6-membered heteroaryl, said 5- to 6-membered heteroaryl is optionally substituted with one or more $R^e$; preferably, $R^3$ is selected from $-C(O)NR^aR^b$, $-S(O)_2NR^aR^b$, $-S(O)(NR^a)R^b$, $-S(O)R^c$, $-NHS(O)_2R^c$, $-S(O)_2R^c$;

$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are attached form a 3- to 6-membered heterocyclyl, $R^4$ and $R^5$ may be the same or different, and provided that: $R^4$ and $R^5$ are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;

$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;

$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;

each $R^c$ is independently selected from $C_{1-3}$ alkyl, $C_{3-6}$ cycloalkyl, phenyl, $-(CH_2)_n-C_{3-6}$ cycloalkane, $-(CH_2)_n$-benzene, wherein said $C_{1-3}$ alkyl, $C_{3-6}$ cycloalkyl, phenyl, $-(CH_2)_n-C_{3-6}$ cycloalkane, $-(CH_2)_n$-benzene are each optionally substituted with one or more R'; preferably, each $R^c$ is independently selected from methyl, ethyl, propyl, isopropyl, cyclopropyl, cyclopropylmethyl, benzyl, phenyl, further preferably, each $R^c$ is independently selected from methyl and ethyl;

each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'; in another embodiment, each $R^d$ is independently selected from methyl, ethyl, propyl, isopropyl, cyclopropyl and cyclopropylmethyl;

$R^e$ is selected from hydrogen, fluorine, chlorine, bromine, methyl, methoxyl, trifluoromethyl and trifluoromethoxyl;

$R^m$ is selected from hydrogen, fluoro and methyl;

each R' is independently selected from H, F, Cl, and Br; preferably, each R' is independently selected from H, F and Cl; and

n is an integer of 1, 2 or 3, preferably n is an integer of 1 or 2.

In a preferred embodiment of the present invention, said compound represented by general formula (I), a stereoisomer thereof or a pharmaceutically acceptable salt thereof, is further represented by general formula (IV):

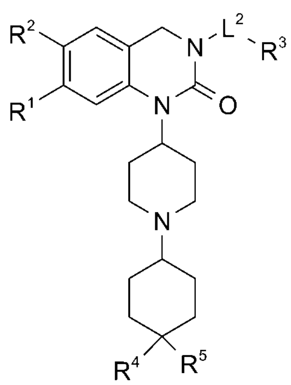

（IV）

wherein:

$R^1$~ $R^5$, $L^2$ in general formula (IV) are as defined hereinabove.

In a preferred embodiment of the present invention, in the general formula (IV),

$R^1$ and $R^2$ are each independently selected from hydrogen, halogen, $C_{1-3}$ alkyl, and provided that: $R^1$ and $R^2$ are not simultaneously hydrogen; preferably, both of $R^1$ and $R^2$ are halogen, further preferably $R^1$ and $R^2$ are both fluorine or chlorine, or $R^1$ is chlorine and $R^2$ is fluorine, or $R^1$ is fluorine and $R^2$ is chlorine; or $R^1$ is chlorine and $R^2$ is methoxyl;
$R^3$ is selected from $-C(O)NR^aR^b$, $-S(O)_2NR^aR^b$, $-S(O)(NR^a)R^b$, $-S(O)R^c$, $-S(O)_2R^c$, $-NHS(O)_2R^c$, $-NHC(O)R^d$, $-C(O)OR^d$, and 5- to 6-membered heteroaryl, said 5- to 6-membered heteroaryl is optionally substituted with one or more $R^e$; preferably, $R^3$ is selected from $-C(O)NR^aR^b$, $-S(O)_2NR^aR^b$, $-S(O)(NR^a)R^b$, $-S(O)R^c$, $-NHS(O)_2R^c$, $-S(O)_2R^c$;
$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are attached form a 3- to 6-membered heterocyclyl, $R^4$ and $R^5$ may be the same or different, and provided that: $R^4$ and $R^5$ are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;
$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;
$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;
each $R^c$ is independently selected from $C_{1-3}$ alkyl, $C_{3-6}$ cycloalkyl, phenyl, $-(CH_2)_n-C_{3-6}$ cycloalkane, $-(CH_2)_n$-benzene, wherein said $C_{1-3}$ alkyl, $C_{3-6}$ cycloalkyl, phenyl, $-(CH_2)_n-C_{3-6}$ cycloalkane, $-(CH_2)_n$-benzene are each optionally substituted with one or more R'; preferably, each $R^c$ is independently selected from methyl, ethyl, propyl, isopropyl, cyclopropyl, cyclopropylmethyl, benzyl and phenyl, further preferably, each $R^c$ is independently selected from methyl and ethyl;
each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'; in another embodiment, each $R^d$ is independently selected from methyl, ethyl, propyl, isopropyl, cyclopropyl and cyclopropylmethyl;
$R^e$ is selected from hydrogen, fluorine, chlorine, bromine, methyl, methoxyl, trifluoromethyl and trifluoromethoxyl;
$R^m$ is selected from hydrogen, fluorine and methyl;
each R' is independently selected from H, F, Cl, and Br; preferably, each R' is independently selected from H, F and Cl; and
n is an integer of 1, 2 or 3, preferably n is an integer of 1 or 2.

In a preferred embodiment of the present invention, said compound represented by general formula (I), a stereoisomer thereof or a pharmaceutically acceptable salt thereof, is further represented by general formula (V):

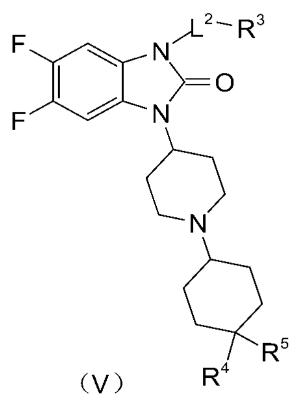

(V)

wherein:
$R^3$~ $R^5$, $L^2$ in general formula (V) are as defined hereinabove.

In a preferred embodiment of the present invention, in the general formula (V),

$R^3$ is selected from -C(O)NR$^a$R$^b$, -S(O)$_2$NR$^a$R$^b$, -S(O)(NR$^a$)R$^b$, -S(O)R$^c$, -S(O)$_2$R$^c$, -NHS(O)$_2$R$^c$, -NHC(O)R$^d$, -C(O)OR$^d$, and 5- to 6-membered heteroaryl, said 5- to 6-membered heteroaryl is optionally substituted with one or more R$^e$; preferably, $R^3$ is selected from -C(O)NR$^a$R$^b$, -S(O)$_2$NR$^a$R$^b$, -S(O)(NR$^a$)R$^b$, -S(O)R$^c$, -NHS(O)$_2$R$^c$, -S(O)$_2$R$^c$;
$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are attached form a 3- to 6-membered heterocyclyl, $R^4$ and $R^5$ may be the same or different, and provided that: $R^4$ and $R^5$ are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;
$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;
$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n$-OH, wherein said $C_{1-6}$ alkyl, $C_{3-6}$ cycloalkyl, $-(CH_2)_n-C_{3-6}$ cycloalkane, $-(CH_2)_n$-OH are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;
each $R^c$ is independently selected from $C_{1-3}$ alkyl, $C_{3-6}$ cycloalkyl, phenyl, $-(CH_2)_n-C_{3-6}$ cycloalkane, $-(CH_2)_n$-benzene, wherein said $C_{1-3}$ alkyl, $C_{3-6}$ cycloalkyl, phenyl, $-(CH_2)_n-C_{3-6}$ cycloalkane, $-(CH_2)_n$-benzene are each optionally substituted with one or more R'; preferably, each $R^c$ is independently selected from methyl, ethyl, propyl, isopropyl, cyclopropyl, cyclopropylmethyl, benzyl and phenyl, further preferably, each $R^c$ is independently selected from methyl and ethyl;
each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'; in another embodiment, each $R^d$ is independently selected from methyl, ethyl, propyl, isopropyl, cyclopropyl and cyclopropylmethyl;
$R^e$ is selected from hydrogen, fluorine, chlorine, bromine, methyl, methoxyl, trifluoromethyl and trifluoromethoxyl;
$R^m$ is selected from hydrogen, fluorine and methyl;
each R' is independently selected from H, F, Cl, and Br; preferably, each R' is independently selected from H, F and Cl; and
n is an integer of 1, 2 or 3, preferably n is an integer of 1 or 2.

In a preferred embodiment of the present invention, said compound represented by general formula (I), a stereoisomer thereof or a pharmaceutically acceptable salt thereof, is further represented by general formula (VI):

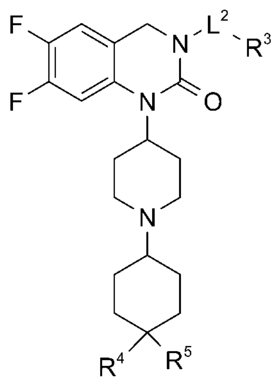

(VI)

wherein:
$R^3$~ $R^5$, $L^2$ in general formula (VI) are as defined hereinabove.
In a preferred embodiment of the present invention, in the general formula(VI),

$R^3$ is selected from -C(O)NR$^a$R$^b$, -S(O)$_2$NR$^a$R$^b$, -S(O)(NR$^a$)R$^b$, -S(O)R$^c$, -S(O)$_2$R$^c$, -NHS(O)$_2$R$^c$, -NHC(O)R$^d$, -C(O)

$OR^d$, and 5- to 6-membered heteroaryl, wherein said 5- to 6-membered heteroaryl is optionally substituted with one or more $R^e$; preferably, $R^3$ is selected from $-C(O)NR^aR^b$, $-S(O)_2NR^aR^b$, $-S(O)(NR^a)R^b$, $-S(O)R^c$, $-NHS(O)_2R^c$, $-S(O)_2R^c$;
$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are attached form a 3- to 6-membered heterocyclyl, $R^4$ and $R^5$ may be the same or different, and provided that: $R^4$ and $R^5$ are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;
$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;
$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;
each $R^c$ is independently selected from $C_{1-3}$ alkyl, $C_{3-6}$ cycloalkyl, phenyl, $-(CH_2)_n-C_{3-6}$ cycloalkane, $-(CH_2)_n-$benzene, wherein said $C_{1-3}$ alkyl, $C_{3-6}$ cycloalkyl, phenyl, $-(CH_2)_n-C_{3-6}$ cycloalkane, $-(CH_2)_n-$benzene are each optionally substituted with one or more R'; preferably, each $R^c$ is independently selected from methyl, ethyl, propyl, isopropyl, cyclopropyl, cyclopropylmethyl, benzyl and phenyl, further preferably, each $R^c$ is independently selected from methyl and ethyl;
each $R^d$ is independently selected from $C_{1-3}$ alkyl, $C_{3-6}$ cycloalkyl, $-(CH_2)_n-C_{3-6}$ cycloalkane, wherein said $C_{1-3}$ alkyl, $C_{3-6}$ cycloalkyl, $-(CH_2)_n-C_{3-6}$ cycloalkane are each optionally substituted with one or more R'; in another embodiment, each $R^d$ is independently selected from methyl, ethyl, propyl, isopropyl, cyclopropyl and cyclopropylmethyl;
$R^e$ is selected from hydrogen, fluorine, chlorine, bromine, methyl, methoxyl, trifluoromethyl and trifluoromethoxyl;
$R^m$ is selected from hydrogen, fluorine and methyl;
each R' is independently selected from H, F, Cl, and Br; preferably, each R' is independently selected from H, F and Cl; and
n is an integer of 1, 2 or 3, preferably n is an integer of 1 or 2.

In a preferred embodiment of the present invention, said compound represented by general formula (I), a stereoisomer thereof or a pharmaceutically acceptable salt thereof, is further represented by general formula (VII):

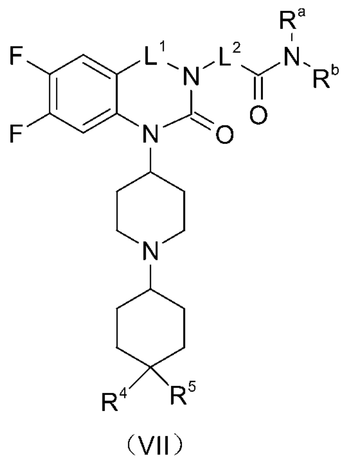

(VII)

wherein:
$R^4$, $R^5$, $R^a$, $R^b$, $L^1$, $L^2$ in general formula (VII) are as defined hereinabove.
In a preferred embodiment of the present invention, in the general formula (VII),

$R^4$ and $R^5$ are each independently selected from hydrogen, halogen, $C_{1-3}$ alkyl, or $R^4$ and $R^5$ together with the carbon atom to which they are attached form $C_{3-6}$ cycloalkyl, or $R^4$ and $R^5$ together with the carbon atom to which they are attached form a 3- to 6-membered heterocyclyl, $R^4$ and $R^5$ may be the same or different, and provided that: $R^4$ and $R^5$

are not simultaneously hydrogen, wherein said $C_{1-3}$ alkyl, $C_{3-6}$ cycloalkyl and 3- to 6-membered heterocyclyl are each optionally substituted with one or more R'; preferably, both $R^4$ and $R^5$ are $C_{1-3}$ alkyl, or one of $R^4$ and $R^5$ is $C_{1-3}$ alkyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl;

$L^1$ is selected from a single bond and $-CH_2-$;

$L^2$ is selected from $C_{1-3}$ alkylene group, said $C_{1-3}$ alkylene group is optionally substituted with one or more $R^m$, preferably, $L^2$ is selected from $-CH_2-$, $-CH_2CH_2-$, $-CH(CH_3)-$;

$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', $R^a$ and $R^b$ may be the same or different; preferably, one of $R^a$ and $R^b$ is selected from hydrogen and the other is selected from methyl, ethyl, cyclopropyl, cyclopropylmethyl, hydroxyl, hydroxyethyl; or both of $R^a$ and $R^b$ are methyl; or both of $R^a$ and $R^b$ are hydrogen; further preferably, one of $R^a$ and $R^b$ is selected from hydrogen and the other is selected from methyl, or both of $R^a$ and $R^b$ are methyl;

$R^m$ is selected from hydrogen, fluorine and methyl;

each R' is independently selected from H, F, Cl, and Br; preferably, each R' is independently selected from H, F and Cl; and

n is an integer of 1, 2 or 3, preferably n is an integer of 1 or 2.

Preferred compounds of the present invention include, but are not limited to, the compounds listed below, stereoisomers thereof or pharmaceutically acceptable salts thereof:

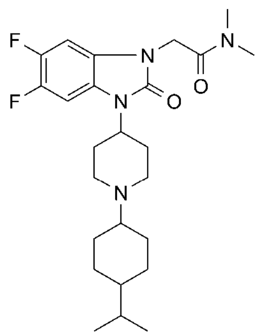

A-1

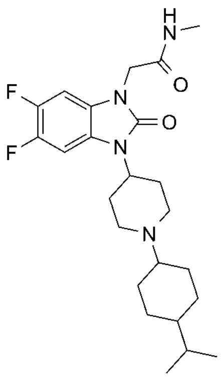

A-2

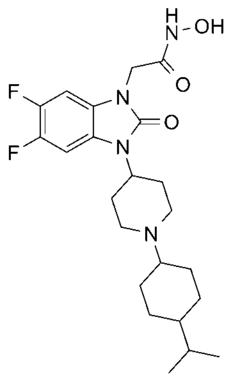

A-3

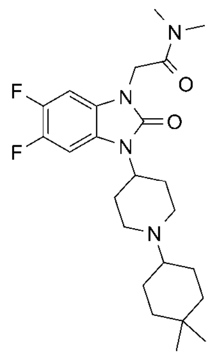

4

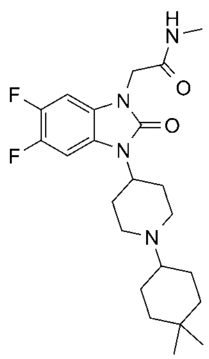

5

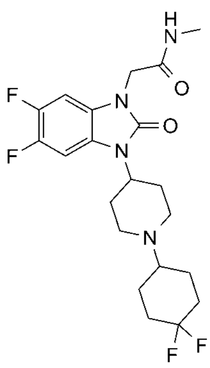

6

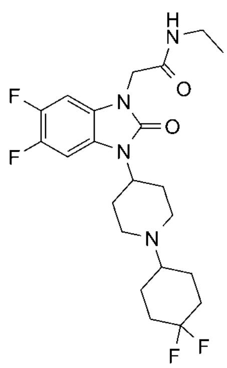

7

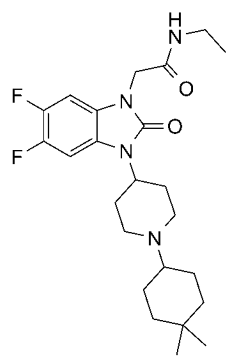

8

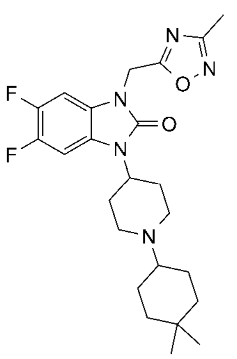

**9**

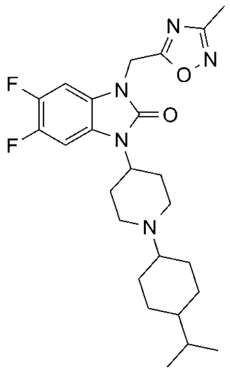

**A-10**

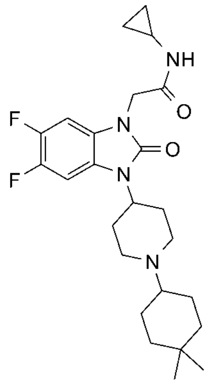

**11**

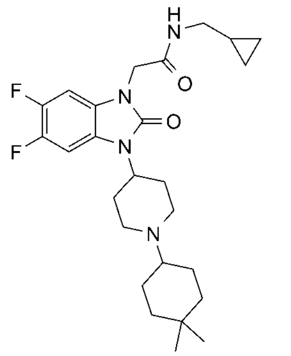

**12**

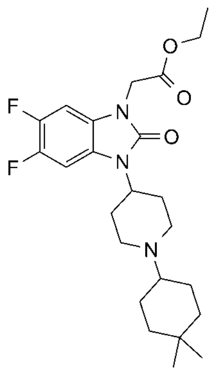

**13**

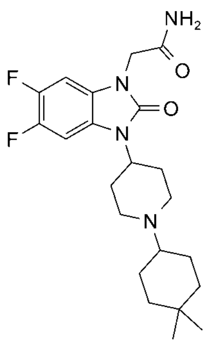

**14**

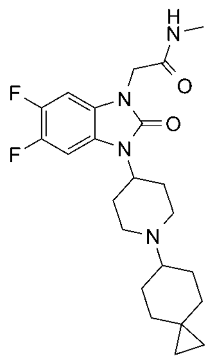

**15**

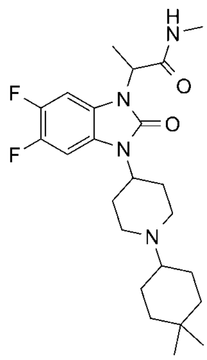

**16**

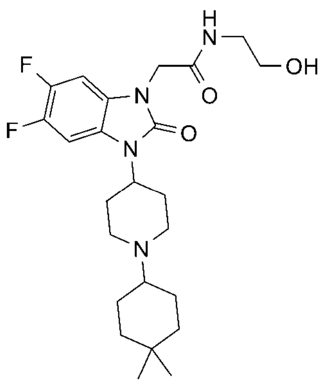

**17**

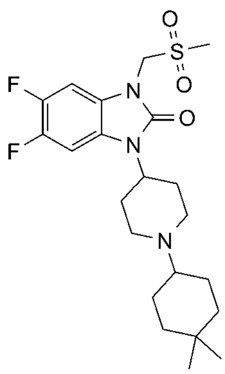

**18**

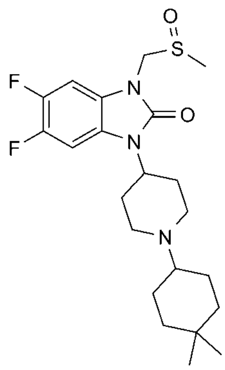

**19**

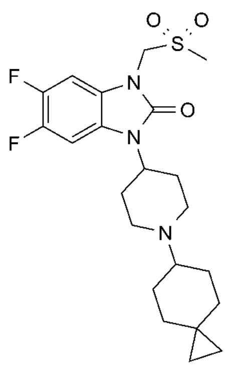

**20**

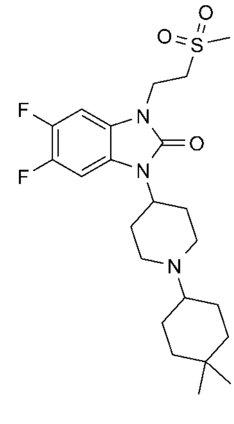

21

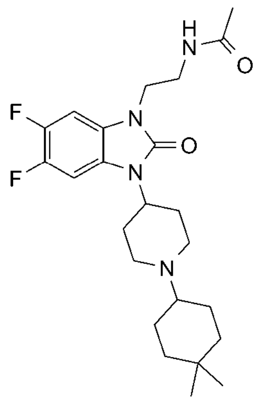

22

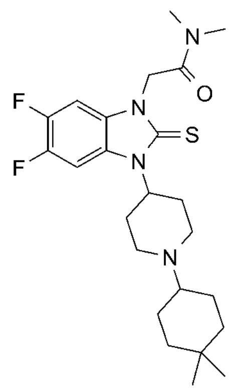

23

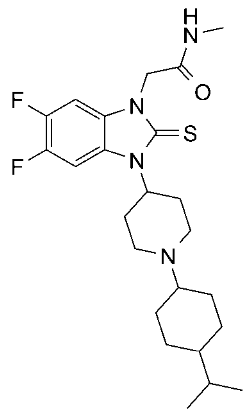

A-24

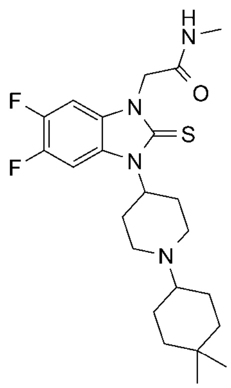

25

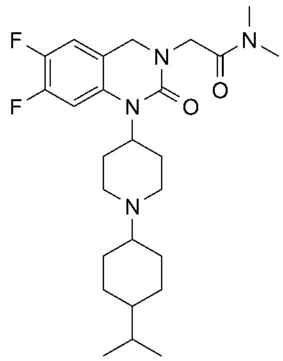

A-26

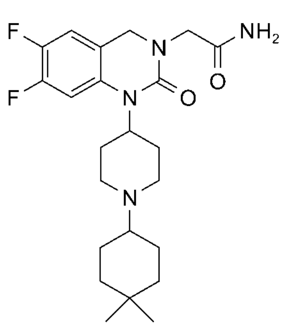

27

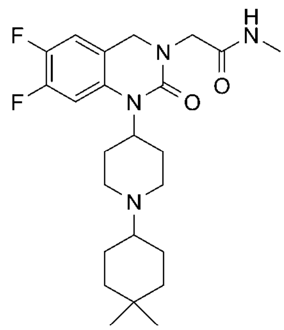

28

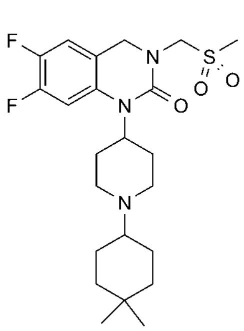

29

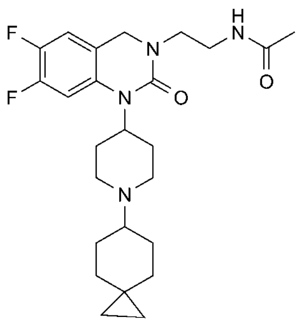

30

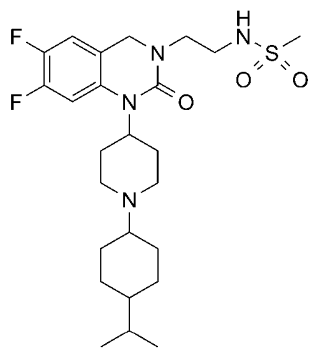

A-31

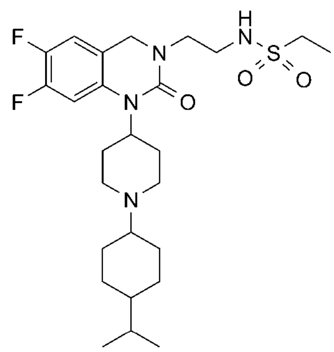

A-32

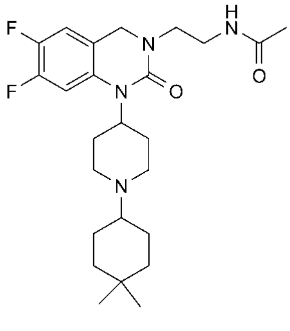

33

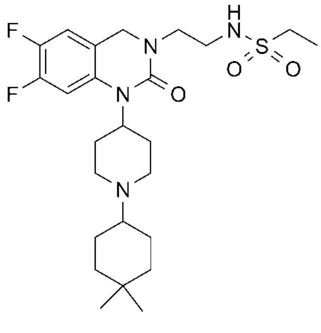

34

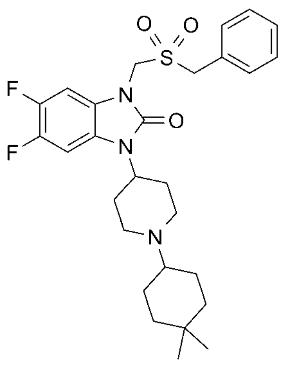

35

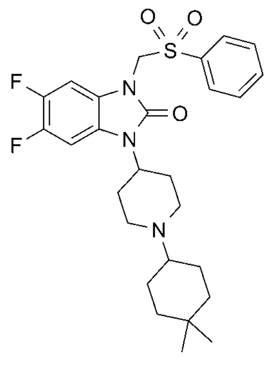

36

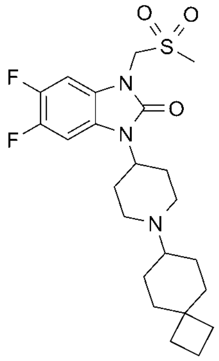

37

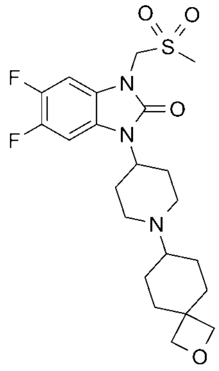

38

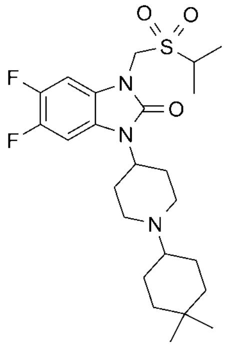

39

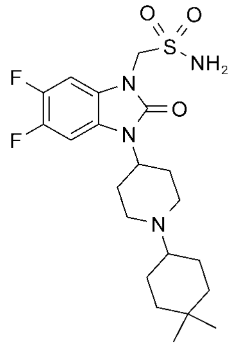

40

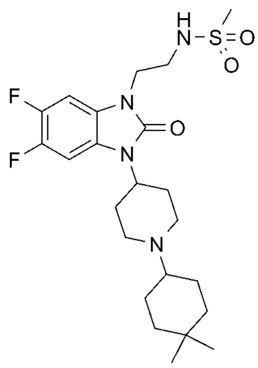

41

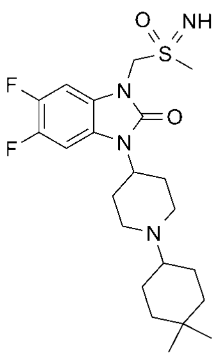

42

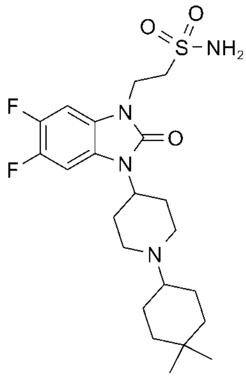

43

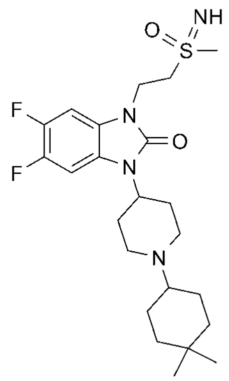

44

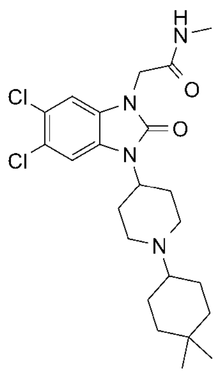

45

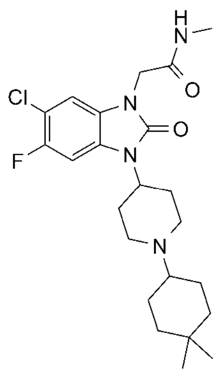

46

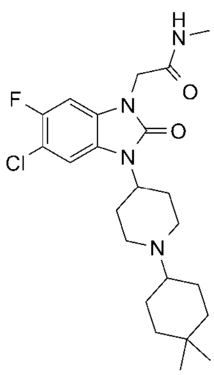

47

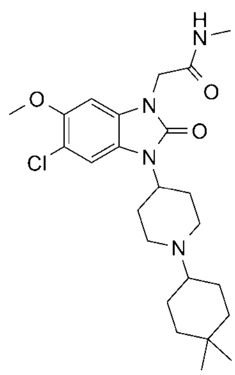

48 ;

Optionally, the compounds are selected from one of the following chemical structural formula:

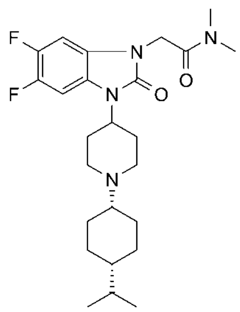

1

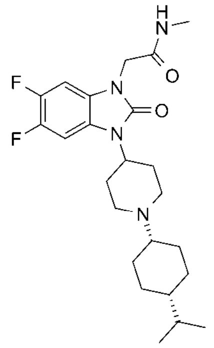

2

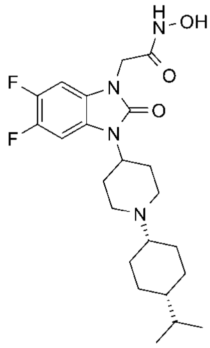

3

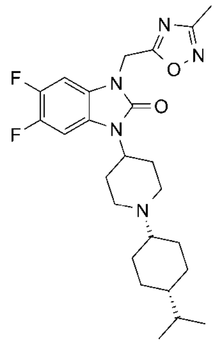

10

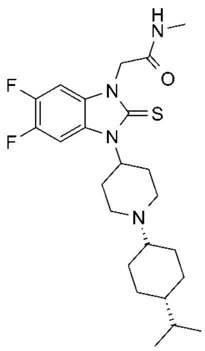

24

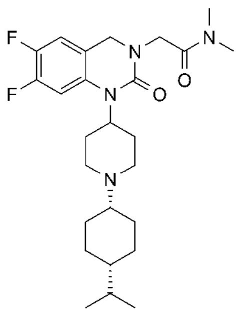

26

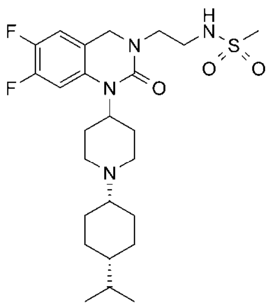

31

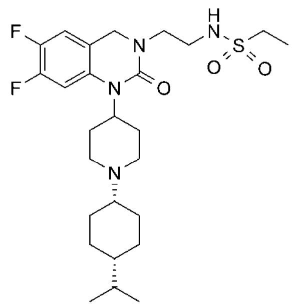

32

.

In another aspect, the present invention also provides a method for preparing the compound of the present invention, a stereoisomer thereof, or a pharmaceutically acceptable salt thereof, comprising the following steps:

(1) a compound of formula (I-1) undergoes a substitution reaction with a compound of formula (I-2) to yield a compound of formula (I-3);

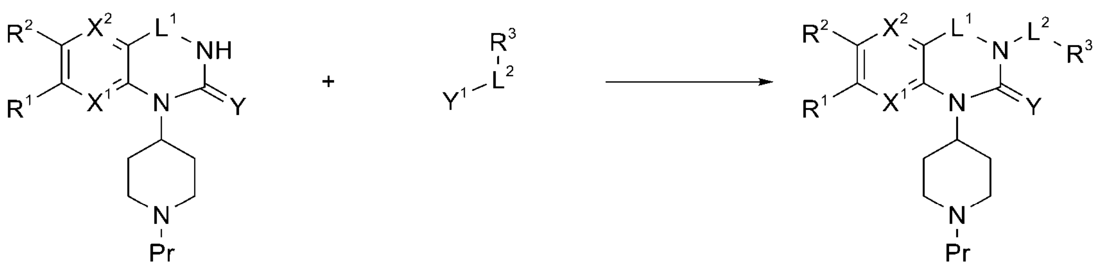

(I-1) (I-2) (I-3)

(2) the compound of formula (I-3) undergoes a deprotection reaction to yield a compound of formula (I-4), and then undergoes a reductive amination reaction with a compound of formula (I-5) to obtain a compound of formula (I);

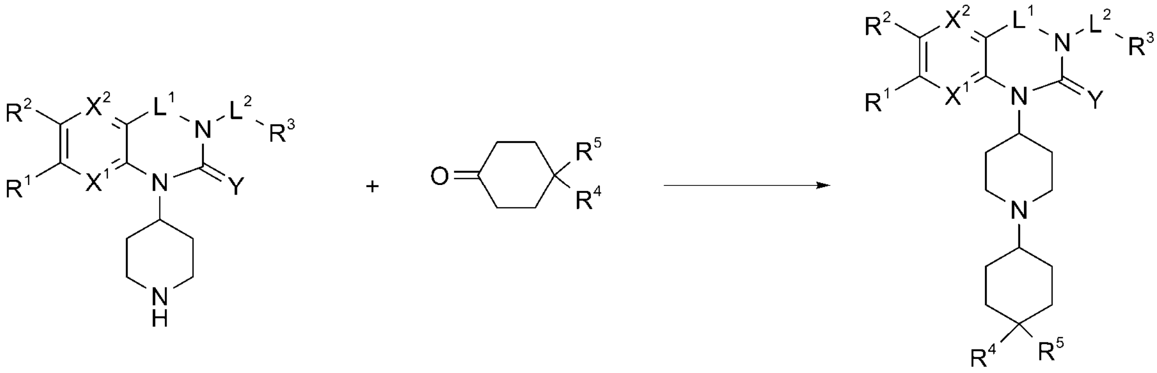

(I-4) (I-5) (I) ;

or,

(1') a compound of formula (I-6) undergoes a reductive amination reaction with the compound of formula (I-5) to yield a compound of formula (I-7);

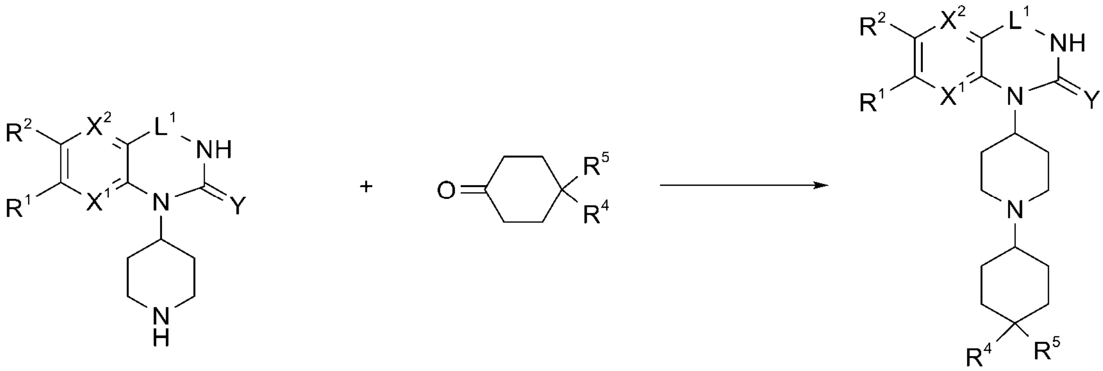

(I-6) (I-5) (I-7)

(2') the compound of formula (I-7) undergoes a substitution reaction with the compound of formula (I-2) to yield the compound of formula (I);

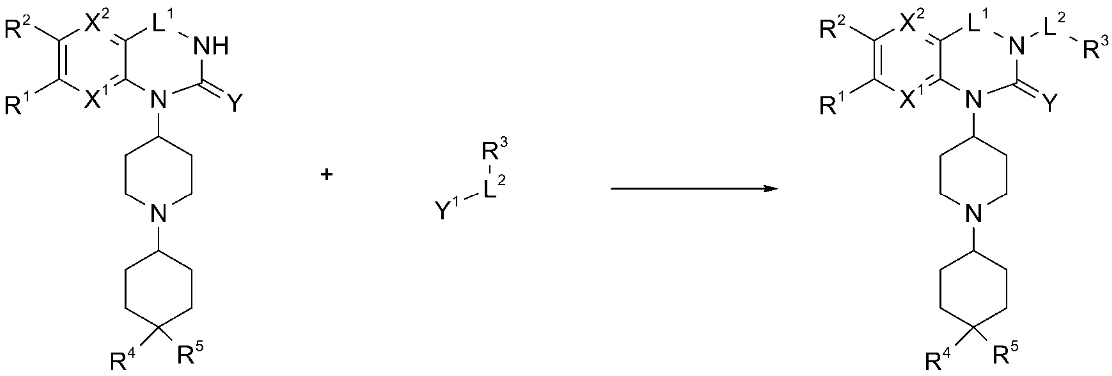

(I-7) (I-2) (I) .

In the above-mentioned preparation method, $Y^1$ in formula (I-2) represents a leaving group such as bromine, chlorine, or a sulfonate group; Pr in formula (I-1) and formula (I-3) represent an amino protecting group such as tert-butoxycarbonyl (Boc), benzyloxycarbonyl (CBz) or fluorenylmethoxycarbonyl (Fmoc), etc.; the definitions of other groups in formulas (I-1) to (I-7) and formula (I) are as described above.

In another aspect, the present invention also provides a pharmaceutical composition containing a compound of the present invention, a stereoisomer thereof, or a pharmaceutically acceptable salt thereof, and a pharmaceutically acceptable excipient. In specific embodiments, the compound of the present invention is provided in an effective amount in

the pharmaceutical composition. In specific embodiments, the compound of the present invention is provided in a therapeutically effective amount. In specific embodiments, the compound of the present invention is provided in a prophylactically effective amount.

In another aspect, the present invention also provides use of a compound of the present invention, a stereoisomer thereof, or a pharmaceutically acceptable salt thereof, or a pharmaceutical composition of the present invention in the manufacture of a medicament for the treatment and/or prevention of diseases associated with NOP receptors.

In another aspect, the present invention also provides a compound of the present invention, a stereoisomer thereof, or a pharmaceutically acceptable salt thereof or a pharmaceutical composition thereof, for use as a medicament.

In another aspect, the present invention also provides a compound of the present invention, a stereoisomer thereof, or a pharmaceutically acceptable salt thereof, or a pharmaceutical composition thereof, for use in the treatment and/or prevention of a disease associated with the NOP receptor.

In another aspect, the present invention also provides a method for the treatment and/or prevention of diseases associated with NOP receptors, and said method includes administering to a subject in need thereof a therapeutically effective amount of a compound of the present invention, a stereoisomer thereof, or a pharmaceutically acceptable salt thereof, or a pharmaceutical composition thereof.

In a specific embodiment, the disease associated with the NOP receptor is selected from pain, cough, sleep disorders, hypertension, urinary incontinence, epilepsy, traumatic injury, eating disorders, anxiety, depression, alcohol addiction, drug withdrawal syndrome, memory loss caused by Alzheimer's disease or other dementias, preferably pain.

In a specific embodiment, the pain is selected from postoperative pain, cancer-induced pain, neuropathic pain, traumatic pain and inflammation-induced pain.

Detailed Description

The terms appearing in the specification and claims of the present application that are used to describe the present application are defined below. For a particular term, the meaning defined in the present application shall prevail if the meaning defined in the present application is inconsistent with that commonly understood by those skilled in the art; and if it is not defined in the present application, it shall have the meaning commonly understood by those skilled in the art.

In the present application, the chemical name of the compound corresponds to its structural formula, and when the chemical name of the compound is inconsistent with the structural formula, the structural formula shall prevail, or it is deduced based on the specific situation of the present application combined with the common knowledge of those skilled in the art.

"$C_{6-10}$ aryl" refers to a group of a monocyclic or polycyclic (e.g., bicyclic or tricyclic) 4n+2 aromatic ring system (e.g., having 6 or 10 $\pi$ electrons shared in a cyclic arrangement) having 6-10 ring carbon atoms and zero heteroatom. In some embodiments, an aryl group has six ring carbon atoms ("$C_6$ aryl"; e.g., phenyl). In some embodiments, an aryl group has ten ring carbon atoms ("$C_{10}$ aryl"; e.g., naphthyl, such as, 1-naphthyl and 2-naphthyl). In some embodiments, $C_6$ aryl is particularly preferred. Aryl also includes ring systems wherein the aryl ring as defined above is fused with one or more cycloalkyl or heterocyclyl groups and the point of attachment is located on the aryl ring, in which case the number of carbon atoms continue to designate the number of carbon atoms in the aryl ring system.

"5- to 10-membered heteroaryl" refers to a group of a 5-10 membered monocyclic or bicyclic 4n+2 aromatic ring system (e.g., having 6 or 10 $\pi$ electrons shared in a cyclic arrangement) having ring carbon atoms and 1-4 ring heteroatoms, wherein each heteroatom is independently selected from nitrogen, oxygen and sulfur. In heteroaryl groups that contain one or more nitrogen atoms, the point of attachment can be a carbon or nitrogen atom, as valency permits. Heteroaryl bicyclic ring systems can include one or more heteroatoms in one or both rings. Heteroaryl also includes ring systems in which the heteroaryl ring as defined above is fused with one or more cycloalkyl or heterocyclyl groups and the point of attachment is located on the heteroaryl ring, in which case the number of carbon atoms continue to designate the number of carbon atoms in the heteroaryl ring system. In some embodiments, 5- to 6-membered heteroaryl groups are particularly preferred, which are 5-6 membered monocyclic or bicyclic 4n+2 aromatic ring systems having ring carbon atoms and 1-4 ring heteroatoms.

Exemplary 5-membered heteroaryl groups containing one heteroatom include, without limitation, pyrrolyl, furyl, and thienyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5, 6-bicyclic heteroaryl groups include, without limitation, indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indenazinyl, and purinyl. Exemplary 6, 6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Halogen" refers to fluorine, chlorine, bromine and iodine atoms.

"Alkyl" refers to a straight or branched monovalent saturated hydrocarbon group.

"$C_{1-6}$ alkyl" refers to a straight or branched chain saturated hydrocarbon group having 1 to 6 carbon atoms, also referred to as "lower alkyl". Examples of said alkyl include, but are not limited to, methyl, ethyl, n-propyl, isopropyl, n-butyl, isobutyl, s-butyl and t-butyl, n-pentyl, isopentyl, neopentyl, hexyl. Alkyl in this application is preferably $C_{1-3}$ alkyl.

"Halogenated $C_{1-6}$ alkyl" refers that the above-mentioned "$C_{1-6}$ alkyl" is substituted with one or more halogen groups. In some embodiments, halogenated $C_{1-3}$ alkyl groups are particularly preferred. Exemplary halogenated alkyl groups include, but are not limited to: $-CF_3$, $-CH_2F$, $-CHF_2$, $-CH_3CH_2F$, $-CH_2CHF_2$, $-CF_2CF_3$, $-CCl_3$, $-CH_2Cl$, $-CHCl_2$, and the like.

"$C_{1-6}$ alkoxy" refers to the group -OR, in which R is a substituted or unsubstituted $C_1$-$C_6$ alkyl. In some embodiments, $C_{1-3}$ alkoxy groups are particularly preferred. In some embodiments, specific alkoxy groups include, but are not limited to: methoxyl, ethoxyl, n-propoxy, isopropoxy, n-butoxy, tert-butoxy, sec-butoxy, n-pentyloxy, n-hexyloxy and 1, 2-dimethylbutoxy.

"$C_{3-8}$ cycloalkyl" refers to a non-aromatic cyclic hydrocarbon group having 3 to 8 ring carbon atoms and zero heteroatom. In some embodiments, $C_{3-6}$ cycloalkyl is preferred, more preferably $C_3$ cycloalkyl. Cycloalkyl also includes ring systems in which the cycloalkyl ring described above is fused with one or more aryl or heteroaryl groups, in which the point of attachment is located on the cycloalkyl ring, and in such instances, the number of carbons continue to designate the number of carbons in the cycloalkyl system. Exemplary cycloalkyl groups include, but are not limited to: cyclopropyl, cyclobutyl, cyclopentyl, cyclohexyl.

"3- and 8-membered heterocyclyl" refers to a group of a 3-8 membered non-aromatic ring system having ring carbon atoms and 1 to 3 ring heteroatoms, in which each heteroatom is independently selected from nitrogen, oxygen, sulfur, boron, phosphorus and silicon. In heterocyclyl groups that contain one or more nitrogen atoms, the point of attachment can be a carbon or nitrogen atom, as valency permits. In some embodiments, 3- to 6-membered heterocyclyl groups are particularly preferred, which are 3- to 6-membered non-aromatic ring systems having ring carbon atoms and 1 to 3 ring heteroatoms; more preferred is a 3- to 4-membered heterocyclyl group, which is a 3- to 4-membered non-aromatic ring system having ring carbon atoms and 1 to 3 ring heteroatoms. Heterocyclyl also includes ring systems in which the heterocyclyl ring, as defined above, is fused with one or more cycloalkyl, aryl or heteroaryl groups, in which the point of attachment is located on the heterocyclyl ring; and in such instances, the number of ring members continue to designate the number of ring members in the heterocyclyl ring system. Exemplary heterocyclyl groups include, but are not limited to: azetidinyl, tetrahydrofuryl, tetrahydrothienyl, pyrrolidinyl, piperidinyl, tetrahydropyranyl, piperazinyl, morpholinyl, dithianyl, etc.

"Hydroxyl" refers to -OH.

"Stereoisomer(s)" refers to isomer(s) resulting from differences in the spatial arrangement of atoms in molecules, including *cis-trans* isomers, enantiomers, and conformational isomers.

"Pharmaceutically acceptable salt" refers to a pharmaceutically acceptable organic or inorganic salt as defined above of a compound of the present invention, and the salt possesses the desired pharmacological activity. Such salts include acid addition salts formed with inorganic or organic acids. Pharmaceutically acceptable salts also include base addition salts, which may be formed in the presence of acidic protons capable of reacting with inorganic or organic bases.

The term "optional" or "optionally" means that the subsequently described event or circumstance may occur, but not necessarily occur, and that the description includes the instances in which said event or circumstance occurs and the instances where it does not. For example: the term "optionally substituted with one or more $R^m$" means that it may be substituted, or it may not be substituted. When being substituted, it means that any one or more hydrogen atoms on the particular atom are replaced by the substituent $R^m$.

When any variable (e.g., R) occurs more than once in the composition or structure of a compound, its definition at each occurrence is independent. Thus, for example, if a group is substituted with 1-2 R, the group may optionally be substituted with up to two R, with R in each case having an independent option. Moreover, combinations of substituents and/or variations thereof are permissible only if such combinations result in stable compounds.

Compared with the prior arts, the present invention has one or more of the following beneficial effects:

**The compounds provided by the present invention have excellent agonistic activity and selectivity for NOP receptors, holding great promise for clinical applications in treating and/or preventing diseases associated with NOP receptors. In addition, the compounds of the present invention have significant improvements in NOP agonistic activity and/or pharmacokinetic properties compared with similar compounds reported in the literature. And the compounds of the present invention have no significant effect on the gastrointestinal peristalsis function in rats.**

Additional features and advantages of the present application will be set forth in the description which follows, and in part will become apparent from the description, or may be learned by the practice of the application. Other advantages of the present application may be realized and obtained by the solutions described in the specification.

Examples

The implementation process and the resulting beneficial effects of the present invention are described in detail below through specific examples, intending to help the readers better understand the essence and characteristics of the present invention, which shall not be construed as limiting the scope of implementation of the present invention. The following examples are intended to illustrate the present application only and should not be considered as limiting the scope of the present application.

The structures of the compounds are determined by Nuclear Magnetic Resonance (NMR) and/or Mass Spectrometry (MS). The NMR shift (δ) were given in units of $10^{-6}$. NMR was measured using a (Bruker Ultrashield 400 MHz) nuclear magnetic resonance instrument, and the measurement solvent was deuterated dimethyl sulfoxide (DMSO-$d_6$), deuterated chloroform ($CDCl_3$), deuterated methanol ($CH_3OD$), heavy water ($D_2O$) as solvent, the chemical shift δ values (ppm) were reported relative to tetramethylsilane (TMS) as internal standard.

LCMS was measured using Waters ACQUITY UPLC.

High Performance Liquid Chromatography (HPLC) analysis was performed on Waters preparative high performance liquid chromatograph system equipped with a YMC-Triart-C18 EXRS column (20 mm × 100 mm × 5 μm).

The silica gel plates used for thin layer chromatography were GF254 silica gel plates purchased from Xiya Reagent.

Column chromatography was carried out by using 200-300 mesh silica gel (Qingdao Ocean Chemical Co., Ltd.) as the support.

Unless otherwise stated, starting materials and reagents used in the following examples are commercially available or can be prepared by known methods. The following abbreviations are employed herein: equiv. stands for ratio of equivalents; sat. stands for saturated; M stands for mol/L; rt stands for room temperature; EtOH stands for ethanol; MeOH stands for methanol; DCM stands for dichloromethane; DCE stands for 1,2-dichloroethane; DMSO stands for dimethyl sulfoxide; DMF stands for N,N-dimethylformamide; EA stands for ethyl acetate; PE stands for petroleum ether; THF stands for tetrahydrofuran; TEA stands for triethylamine; TFA stands for trifluoroacetic acid; AcOH stands for acetic acid; CDI stands for N,N'-carbonyldiimidazole; Boc stands for tert-butoxycarbonyl (a protecting group for amine); $NaBH(OAc)_3$ stands for sodium triacetoxyborohydride; $Boc_2O$ stands for di-tert-butyl dicarbonate; MsCl stands for Mesyl chloride; Xantphos stands for 4,5-bis(diphenylphosphino)-9,9-dimethylxanthene (a ligand for palladium catalysts); $Pd_2(dba)_3$ stands for tris(dibenzylideneacetone)dipalladium; $ddH_2O$ stands for doubly distilled water; TLC: Thin Layer Chromatography; HPLC: High Performance Liquid Chromatography; $^1H$ NMR: Proton Nuclear Magnetic Resonance Spectroscopy; LC-MS: Liquid Chromatography-Mass Spectrometry. Compounds are named according to conventional naming conventions in the art or using ChemDraw® software (PerkinElmer), and the commercially available reagents were used as supplied under their catalog names.

**Example 1:** Synthesis of 2-(5,6-difluoro-3-(1-(cis-4-isopropylcyclohexyl)piperidin-4-yl)-2-oxo-2,3-dihydro-1*H*-benzo[*d*]imidazol-1-yl)-N,N-dimethylacetamide (**1**)

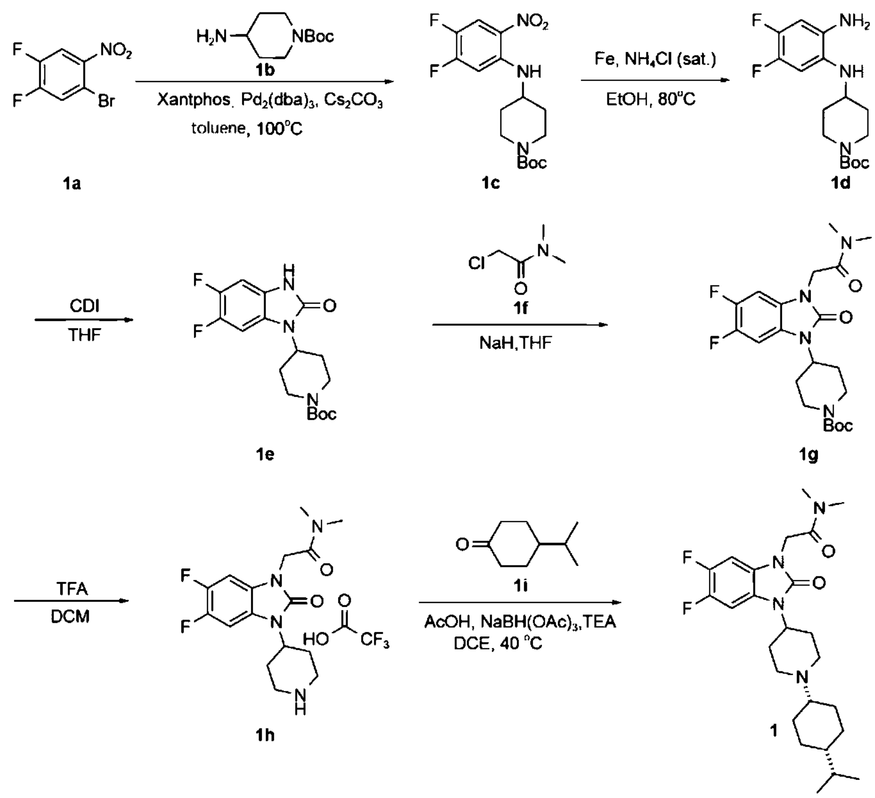


Synthesis of intermediate **1c**: To a clean, dry flask (250 mL) were added sequentially 2-bromo-4,5-difluoronitrobenzene (**1a**) (12.50 g, 52.52 mmol), Xantphos ligand (1.52 g, 2.63 mmol), and tris(dibenzylideneacetone)dipalladium (1.20 g, 1.31 mmol). After being purged with nitrogen, toluene (150 mL) was added. Under stirring at room temperature, anhydrous cesium carbonate (23.96 g, 73.53 mmol) and 4-amino-1-tert-butoxycarbonylpiperidine (**1b**) (12.62 g, 63.02 mmol) were added sequentially. The mixture was heated to 100 °C, and the reaction was monitored by TLC until starting material **1a** had reacted substantially to completion. After cooling to room temperature, the reaction solution was filtered, and the filter cake was washed with dichloromethane. The filtrate was concentrated, and the crude product was purified by column chromatography (the mobile phase was petroleum ether and ethyl acetate, and the volume ratio was 5:1) to obtain 9.45 g of a yellow solid, with a yield of 50.40%. LC-MS (ESI) m/z: 358.16 $(M + H)^+$.

Synthesis of intermediate **1d**: Intermediate **1c** (9.45 g, 26.44 mmol) was placed in a single-neck flask (500 mL), and anhydrous ethanol (270 mL) was added thereto. Under stirring at room temperature, iron powder (9.15 g, 163.86 mmol) and saturated aqueous $NH_4Cl$ solution (27 mL) were added to the reaction system. The mixture was heated to 80 °C, and the reaction was monitored by TLC until the starting material 1c had reacted substantially to completion. Upon cooling to room temperature, the reaction solution was filtered through celite, and the filter cake was washed with a small amount of anhydrous ethanol. The filtrate was concentrated, and the crude product was purified by column chromatography (the mobile phase was petroleum ether and ethyl acetate, and the volume ratio was 5:1) to obtain 7.31 g of a gray solid, with a yield of 84.45%. LC-MS (ESI) m/z: 328.36 $(M + H)^+$.

Synthesis of intermediate **1e**: Intermediate **1d** (4.52 g, 13.80 mmol) and anhydrous tetrahydrofuran (60 mL) were placed in a clean, dry flask. After being purged with nitrogen, N,N'-carbonyldiimidazole (3.12 g, 19.24 mmol) was added at a temperature of 0 °C. The mixture was stirred at room temperature for 12 h. The reaction was quenched after the reaction was confirmed to be complete by TLC. The reaction solution was concentrated under reduced pressure, and the crude product was purified by column chromatography (the mobile phase was petroleum ether and ethyl acetate, and the volume ratio was 1:1) to obtain 3.56 g of a yellow solid, with a yield of 73.06%. LC-MS (ESI) m/z: 354.22 $(M + H)^+$; $^1H$ NMR (400 MHz, DMSO-$d_6$) $\delta$ 11.03 (s, 1H), 7.44 (dd, J = 9.9, 8.3 Hz, 1H), 7.03 (dd, J = 9.4, 8.1 Hz, 1H), 4.28 (tt, J = 12.2, 3.9 Hz, 1H), 4.08 (d, J = 13.1 Hz, 2H), 2.83 (s, 2H), 2.19 (qd, J = 12.5, 4.4 Hz, 2H), 1.66 (d, J = 12.0 Hz, 2H), 1.43 (s, 9H).

Synthesis of intermediate **1g**: To a clean, dry flask were added intermediate **1e** (2.76 g, 7.82 mmol) and THF (30 mL). After being purged with nitrogen, 60% NaH by mass (0.93 g, 23.46 mmol) was slowly added at 0 °C, and the mixture was stirred for 0.5 h. 2-chloro-N,N-dimethylacetamide (**1f**) (1.14 g, 9.38 mmol) was added. After stirring at room temperature for 16 h, the reaction was confirmed to be complete by TLC. At 0 °C, water was added dropwise to quench the reaction. The mixture was extracted with ethyl acetate (30 mL $\times$ 3). The organic phase was dried over anhydrous $Na_2SO_4$. After filtering, the filtrate was evaporated via a rotary evaporator under reduced pressure to remove the solvent and thus obtain 3.24 g of a white solid, with a yield of 94.60%. LC-MS (ESI) m/z: 439.13 $(M + H)^+$.

Synthesis of intermediate **1h**: To a clean, dry flask was added intermediate **1g** (2.12 g, 4.84 mmol), and anhydrous dichloromethane (20 mL) was added thereto. At 0 °C, trifluoroacetic acid (5 mL) was added dropwise. After the addition was complete, the ice-water bath was removed. The mixture was stirred at room temperature for 4 h. It was confirmed by monitoring with TLC plates that the starting materials had reacted substantially to completion. The mixture was evaporated via a rotary evaporator under reduced pressure to remove the solvent and thus obtain a light brown oily crude product. Diethyl ether (50 mL) was added to the crude product, and the mixture was dispersed, stirred, filtered, and dried to obtain 2.18 g of a yellow solid, with a yield of 99.68%. The yellow solid was used directly in the next experiment without further purification.

Synthesis of compound **1**: To a clean, dry flask were added intermediate **1h** (2.18 g, 4.84 mmol) and anhydrous 1,2-dichloroethane (10mL). Triethylamine was added dropwise to adjust the pH to approximately 8, with stirring for 10 minutes. 4-isopropylcyclohexanone (**1i**) (1.69 g, 12.10 mmol) was added. Acetic acid (0.38 g, 6.29 mmol) was added to adjust the pH to approximately 5. After stirring at room temperature for 2 h, sodium triacetoxyborohydride (3.08 g, 14.52 mmol) was added. The reaction was carried out at 40 °C for 16 h, and the reaction was confirmed to be complete by TLC. The mixture was evaporated via a rotary evaporator under reduced pressure to remove the solvent and thus obtain a yellow solid. The crude product was purified by preparative HPLC (YMC Triart C18 EXRS, 4.6 × 150 mm, 3 μm; mobile phase A: 0.1% aqueous ammonia solution; mobile phase B: 0.1% ammonia in acetonitrile; isocratic elution: 90% B/A (15 min); flow rate: 1.0 mL/min, retention time: 3.63 min) to obtain 200 mg of a white solid, with a yield of 8.93%. LC-MS (ESI) m/z: 463.36 $(M + H)^+$; $^1H$ NMR (400 MHz, DMSO-$d_6$) δ 7.44 (dd, *J* = 11.0, 7.0 Hz, 1H), 7.30 (dd, *J* = 10.8, 7.1 Hz, 1H), 4.69 (s, 2H), 4.18 - 3.97 (m, 0H), 3.07 (s, 4H), 2.83 (s, 3H), 2.30 (dt, *J* = 12.5, 8.9 Hz, 3H), 2.11 (t, *J* = 11.6 Hz, 2H), 1.79 - 1.48 (m, 8H), 1.48 - 1.24 (m, 2H), 1.09 (s, 1H), 0.86 (d, *J* = 6.6 Hz, 6H).

**Example 2:** Synthesis of 2-(5,6-difluoro-3-(1-(*cis*-4-isopropylcyclohexyl)piperidin-4-yl)-2-oxo-2,3-dihydro-1*H*-benzo[*d*]imidazol-1-yl)-N-methylacetamide (**2**)

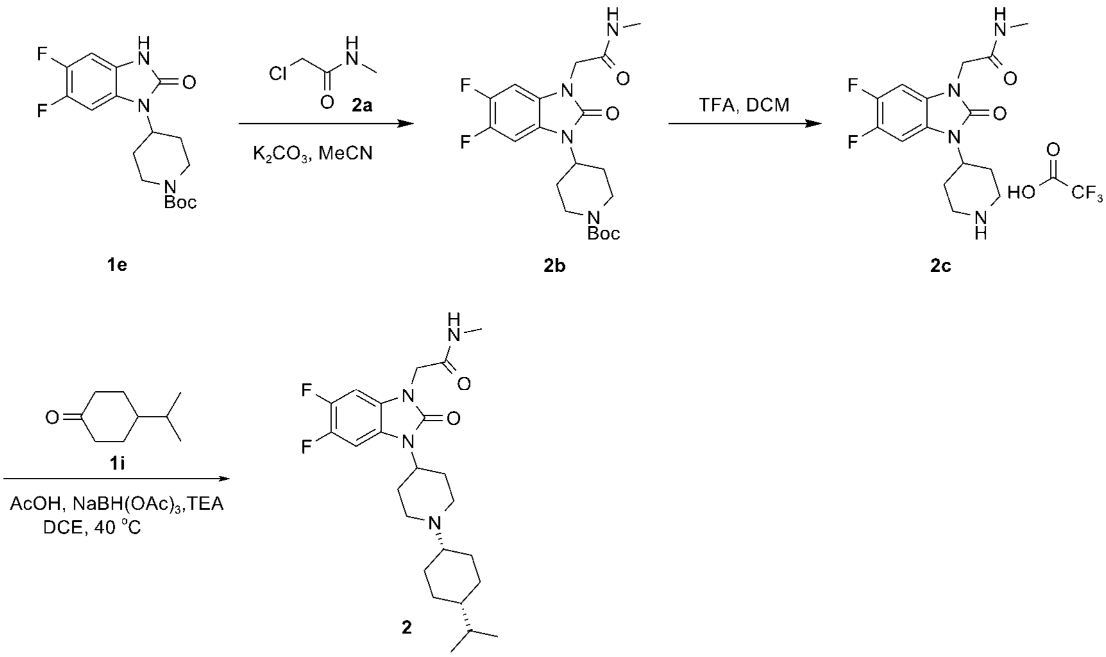


**1e** **2b** **2c**

**2**

Synthesis of intermediate **2b**: To a clean, dry flask were added sequentially intermediate **1e** (1.20 g, 3.40 mmol), acetonitrile (15 mL), potassium carbonate (0.94 g, 6.81 mmol), and 2-chloro-N-methylacetamide (**2a**) (0.48 g, 4.42 mmol). The mixture was stirred under reflux at 70 °C for 12 h, and the reaction was confirmed to be complete by TLC. The mixture was evaporated via a rotary evaporator under reduced pressure to remove the solvent. Water and dichloromethane were added to the residue, and the mixture was extracted three times. The organic phase was dried over anhydrous $Na_2SO_4$ and then filtered, and the filtrate was evaporated via a rotary evaporator under reduced pressure to remove the solvent. The crude product was purified by column chromatography (the mobile phase was dichloromethane and methanol, and the volume ratio was 30:1) to obtain 1.24 g of a white solid, with a yield of 86.11%.

Synthesis of intermediate **2c**: To a clean, dry flask was added intermediate **2b** (0.80 g, 1.88 mmol), and anhydrous dichloromethane (9 mL) was added thereto. At 0 °C, trifluoroacetic acid (2.5 mL) was added dropwise. After

the addition was complete, the ice-water bath was removed, and the mixture was stirred at room temperature for 3 h. It was confirmed by monitoring with TLC plates that the starting materials had reacted substantially to completion. The mixture was evaporated via a rotary evaporator under reduced pressure to remove the solvent and thus obtain a crude product. Diethyl ether (50 mL) was added to the crude product, and the mixture was dispersed, stirred, filtered, and dried to obtain 0.81 g of a yellow solid, with a yield of 98.06%.

The yellow solid was used directly in the next experiment without further purification.

Synthesis of compound **2**: To a clean, dry flask were added intermediate **2c** (0.60 g, 1.37 mmol) and anhydrous 1,2-dichloroethane (8 mL). Triethylamine was added dropwise to adjust the pH to approximately 8, with stirring for 10 min. 4-isopropylcyclohexanone (0.48 g, 3.42 mmol) was added. Acetic acid (0.12 g, 2.05 mmol) was added to adjust the pH to approximately 5. After stirring at room temperature for 2 h, sodium triacetoxyborohydride (0.87 g, 4.11 mmol) was added. The mixture was reacted at 40 °C for 16 h, and the reaction was confirmed to be complete by TLC. The mixture was evaporated via a rotary evaporator under reduced pressure to remove the solvent and thus obtain a yellow solid. The crude product was purified by preparative HPLC (YMC Triart C18 EXRS, 4.6 × 150 mm, 3 $\mu m$; mobile phase A: 0.1% aqueous ammonia solution; mobile phase B: 0.1% ammonia in acetonitrile; isocratic elution: 90% B/A (15 min); flow rate: 1.0 mL/min; retention time: 8.42 min) to obtain 203 mg of a white solid, with a yield of 16.53%. LC-MS (ESI) m/z: 449.33 $(M + H)^+$; $^1H$ NMR (400 MHz, DMSO-$d_6$) δ 8.08 (d, *J* = 4.8 Hz, 1H), 7.45 (dd, *J* = 11.0, 6.9 Hz, 1H), 7.28 (dd, *J* = 10.7, 7.1 Hz, 1H), 4.41 (s, 2H), 4.21 - 3.98 (m, 1H), 3.07 (d, *J* = 11.1 Hz, 2H), 2.60 (d, *J* = 4.5 Hz, 3H), 2.37 - 2.21 (m, 3H), 2.10 (t, *J* = 11.6 Hz, 2H), 1.66 (d, *J* = 11.5 Hz, 4H), 1.54 (dq, *J* = 14.2, 7.3, 6.7 Hz, 3H), 1.48 - 1.28 (m, 4H), 1.16 - 1.03 (m, 1H), 0.86 (d, *J* = 6.6 Hz, 6H).

**Example 3:** Synthesis of 2-(3-(1-(4,4-dimethylcyclohexyl)piperidin-4-yl)-5,6-difluoro-2-oxo-2,3-dihydro-1*H*-benzo[*d*]imidazol-1-yl)-N,N-dimethylacetamide (**4**)

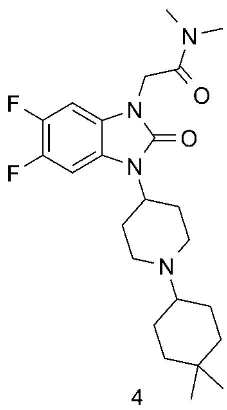

4

Intermediate **1h** (0.46 g, 1.02 mmol) and 4,4-dimethylcyclohexanone (0.32 g, 2.55 mmol) were used as stating materials. This compound was synthesized following the synthesis method in Example 1 to obtain 320 mg of a white solid, with a yield of 72.40%. LC-MS (ESI) m/z: 449.37 $(M + H)^+$; $^1H$ NMR (400 MHz, DMSO-$d_6$) δ 7.56 (t, *J* = 9.0 Hz, 1H), 7.39 (t, *J* = 9.0 Hz, 1H), 4.73 (s, 2H), 4.60 (d, *J* = 12.7 Hz, 1H), 3.60 (d, *J* = 11.7 Hz, 2H), 3.31 - 3.12 (m, 3H), 3.07 (s, 3H), 2.83 (s, 3H), 2.74 - 2.54 (m, 2H), 2.07 - 1.76 (m, 4H), 1.75 - 1.55 (m, 2H), 1.49 (d, *J* = 13.1 Hz, 2H), 1.26 (td, *J* = 13.3, 3.5 Hz, 2H), 0.92 (s, 6H).

**Example 4:** Synthesis of 2-(3-(1-(4, 4-dimethylcyclohexyl)piperidin-4-yl)-5,6-difluoro-2-oxo-2,3-dihydro-1*H*-benzo[d]imidazol-1-y l)-N-methylacetamide (**5**)

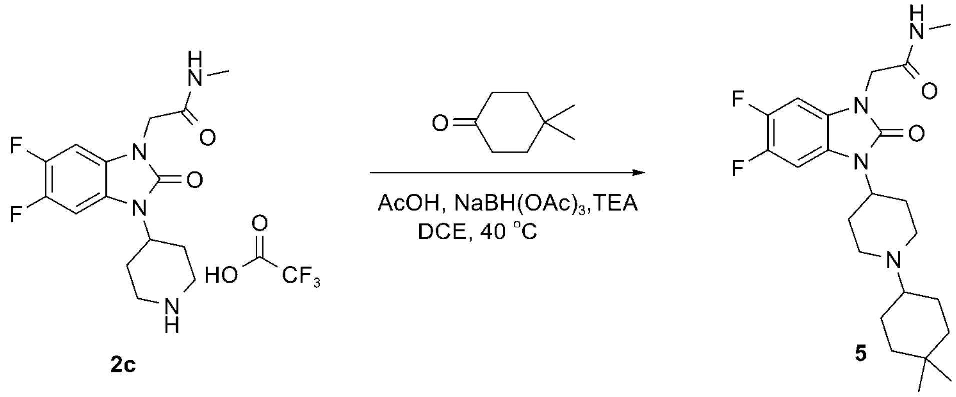


To a clean, dry flask were added intermediate **2c** (0.30 g, 0.68 mmol) and anhydrous 1,2-dichloroethane (4 mL). Triethylamine was added dropwise to adjust the pH to approximately 8, with stirring for 10 min. 4,4-dimethylcyclohexanone (0.22 g, 1.70 mmol) was added. Acetic acid was added to adjust the pH to approximately 5. After stirring at room temperature for 2 h, sodium triacetoxyborohydride (0.43 g, 2.04 mmol) was added. The mixture was reacted at 40 °C for 16 h, and the reaction was confirmed to be complete by TLC. The mixture was evaporated via a rotary evaporator under reduced pressure to remove the solvent, and the crude product was purified by column chromatography (the mobile phase was dichloromethane and methanol, and the volume ratio was 20:1) to obtain 230 mg of a white solid, with a yield of 78.23%. LC-MS (ESI) m/z: 435.37 $(M + H)^+$; $^1H$ NMR (400 MHz, DMSO-$d_6$) δ 8.11 (q, *J* = 4.6 Hz, 1H), 7.58 (t, *J* = 8.9 Hz, 1H), 7.35 (t, *J* = 8.9 Hz, 1H), 4.52 (s, 1H), 4.44 (s, 2H), 3.50 (s, 2H), 3.08 (q, *J* = 7.3 Hz, 3H), 2.61 (d, *J* = 4.5 Hz, 3H), 1.86 (d, *J* = 36.9 Hz, 4H), 1.60 (d, *J* = 12.6 Hz, 2H), 1.48 (d, *J* = 13.1 Hz, 2H), 1.35 - 1.20 (m, 2H), 1.17 (t, *J* = 7.3 Hz, 2H), 0.91 (s, 6H).

**Example 5:** Synthesis of 2-(3-(1-(4,4-difluorocyclohexyl)piperidin-4-yl)-5,6-difluoro-2-oxo-2,3-dihydro-1*H*-benzo[*d*]imidazol-1-yl)-N-methylacetamide (**6**)

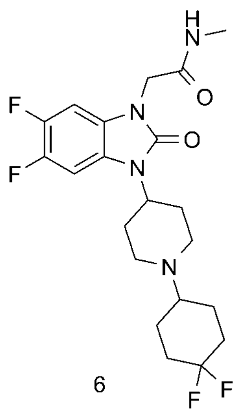

6

Intermediate **2c** (0.30 g, 0.68 mmol) and 4,4-difluorocyclohexanone (0.23 g, 1.70 mmol) were used as starting materials. This compound was synthesized following the synthesis method in Example 4 to obtain 220 mg of a white solid, with a yield of 73.09%. LC-MS (ESI) m/z: 443.22 $(M + H)^+$; $^1H$ NMR (400 MHz, DMSO-$d_6$) δ 8.08 (d, *J* = 4.8 Hz, 1H), 7.48 (dd, *J* = 11.1, 6.9 Hz, 1H), 7.27 (dd, *J* = 10.7, 7.1 Hz, 1H), 4.41 (s, 2H), 4.22 - 3.98 (m, 1H), 3.49 - 3.45 (m, 1H), 2.95 (d, *J* = 6.8 Hz, 2H), 2.60 (d, *J* = 4.6 Hz, 3H), 2.41 - 2.20 (m, 4H), 2.05 (d, *J* = 10.8 Hz, 3H), 1.93 - 1.73 (m, 4H), 1.73 - 1.60 (m, 2H), 1.54 (q, *J* = 13.3, 12.6 Hz, 2H).

**Example 6:** Synthesis of 2-(3-(1-(4,4-difluorocyclohexyl)piperidin-4-yl)-5,6-difluoro-2-oxo-2,3-dihydro-1*H*-benzo[*d*]imidazol-1-yl)-N-ethylacetamide (**7**)

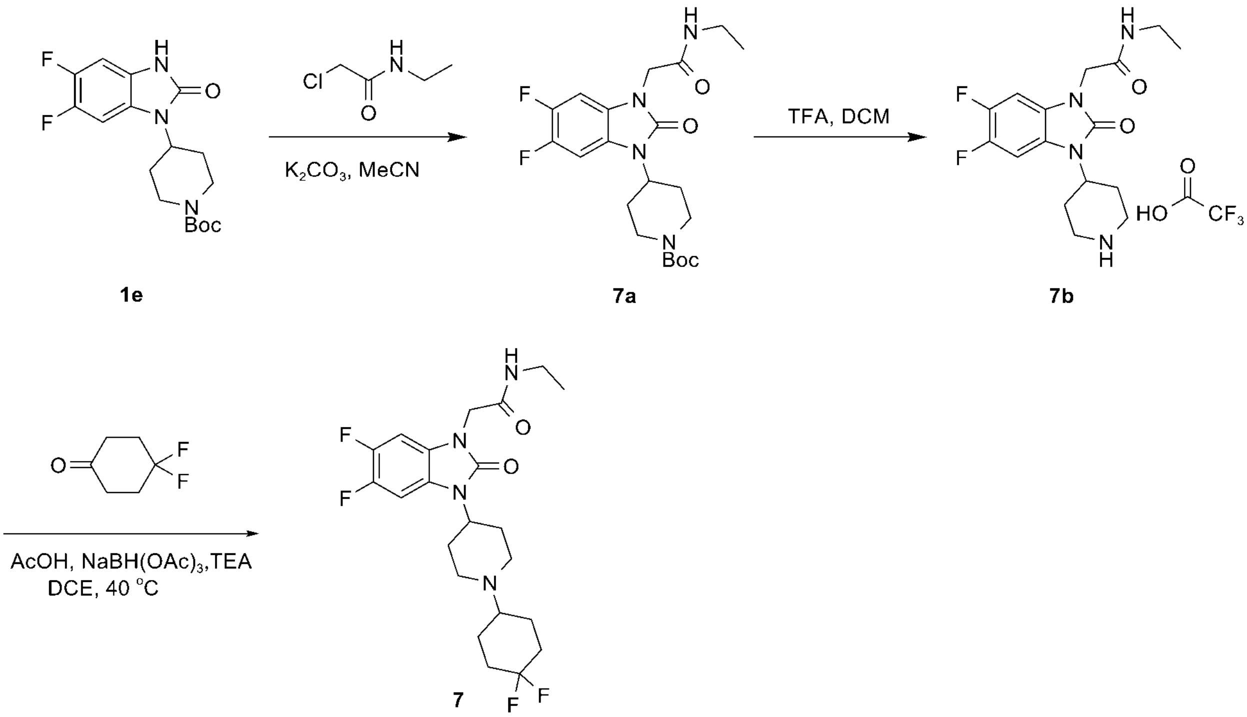


1e 7a 7b

7

Synthesis of intermediate **7a**: To a clean, dry flask were added sequentially intermediate **1e** (1.20 g, 3.40 mmol), acetonitrile (15 mL), potassium carbonate (0.94 g, 6.81 mmol), and 2-chloro-N-ethylacetamide (0.54 g, 4.42 mmol). The mixture was stirred under reflux at 70 °C for 12 h, and the reaction was confirmed to be complete by TLC. The mixture was evaporated via a rotary evaporator under reduced pressure to remove the solvent. Water and dichloromethane were added to the residue, and the mixture was extracted three times. The organic phase was dried over anhydrous $Na_2SO_4$ and then filtered, and the filtrate was evaporated via a rotary evaporator under reduced pressure to remove the solvent. The crude product was purified by column chromatography (the mobile phase was dichloromethane and methanol, and the volume ratio was 30:1) to obtain 1.30 g of a white solid, with a yield of 87.25%.

Synthesis of intermediate **7b**: To a clean, dry flask was added intermediate **7a** (0.80 g, 1.82 mmol), and anhydrous dichloromethane (9 mL) was added thereto. At 0 °C, trifluoroacetic acid (2.5 mL) was added dropwise. After the addition was complete, the ice-water bath was removed, and the mixture was stirred at room temperature for 3 h. It was confirmed by monitoring with TLC plates that the starting materials had reacted substantially to completion. The mixture was evaporated via a rotary evaporator under reduced pressure to remove the solvent and thus obtain a crude product. Diethyl ether (5 mL) was added to the crude product, and the mixture was dispersed, stirred, filtered, and dried to obtain 0.82 g of a yellow solid, with a yield of 99.39%. The yellow solid was used directly in the next experiment without further purification.

Synthesis of compound **7**: To a clean, dry flask were added intermediate **7b** (0.82 g, 1.81 mmol) and anhydrous 1,2-dichloroethane (8 mL). Triethylamine was added dropwise to adjust the pH to approximately 8, with stirring for 10 min. 4,4-difluorocyclohexanone (607 mg, 4.53 mmol) was added. Acetic acid was added to adjust the pH to approximately 5. After stirring at room temperature for 2 h, sodium triacetoxyborohydride (1.15 g, 5.43 mmol) was added. The reaction was carried out at 40 °C for 16 hours, and the reaction was confirmed to be complete by TLC. The mixture was evaporated via a rotary evaporator under reduced pressure to remove the solvent, and the crude product was purified by column chromatography (the mobile phase was dichloromethane and methanol, and the volume ratio was 20:1) to obtain 640 mg of a white solid, with a yield of 77%. LC-MS (ESI) m/z: 457.31 $(M + H)^+$; $^1H$ NMR (400 MHz, DMSO-$d_6$) δ 8.17 (t, *J* = 5.4 Hz, 1H), 7.48 (dd, *J* = 11.0, 7.0 Hz, 1H), 7.26 (dd, *J* = 10.7, 7.1 Hz, 1H), 4.40 (s, 2H), 4.20 - 4.02 (m, 1H), 3.49 - 3.45 (m, 1H), 3.17 - 3.02 (m, 2H), 2.95 (d, *J* = 6.8 Hz, 2H), 2.28 (h, *J* = 10.6 Hz, 4H), 2.05 (d, *J* = 11.3 Hz, 2H), 1.94 - 1.71 (m, 4H), 1.71 - 1.61 (m, 2H), 1.54 (q, *J* = 12.1 Hz, 2H), 1.03 (t, *J* = 7.2 Hz, 3H).

**Example 7:** Synthesis of 2-(3-(1-(4,4-dimethylcyclohexyl)piperidin-4-yl)-5,6-difluoro-2-oxo-2,3-dihydro-1*H*-benzo[*d*]i midazol-1-yl)-N-ethylacetamide (**8**)

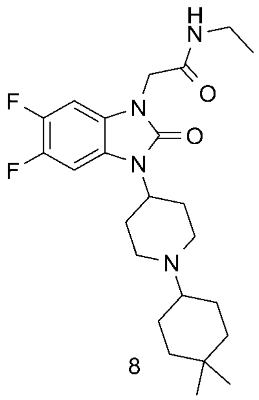

8

Intermediate **7b** (0.60 g, 1.33 mmol) and 4,4-dimethylcyclohexanone (418 mg, 3.32 mmol) were used as starting materials. This compound was synthesized following the synthesis method in Example 6 to obtain 470 mg of a yellow solid, with a yield of 78.73%. LC-MS (ESI) m/z: 449.39 $(M + H)^+$; $^1H$ NMR (400 MHz, DMSO-$d_6$) δ 8.18 (t, *J* = 5.5 Hz, 1H), 7.52 (dd, *J* = 10.8, 7.2 Hz, 1H), 7.29 (dd, *J* = 10.4, 7.3 Hz, 1H), 4.42 (s, 2H), 4.35 - 4.20 (m, 1H), 3.14 - 3.04 (m, 2H), 2.85 - 2.56 (m, 3H), 2.45 - 2.23 (m, 3H), 1.83 - 1.64 (m, 4H), 1.47 (dd, *J* = 23.1, 12.8 Hz, 4H), 1.29 - 1.14 (m, 3H), 1.03 (t, *J* = 7.2 Hz, 3H), 0.89 (s, 6H).

**Example 8:** Synthesis of 1-(l-(4,4-dimethylcyclohexyl)piperidin-4-yl)-5,6-difluoro-3-((3-methyl-1,2,4-oxadiazol-5-yl)methyl)-1,3-dihydro-2*H*-benzo[*d*]imidazol-2-one (**9**)

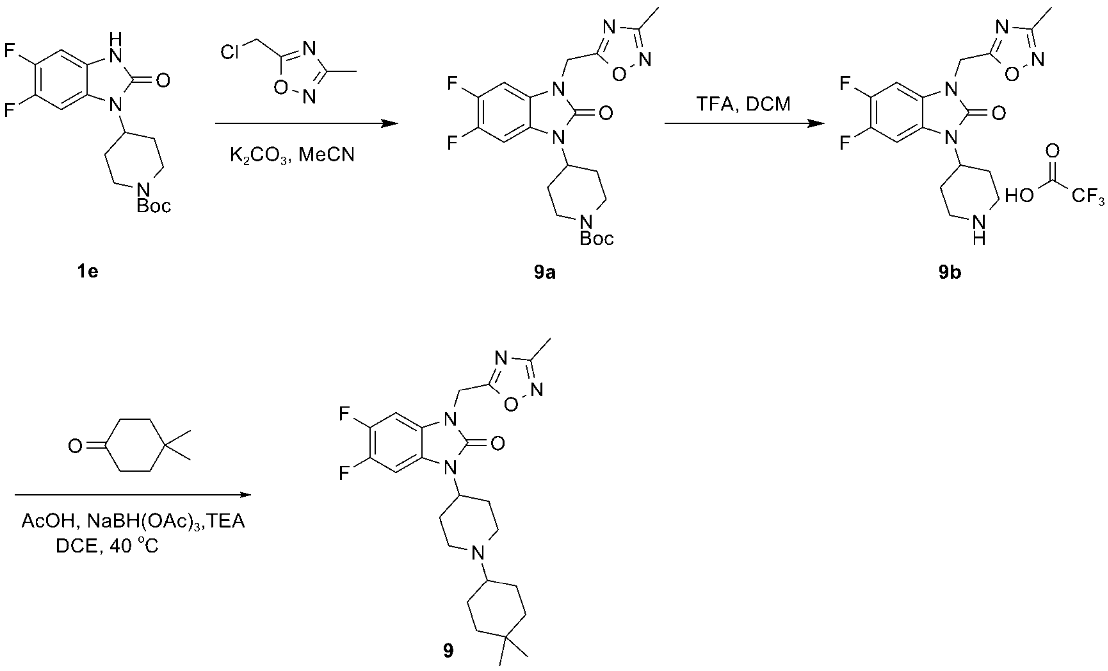


**1e** **9a** **9b** **9**

Synthesis of intermediate **9a**: To a clean, dry flask were added sequentially intermediate **1e** (1.50 g, 4.25 mmol), acetonitrile (15 mL), potassium carbonate (1.17 g, 8.50 mmol), and 3-methyl-5-(chloromethyl)-1,2,4-oxadiazole (0.73 g, 5.53 mmol). The mixture was stirred under reflux at 70 °C for 12 hours, and the reaction was confirmed to be complete by TLC. After the reaction was complete, the reaction system was cooled to room temperature and filtered, and the filter cake was washed with a small amount of acetonitrile. The filtrate was evaporated via a rotary evaporator under reduced pressure to remove the solvent. The crude product was purified by column chromatography (the mobile phase was dichloromethane and methanol, and the volume ratio was 20:1) to obtain 1.50 g of a yellow solid, with a yield of 78.57%. LC-MS (ESI) m/z: 450.23 $(M + H)^+$.

Synthesis of intermediate **9b**: To a clean, dry flask was added intermediate **9a** (1.20 g, 2.67 mmol), and anhydrous dichloromethane (8 mL) was added thereto. At 0 °C, trifluoroacetic acid (2 mL) was added dropwise. After

the addition was complete, the ice-water bath was removed, and the mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a crude product. Diethyl ether (4 mL) was added to the crude product, and the mixture was dispersed, stirred, filtered, and dried to obtain 1.24 g of a white solid. The white solid was used directly in the next experiment without further purification.

Synthesis of compound **9**: To a clean, dry flask were added intermediate **9b** (0.20 g, 0.43 mmol) and anhydrous 1,2-dichloroethane (4 mL). Triethylamine was added dropwise to adjust the pH to approximately 8, with stirring for 10 min. 4,4-dimethylcyclohexanone (136 mg, 1.08 mmol) was added. Acetic acid (0.12 g, 2.05 mmol) was added to adjust the pH to approximately 5. After stirring at room temperature for 2 hours, sodium triacetoxyborohydride (273 mg, 1.29 mmol) was added. The reaction was carried out at 40 °C for 24 hours, and the reaction was confirmed to be complete by TLC. To the reaction solution was added a saturated aqueous solution of sodium bicarbonate (5 mL), and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was purified by column chromatography (the mobile phase was dichloromethane and methanol, and the volume ratio was 30:1) to obtain 130 mg of a white solid, with a yield of 65.83%. LC-MS (ESI) m/z: 460.58 $(M + H)^+$; $^1H$ NMR (400 MHz, DMSO-$d_6$) δ 7.57 (dd, *J* = 11.0, 6.9 Hz, 1H), 7.47 (dd, *J* = 10.6, 7.0 Hz, 1H), 5.39 (s, 2H), 4.23 - 3.97 (m, 1H), 2.96 (d, *J* = 8.2 Hz, 2H), 2.39 - 2.17 (m, 8H), 1.75 - 1.51 (m, 4H), 1.39 (d, *J* = 11.8 Hz, 4H), 1.29 - 1.09 (m, 2H), 0.87 (d, *J* = 3.2 Hz, 6H).

**Example 9:** Synthesis of 5,6-difluoro-1-(l-(*cis*-4-isopropylcyclohexyl)piperidin-4-yl)-3-((3-methyl-1,2,4-oxadiazol-5-yl) methyl)-1,3-dihydro-2*H*-benzo[*d*]imidazol-2-one (**10**)

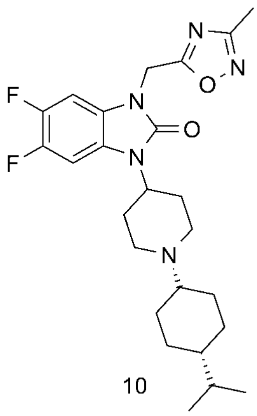

10

Intermediate **9b** (0.80 g, 1.73 mmol) and 4-isopropylcyclohexanone (605 mg, 4.32 mmol) were used as starting materials. This compound was synthesized following the synthesis method in Example 8. After preparation and separation (the separation conditions were the same as in Example 1), 200 mg of a white solid was obtained, with a yield of 24.42%. LC-MS (ESI) m/z: 474.45 $(M + H)^+$; $^1H$ NMR (400 MHz, DMSO-$d_6$) δ 7.54 (dd, *J* = 11.0, 7.0 Hz, 1H), 7.48 (dd, *J* = 10.6, 7.1 Hz, 1H), 5.40 (s, 2H), 4.19 - 4.04 (m, 1H), 3.07 (d, *J* = 11.1 Hz, 2H), 2.39 - 2.19 (m, 6H), 2.10 (t, *J* = 11.5 Hz, 2H), 1.68 (d, *J* = 11.6 Hz, 4H), 1.63 - 1.48 (m, 3H), 1.47 - 1.27 (m, 4H), 1.15 - 1.03 (m, 1H), 0.86 (d, *J* = 6.6 Hz, 6H).

**Example 10:** Synthesis of N-cyclopropyl-2-(3-(1-(4,4-dimethylcyclohexyl)piperidin-4-yl)-5,6-difluoro-2-oxo-2,3-dihydro -1*H*-benzo[*d*]imidazol-1-yl)acetamide (**11**)

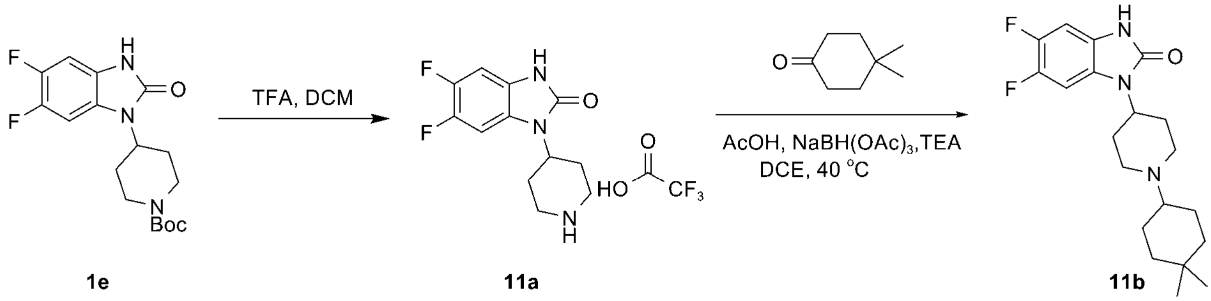


1e 11a 11b

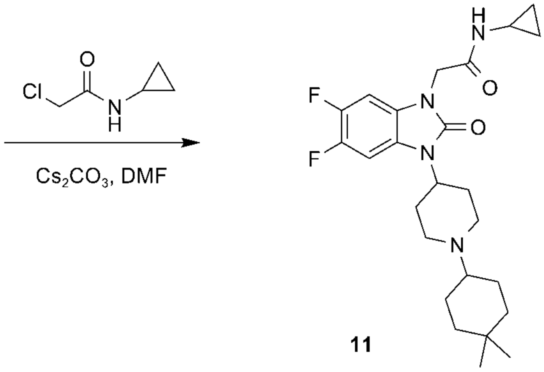


11

Synthesis of intermediate **11a**: To a clean, dry flask was added intermediate **1e** (2.00 g, 5.66 mmol), and anhydrous dichloromethane (16 mL) was added thereto. At 0 °C, trifluoroacetic acid (4 mL) was added dropwise. After the addition was complete, the ice-water bath was removed. The mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a crude product. Methyl tert-butyl ether (5 mL) was added to the crude product, and the mixture was dispersed, stirred, filtered, and dried to obtain 2.06 g of a white solid, with a yield of 98.94%. The white solid was used directly in the next experiment without further purification.

Synthesis of compound **11b**: To a clean, dry flask were added intermediate **11a** (2.06 g, 5.60 mmol) and anhydrous 1,2-dichloroethane (25 mL). Triethylamine was added dropwise to adjust the pH to approximately 8, with stirring for 10 minutes. 4,4-dimethylcyclohexanone (1.42 g, 11.20 mmol) was added. Acetic acid (0.51 g, 8.43 mmol) was added to adjust the pH to approximately 5. After stirring at room temperature for 2 h, sodium triacetoxyborohydride (3.57 g, 16.86 mmol) was added. The reaction was carried out at 40 °C for 24 hours, and the reaction was confirmed to be complete by TLC. To the reaction solution was added a saturated aqueous solution of sodium bicarbonate (20 mL), and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was purified by column chromatography (the mobile phase was dichloromethane and methanol, and the ratio was 15:1) to obtain 1.80 g of a white solid, with a yield of 88.39%. LC-MS (ESI) m/z: 364.26 $(M + H)^+$.

Synthesis of compound **11**: To a clean, dry flask were added sequentially intermediate **11b** (0.20 g, 0.55 mmol), N,N-dimethylformamide (5 mL), cesium carbonate (358 mg, 1.10 mmol) and 2-chloro-N-cyclopropylacetamide (96 mg, 0.72 mmol). After stirring at 65 °C for 12 hours, the reaction was confirmed to be complete by TLC. The reaction system was cooled to room temperature, water (10 mL) was added and the mixture was extracted three times with dichloromethane. The organic phases were combined, and it was evaporated via a rotary evaporator under reduced pressure to remove the solvent. The crude product was purified by column chromatography (the mobile phase was dichloromethane and methanol, and the volume ratio was 15:1) to obtain 200 mg of a yellow solid, with a yield of 79.05%. LC-MS (ESI) m/z: 461.61 $(M + H)^+$; $^1H$ NMR (400 MHz, DMSO-$d_6$) δ 8.28 (d, *J* = 4.2 Hz, 1H), 7.46 (dd, *J* = 10.9, 6.9 Hz, 1H), 7.27 (dd, *J* = 10.7, 7.1 Hz, 1H), 4.38 (s, 2H), 4.23 - 4.00 (m, 1H), 3.14 - 2.87 (m, 2H), 2.63 (tq, *J* = 7.7, 4.2 Hz, 2H), 2.43 - 2.11 (m, 4H), 1.78 - 1.50 (m, 4H), 1.41 (d, *J* = 12.5 Hz, 4H), 1.29 - 1.12 (m, 2H), 0.88 (d, *J* = 2.7 Hz, 6H), 0.62 (td, *J* = 7.0, 4.7 Hz, 2H), 0.51 - 0.35 (m, 2H).

**Example 11:** Synthesis of N-(cyclopropylmethyl)-2-(3-(1-(4,4-dimethylcyclohexyl)piperidin-4-yl)-5,6-difluoro-2-oxo-2,3 -dihydro-1*H*-benzo[*d*]imidazol-1-yl)acetamide (**12**)

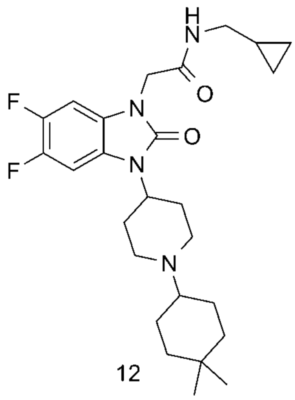

12

Intermediate **11b** (0.20 g, 0.55 mmol) and 2-chloro-N-(cyclopropylmethyl)acetamide (106 mg, 0.72 mmol) were used as starting materials. This compound was synthesized following the synthesis method in Example 10 to obtain 190 mg of a white solid, with a yield of 72.80%. LC-MS (ESI) m/z: 475.31 $(M + H)^+$; $^1H$ NMR (400 MHz, DMSO-$d_6$) δ 8.28 (t, *J* = 5.5 Hz, 1H), 7.46 (dd, *J* = 11.0, 7.0 Hz, 1H), 7.26 (dd, *J* = 10.7, 7.1 Hz, 1H), 4.44 (s, 2H), 4.18 - 4.01 (m, 1H), 3.05 - 2.79 (m, 4H), 2.41 - 2.12 (m, 5H), 1.76 - 1.51 (m, 4H), 1.50 - 1.31 (m, 4H), 1.27 - 1.09 (m, 2H), 0.99 - 0.78 (m, 7H), 0.48 - 0.30 (m, 2H), 0.26 - 0.02 (m, 2H).

**Example 12:** Synthesis of ethyl 2-(3-(1-(4,4-dimethylcyclohexyl)piperidin-4-yl)-5,6-difluoro-2-oxo-2,3-dihydro-1*H*-benzo[*d*]i midazol-1-yl)acetate (**13**)

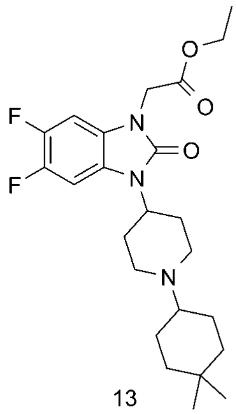

13

Intermediate **11b** (0.20 g, 0.55 mmol) and ethyl chloroacetate (88 mg, 0.72 mmol) were used as starting materials. This compound was synthesized following the synthesis method in Example 10 to obtain 210 mg of a white solid, with a yield of 85.02%. LC-MS (ESI) m/z: 450.27 $(M + H)^+$; $^1H$ NMR (400 MHz, DMSO-$d_6$) δ 7.50 (dd, *J* = 11.0, 7.0 Hz, 1H), 7.45 (dd, *J* = 10.7, 7.1 Hz, 1H), 4.68 (s, 2H), 4.14 (q, *J* = 7.1 Hz, 2H), 4.11 - 4.04 (m, 1H), 2.96 (d, *J* = 8.1 Hz, 2H), 2.40 - 2.15 (m, 5H), 1.73 - 1.51 (m, 4H), 1.40 (d, *J* = 11.6 Hz, 4H), 1.28 - 1.09 (m, 5H), 0.88 (d, *J* = 2.9 Hz, 6H).

**Example 13:** Synthesis of 2-(3-(1-(4,4-dimethylcyclohexyl)piperidin-4-yl)-5,6-difluoro-2-oxo-2,3-dihydro-1*H*-benzo[*d*]i midazol-1-yl)acetamide (**14**)

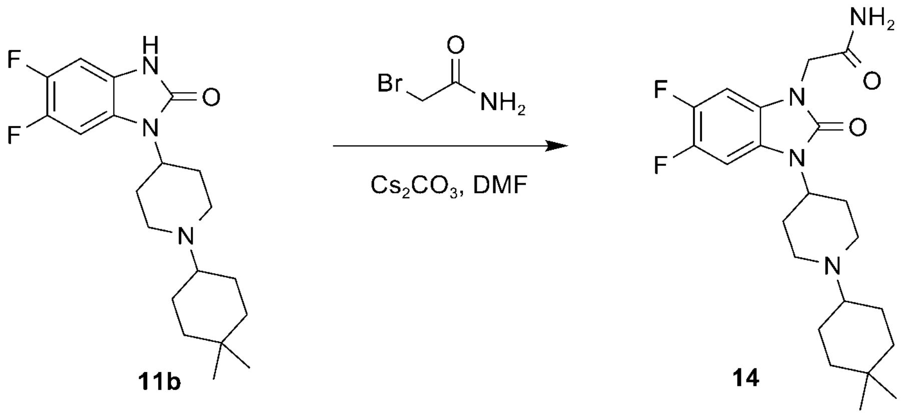


11b 14

Synthesis of compound **14**: To a clean, dry flask were added sequentially intermediate **11b** (0.20 g, 0.55 mmol), N,N-dimethylformamide (5 mL), cesium carbonate (358 mg, 1.10 mmol), and 2-bromoacetamide (98 mg, 0.72 mmol). After stirring at 65 °C for 12 hours, the reaction was confirmed to be complete by TLC. The reaction system was cooled to room temperature, and water (10 mL) was added. The mixture was extracted three times with dichloromethane. The organic phases were combined, and it was evaporated via a rotary evaporator under reduced pressure to remove the solvent. The crude product was purified by column chromatography (the mobile phase was dichloromethane and methanol, and the volume ratio was 15:1) to obtain 200 mg of a yellow solid, with a yield of 86.48%. LC-MS (ESI) m/z: 421.27 $(M + H)^+$; $^1H$ NMR (400 MHz, DMSO-$d_6$) δ 7.62 (s, 1H), 7.45 (dd, *J* = 11.0, 7.0 Hz, 1H), 7.27 (dd, *J* = 10.7, 7.2 Hz, 1H), 7.23 (s, 1H), 4.40 (s, 2H), 4.18 - 3.97 (m, 1H), 2.96 (d, *J* = 8.3 Hz, 2H), 2.39 - 2.12 (m, 5H), 1.76 - 1.50 (m, 4H), 1.49 - 1.30 (m, 4H), 1.29 - 1.07 (m, 2H), 0.88 (d, *J* = 2.8 Hz, 6H).

**Example 14:** Synthesis of 2-(5,6-difluoro-2-oxo-3-(1-(spiro[2.5]oct-6-yl)piperidin-4-yl)-2,3-dihydro-1*H*-benzo[*d*]imidazo l-1-yl)-N-methylacetamide (**15**)

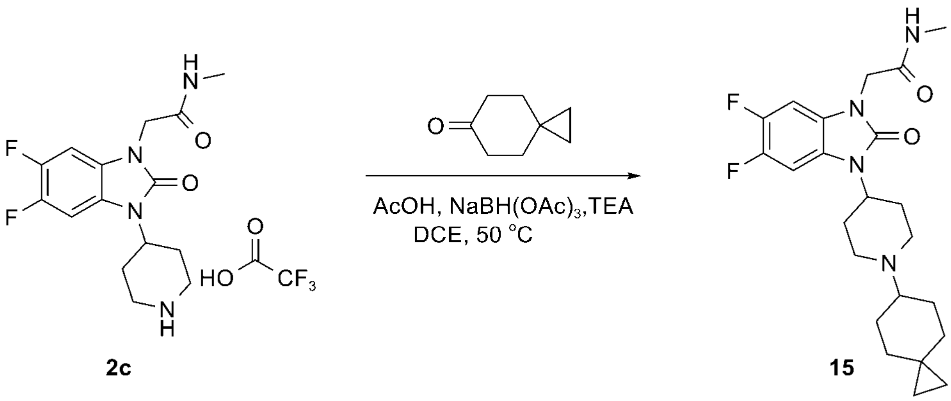


2c 15

To a clean, dry flask were added intermediate **2c** (0.30 g, 0.68 mmol) and anhydrous 1,2-dichloroethane (4 mL). Triethylamine was added dropwise to adjust the pH to approximately 8, with stirring for 10 minutes. Spiro[2.5]octan-6-one (0.21 g, 1.70 mmol) was added. Acetic acid was added to adjust the pH to approximately 5. After stirring at room temperature for 2 h, sodium triacetoxyborohydride (0.43 g, 2.04 mmol). The reaction was carried out at 50 °C for 24 hours, and the reaction was confirmed to be complete by TLC. The mixture was evaporated via a rotary evaporator under reduced pressure to remove the solvent. The crude product was purified by column chromatography (the mobile phase was dichloromethane and methanol, and the volume ratio was 20:1) to obtain 180 mg of a white solid, with a yield of 61.22%. LC-MS (ESI) m/z: 433.56 $(M + H)^+$; $^1H$ NMR (400 MHz, $CDCl_3$) δ 7.56 - 7.38 (m, 1H), 6.92 (dd, *J* = 9.4, 6.6 Hz, 1H), 6.08 (s, 1H), 4.65 - 4.49 (m, 1H), 4.43 (s, 2H), 3.67 - 3.35 (m, 2H), 2.94 - 2.55 (m, 6H), 2.08 - 1.91 (m, 4H), 1.91 - 1.75 (m, 4H), 1.60 (t, *J* = 12.2 Hz, 2H), 1.02 (d, *J* = 13.3 Hz, 2H), 0.44 - 0.31 (m, 2H), 0.30 - 0.13 (m, 2H).

**Example 15:** Synthesis of 2-(3-(1-(4,4-dimethylcyclohexyl)piperidin-4-yl)-5,6-difluoro-2-oxo-2,3-dihydro-1*H*-benzo[*d*]imidazol-1-yl)-N-methylpropanamide (**16**)

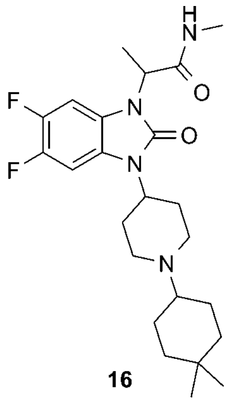

**16**

Intermediate **11b** (0.20 g, 0.55 mmol) and 2-chloro-N-methylpropionamide (88 mg, 0.72 mmol) were used as starting materials. This compound was synthesized following the synthesis method in Example 10 to obtain 180 mg of a white solid, with a yield of 72.87%. LC-MS (ESI) m/z: 449.62 $(M + H)^+$; $^1H$ NMR (400 MHz, DMSO-$d_6$) δ 8.02 (d, *J* = 4.7 Hz, 1H), 7.46 (dd, *J* = 11.0, 7.1 Hz, 1H), 7.26 (dd, *J* = 11.0, 7.2 Hz, 1H), 4.98 (q, *J* = 7.2 Hz, 1H), 4.22 - 4.00 (m, 1H), 2.96 (d, *J* = 8.1 Hz, 2H), 2.58 (d, *J* = 4.5 Hz, 3H), 2.29 (t, *J* = 14.4 Hz, 5H), 1.75 - 1.54 (m, 4H), 1.50 (d, *J* = 7.2 Hz, 3H), 1.47 - 1.33 (m, 4H), 1.19 (p, *J* = 10.7, 9.2 Hz, 2H), 0.88 (d, *J* = 2.9 Hz, 6H).

**Example 16:** Synthesis of 2-(3-(1-(4,4-dimethylcyclohexyl)piperidin-4-yl)-5,6-difluoro-2-oxo-2,3-dihydro-1*H*-benzo[*d*]imidazol-1-yl)-N-(2-hydroxyethyl)acetamide (**17**)

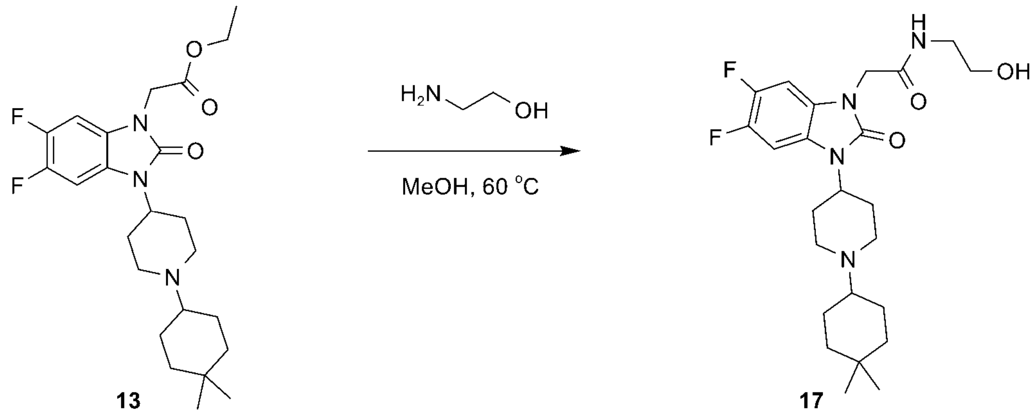


**13** **17**

To a clean, dry flask were added sequentially compound **13** (0.20 g, 0.45 mmol), ethanolamine (82 mg, 1.35 mmol) and methanol (2 mL). The mixture was heated to 60 °C and stirred overnight, and the reaction was confirmed to be essentially complete by TLC. The reaction system was cooled to room temperature. The mixture was evaporated via a rotary evaporator under reduced pressure to remove the solvent. The crude product was purified by column chromatography (the mobile phase was dichloromethane and methanol, and the volume ratio was 20:1) to obtain 130 mg of a white solid, with a yield of 63.40%, LC-MS (ESI) m/z: 465.58 $(M + H)^+$; $^1H$ NMR (400 MHz, DMSO-$d_6$) δ 8.21 (t, *J* = 5.6 Hz, 1H), 7.46 (dd, *J* = 11.0, 6.9 Hz, 1H), 7.25 (dd, *J* = 10.7, 7.1 Hz, 1H), 4.71 (t, *J* = 5.4 Hz, 1H), 4.44 (s, 2H), 4.08 (s, 1H), 3.42 (q, *J* = 5.9 Hz, 2H), 3.14 (q, *J* = 5.9 Hz, 2H), 2.96 (d, *J* = 8.3 Hz, 2H), 2.41 - 2.10 (m, 5H), 1.74 - 1.51 (m, 4H), 1.40 (d, *J* = 11.9 Hz, 4H), 1.30 - 1.06 (m, 2H), 0.88 (d, *J* = 2.8 Hz, 6H).

**Example 17:** Synthesis of 1-(1-(4,4-dimethylcyclohexyl)piperidin-4-yl)-5,6-difluoro-3-((methylsulfonyl)methyl)-1,3-dihydro-2*H*-benzo[*d*]imidazol-2-one (**18**)

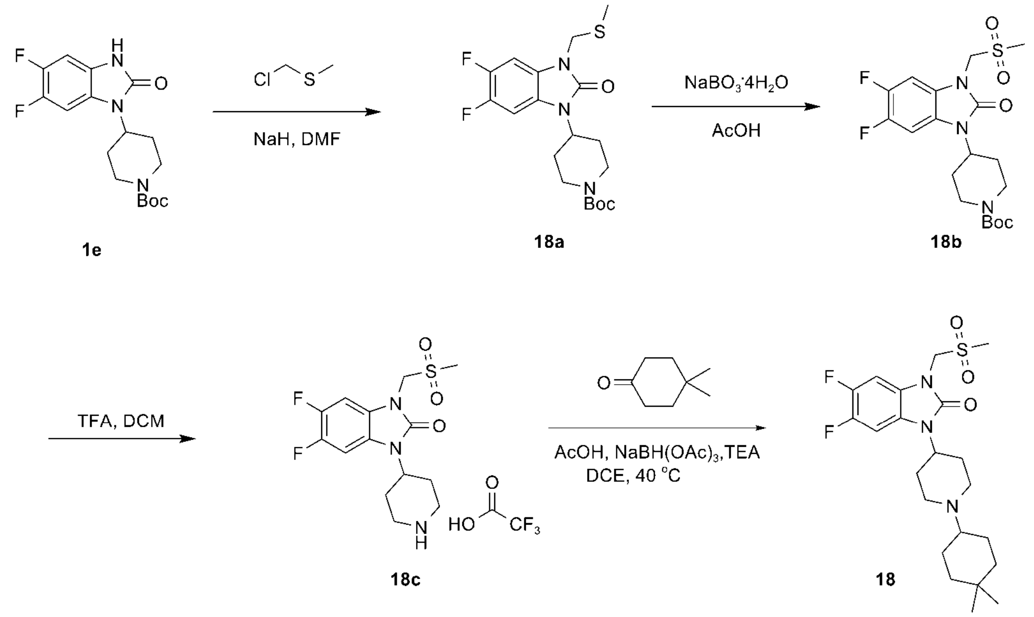


**1e** **18a** **18b** **18c** **18**

Synthesis of intermediate **18a**: To a clean, dry flask were added sequentially intermediate **1e** (2.00 g, 5.67 mmol) and anhydrous DMF (25 mL). After being purged with nitrogen three times, 60% NaH by mass (0.68 g, 17.01 mmol) was slowly added under ice bath condition. The mixture was heated to room temperature and stirred for 30 minutes. Chloromethyl methyl sulfide (1.10 g, 11.34 mmol) was added. Stirring was continued at room temperature for 16 hours, and the reaction was confirmed to be essentially complete by TLC. Saturated aqueous ammonium chloride solution was added to the reaction solution. The mixture was extracted with ethyl acetate. After the organic phase was washed with saturated brine and dried over anhydrous sodium sulfate, it was evaporated via a rotary evaporator under reduced pressure to remove the solvent. The crude product was purified by column chromatography (the mobile phase was dichloromethane and methanol, the volume ratio was 1:1) to obtain 1.80 g of a yellow solid, with a yield of 76.72%.

Synthesis of intermediate **18b**: To a clean, dry flask were added sequentially intermediate **18a** (1.00 g, 2.42 mmol) and glacial acetic acid (15 mL). Sodium perborate tetrahydrate (1.12 g, 7.26 mmol) was slowly added with stirring at room temperature. After stirring at room temperature for 16 hours, it was confirmed by TLC that the starting material had reacted substantially to completion. An aqueous solution of potassium carbonate was added to the reaction solution. The mixture was extracted with ethyl acetate. After the organic phase was washed with saturated brine and dried over anhydrous sodium sulfate, it was evaporated via a rotary evaporator under reduced pressure to remove the solvent, to obtain 0.95 g of a yellow solid, with a yield of 88.02%.

Synthesis of intermediate **18c**: To a clean, dry flask was added intermediate **18b** (0.52 g, 1.17 mmol), and anhydrous dichloromethane (5 mL) was added thereto. At 0 °C, trifluoroacetic acid (1 mL) was added dropwise. After the addition was complete, the ice-water bath was removed, and the mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a crude product. Methyl tert-butyl ether (5 mL) was added to the crude product, and the mixture was dispersed, stirred, filtered, and dried to obtain 520 mg of a white solid, with a yield of 96.58%. The white solid was used directly in the next experiment without further purification.

Synthesis of compound **18**: To a clean, dry flask were added intermediate **18c** (0.52 g, 1.13 mmol) and anhydrous 1,2-dichloroethane (5 mL). Triethylamine was added dropwise to adjust the pH to approximately 8, with stirring for 10 min. 4,4-dimethylcyclohexanone (214 mg, 1.69 mmol) was added. Acetic acid was added to adjust the pH to approximately 5. After stirring at room temperature for 2 h, sodium triacetoxyborohydride (599 mg, 2.83 mmol) was added. The reaction was carried out at 40 °C for 24 hours, and the reaction was confirmed to be complete by TLC. To the reaction solution was added a saturated aqueous solution of sodium bicarbonate (20 mL), and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was

purified by column chromatography (the mobile phase was dichloromethane and methanol, and the volume ratio was 40:1) to obtain 300 mg of a white solid, with a yield of 58.18%. LC-MS (ESI) m/z: 456.50(M + H)$^+$; $^1$H NMR (400 MHz, DMSO-$d_6$) δ 7.60 (td, *J* = 11.4, 7.0 Hz, 2H), 5.40 (s, 2H), 4.72 - 4.45 (m, 1H), 3.61 (d, *J* = 11.9 Hz, 2H), 3.28 - 3.11 (m, 3H), 3.06 (s, 3H), 2.62 (q, *J* = 12.4 Hz, 2H), 2.03 (d, *J* = 13.6 Hz, 2H), 1.86 (d, *J* = 11.9 Hz, 2H), 1.64 (q, *J* = 12.5 Hz, 2H), 1.50 (d, *J* = 13.1 Hz, 2H), 1.27 (dd, *J* = 14.9, 11.5 Hz, 2H), 0.92 (d, *J* = 1.5 Hz, 6H).

**Example 18:** Synthesis of 5,6-difluoro-1-((methylsulfonyl)methyl)-3-(1-(spiro[2.5]oct-6-yl)piperidin-4-yl)-1,3-dihydro-2*H*-benzo[*d*]imidazol-2-one (**20**)

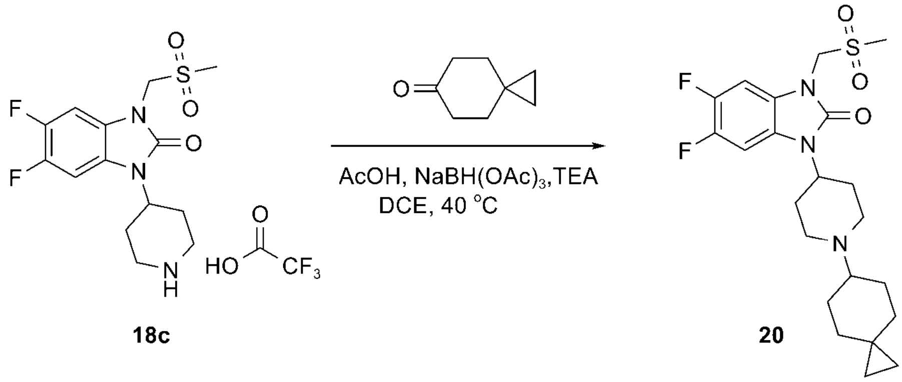


**18c** **20**

To a clean, dry flask were added intermediate **18c** (0.30 g, 0.65 mmol) and anhydrous 1,2-dichloroethane (4 mL). Triethylamine was added dropwise to adjust the pH to approximately 8, with stirring for 10 min. Spiro[2.5]octan-6-one (202 mg, 1.63 mmol) was added. Acetic acid was added to adjust the pH to approximately 5. After stirring at room temperature for 2 h, sodium triacetoxyborohydride (413 mg, 1.95 mmol) was added. The reaction was carried out at 40 °C for 24 hours, and the reaction was confirmed to be complete by TLC. To the reaction solution was added a saturated aqueous solution of sodium bicarbonate (5 mL) and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was purified by column chromatography (the mobile phase was dichloromethane and methanol, and the volume ratio was 40:1) to obtain 120 mg of a white solid, with a yield of 40.71%. LC-MS (ESI) m/z: 454.55(M + H)$^+$; $^1$H NMR (400 MHz, $CDCl_3$) δ 7.66 (s, 1H), 7.15 (dd, *J* = 9.4, 6.6 Hz, 1H), 5.01 (s, 2H), 4.71 - 4.44 (m, 1H), 3.72 - 3.43 (m, 2H), 3.29 - 3.10 (m, 1H), 2.98 (s, 3H), 2.96 - 2.81 (m, 3H), 2.20 - 2.06 (m, 2H), 1.99 (d, *J* = 11.9 Hz, 2H), 1.89 (t, *J* = 13.0 Hz, 2H), 1.64 (t, *J* = 12.0 Hz, 3H), 1.04 (d, *J* = 13.4 Hz, 2H), 0.45 - 0.35 (m, 2H), 0.29 - 0.19 (m, 2H).

**Example 19:** Synthesis of 1-(1-(4,4-dimethylcyclohexyl)piperidin-4-yl)-5,6-difluoro-3-(2(methylsulfonyl)ethyl)-1,3-dihyd ro-2*H*-benzo[*d*]imidazol-2-one (**21**)

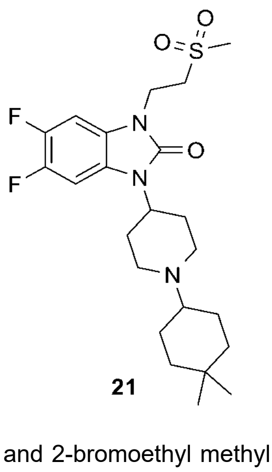

**21**

Intermediate **11b** (0.20 g, 0.55 mmol) and 2-bromoethyl methyl sulfone (135 mg, 0.72 mmol) were used as

starting materials. This compound was synthesized following the synthesis method in Example 10 to obtain 220 mg of a white solid, with a yield of 85.27%. LC-MS (ESI) m/z: 470.49 (M + H)$^+$.

**Example 20:** Synthesis of N-(2-(3-(1-(4,4-dimethylcyclohexyl)piperidin-4-yl)-5,6-difluoro-2-oxo-2,3-dihydro-1*H*-benzo [ *d*]imidazol-1-yl)ethyl)acetamide (**22**)

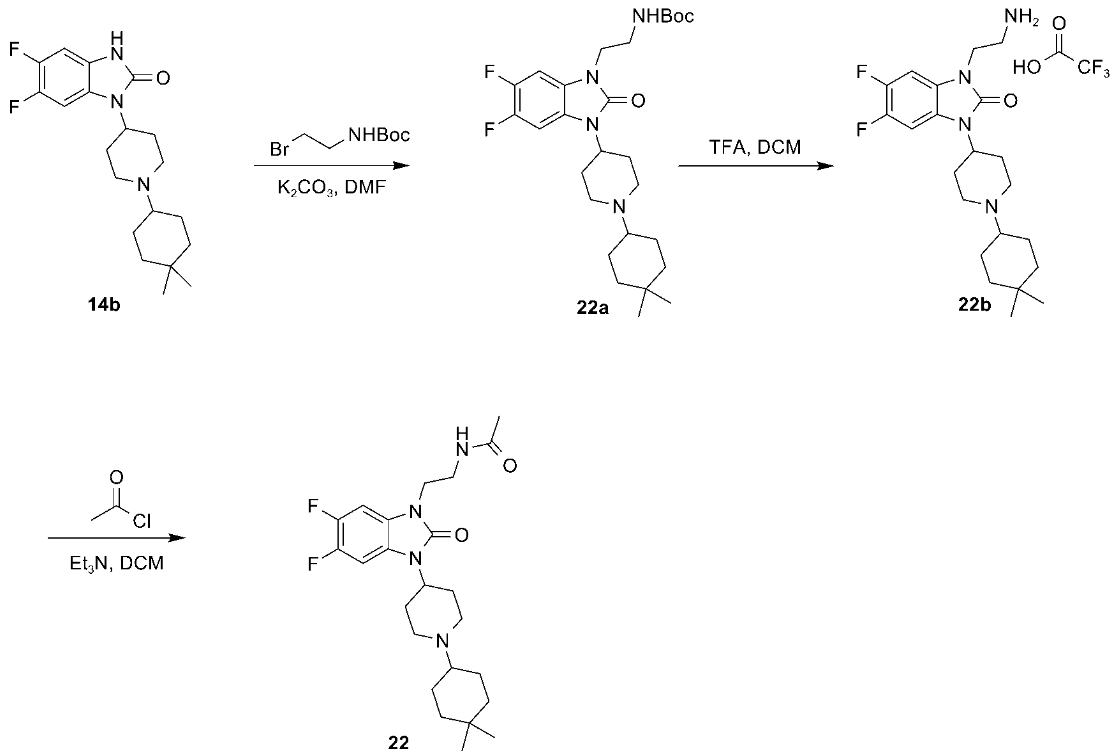


**14b** **22a** **22b** **22**

Synthesis of intermediate **22a**: To a clean, dry flask were added sequentially intermediate **14b** (300 mg, 0.83 mmol), DMF (2 mL), potassium carbonate (229 mg, 1.66 mmol) and N-Boc-bromoethylamine (242 mg, 1.08 mmol). The reaction was stirred at 50 °C for 12 hours and was confirmed to be complete by TLC. After the reaction was complete, the reaction system was cooled to room temperature, and was diluted with the addition of water. Then the mixture was extracted with ethyl acetate and layered. The organic phase was washed with water, washed with saturated brine, and dried over anhydrous sodium sulfate, then filtered, and the filtrate was evaporated via a rotary evaporator under reduced pressure to remove the solvent. The crude product was purified by column chromatography (the mobile phase was petroleum ether and ethyl acetate, and the volume ratio was 1:1) to obtain 377 mg of a yellow solid, with a yield of 89.65%. LC-MS (ESI) m/z: 507.46 (M + H)$^+$.

Synthesis of intermediate **22b**: To a clean, dry flask was added intermediate **22a** (377 mg, 0.75 mmol), and anhydrous dichloromethane (4 mL) was added thereto. At 0 °C, trifluoroacetic acid (1 mL) was added dropwise. After the addition was complete, the ice-water bath was removed, and the mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a crude product. Diethyl ether was added to the crude product, and the mixture was dispersed, stirred, filtered, and dried to obtain 390 mg of a yellow solid, with a yield of 100%. The product was used directly in the next experiment without further purification.

Synthesis of compound **22**: To a clean, dry flask were added intermediate **22b** (390 mg, 0.75 mmol) and anhydrous dichloromethane (4 mL), and triethylamine (227 mg, 2.25 mmol) were added thereto. Acetyl chloride (71 mg, 0.90 mmol) was slowly added under an ice-water bath. Continue stirring at room temperature for 3 hours, and the reaction was confirmed to be essentially complete by TLC. The mixture was evaporated via a rotary evaporator under reduced pressure to remove the solvent. The crude product was purified by column chromatography (the mobile phase was dichloromethane and methanol, and the volume ratio was 20:1) to obtain 64 mg of a white solid, with a yield of 19.04%. LC-MS (ESI) m/z: 449.60 (M + H)$^+$; $^1$H NMR (400 MHz, DMSO-$d_6$) δ 7.93 (t, *J* = 5.9 Hz, 1H), 7.44 (dd, *J* = 11.0, 7.0 Hz, 1H), 7.30 (dd, *J* = 10.7, 7.1 Hz, 1H), 4.19 - 3.95 (m, 1H), 3.81 (t, *J* = 6.1 Hz, 2H), 3.27 (q, *J* = 6.0 Hz, 2H), 2.95 (s, 1H), 2.41 - 2.08 (m,

5H), 1.68 (s, 3H), 1.67 - 1.52 (m, 4H), 1.48 - 1.30 (m, 4H), 1.29 - 1.07 (m, 2H), 0.88 (d, *J* = 2.9 Hz, 6H).

**Example 21:** Synthesis of 2-(3-(l-(4,4-dimethylcyclohexyl)piperidin-4-yl)-5,6-difluoro-2-thio-2,3-dihydro-1*H*-benzo[*d*]imidazol-1-yl)-N,N-dimethylacetamide (**23**)

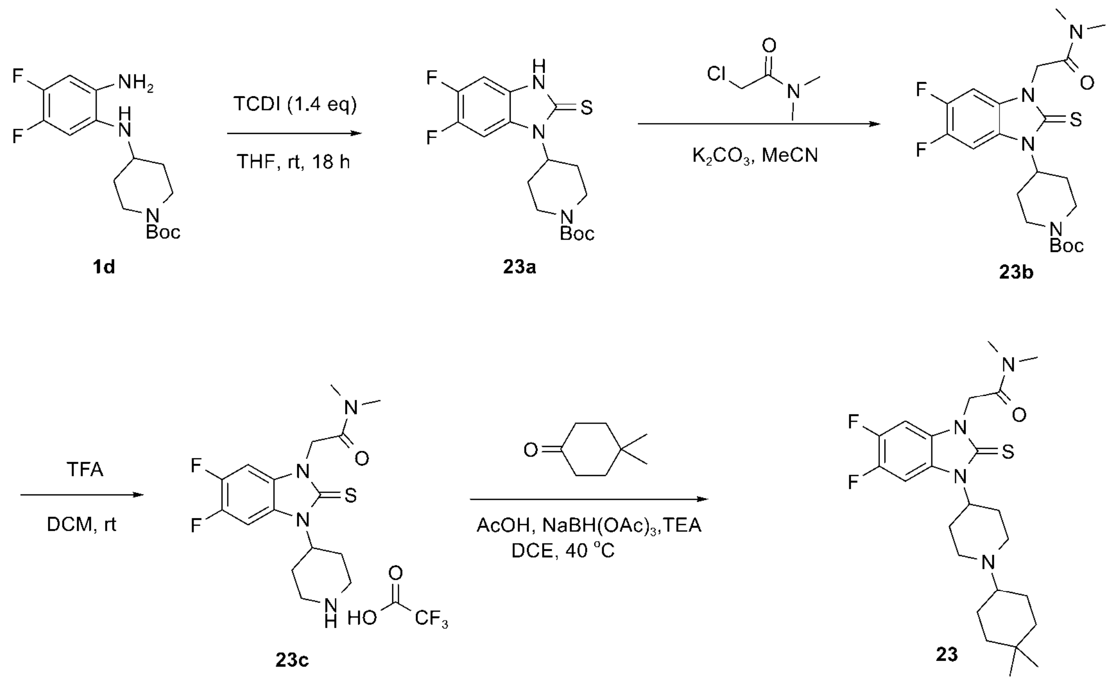


Synthesis of intermediate **23a**: Intermediate **1d** (2.00 g, 6.11 mmol) and anhydrous tetrahydrofuran (15 mL) were paced in a clean, dry flask. After being purged with nitrogen, N,N'-thiocarbonyldiimidazole (TCDI) (1.52 g, 8.55 mmol) was added at a temperature of 0 °C. The mixture was stirred at room temperature for 18 hours, and the reaction was quenched after the reaction was confirmed to be complete by TLC. The reaction solution was concentrated under reduced pressure. Saturated aqueous solution of $NaHCO_3$ was added. The mixture was extracted with ethyl acetate, and the organic phase was dried over anhydrous sodium sulfate and then filtered. The filtrate was concentrated via a rotary evaporator under reduced pressure. The crude product was purified by column chromatography (the mobile phase was petroleum ether and ethyl acetate, and the volume ratio was 1:1) to obtain 2.20 g of a yellow solid, with a yield of 97.54%. LC-MS (ESI) m/z: 370.28 $(M + H)^+$.

Synthesis of intermediate **23b**: To a clean, dry flask were added sequentially intermediate **23a** (1.00 g, 2.71 mmol), acetonitrile (10 mL), potassium carbonate (748 mg, 5.42 mmol), and 2-chloro-N,N-dimethylacetamide (428 mg, 3.52 mmol). The reaction was stirred at 70 °C for 12 hours and confirmed to be complete by TLC. After the reaction was complete, the reaction system was cooled to room temperature and filtered, and the filter cake was washed with a small amount of acetonitrile. The filtrate was concentrated via a rotary evaporator under reduced pressure, and the crude product was purified by column chromatography (the mobile phase was dichloromethane and methanol, and the volume ratio was 60:1) to obtain 1.20 g of a yellow solid, with a yield of 97.42%. LC-MS (ESI) m/z: 455.26 $(M + H)^+$.

Synthesis of intermediate **23c**: To a clean, dry flask was added intermediate **23b** (1.20 g, 2.64 mmol), and anhydrous dichloromethane (8 mL) was added thereto. At 0 °C, trifluoroacetic acid (2 mL) was added dropwise. After the addition was complete, the ice-water bath was removed, and the mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a light brown oily crude product. Methyl tert-butyl ether was added to the crude product, and the mixture was dispersed, stirred, filtered, and dried to obtain 1.24 g of a white solid, with a yield of 100%. The white solid was used directly in the next experiment without further purification.

Synthesis of compound **23**: To a clean, dry flask were added intermediate **23c** (1.24 g, 2.64 mmol) and anhydrous 1,2-dichloroethane (10 mL). Triethylamine was added dropwise to adjust the pH to approximately 8, with stirring for 10 min. 4,4-dimethylcyclohexanone (678 mg, 5.38 mmol) was added. Acetic acid was added to adjust the pH to approximately 5. After stirring at room temperature for 2 hours, sodium triacetoxyborohydride (1.71 g, 8.07 mmol) was added. The reaction

was carried out at 40 °C for 16 hours, and the reaction was confirmed to be complete by TLC. To the reaction solution was added a saturated aqueous solution of sodium bicarbonate, and the mixture was layered. The organic phase was washed with saturated brine and dried over anhydrous sodium sulfate, and then filtered. The filtrate was concentrated via a rotary evaporator under reduced pressure. The crude product was purified by column chromatography (the mobile phase was dichloromethane and methanol, and the volume ratio was 40:1) to obtain 800 mg of a white solid, with a yield of 65.22%. LC-MS (ESI) m/z: 465.55 $(M + H)^+$; $^1H$ NMR (400 MHz, $CDCl_3$) δ 7.71 (s, 1H), 7.36 (dd, *J* = 10.3, 7.2 Hz, 1H), 4.71 - 4.56 (m, 1H), 4.38 (s, 2H), 3.70 (s, 2H), 3.16 (s, 3H), 2.99 (s, 8H), 2.18 - 2.04 (m, 2H), 1.96 (d, *J* = 11.8 Hz, 2H), 1.83 - 1.51 (m, 4H), 1.34 (t, *J* = 13.3 Hz, 2H), 0.96 (s, 3H), 0.95 (s, 3H).

**Example 22:** Synthesis of 2-(3-(l-(4,4-dimethylcyclohexyl)piperidin-4-yl)-5,6-difluoro-2-thio-2,3-dihydro-1*H*-benzo[*d*]imidazol-1-yl)-N-methylacetamide (**25**)

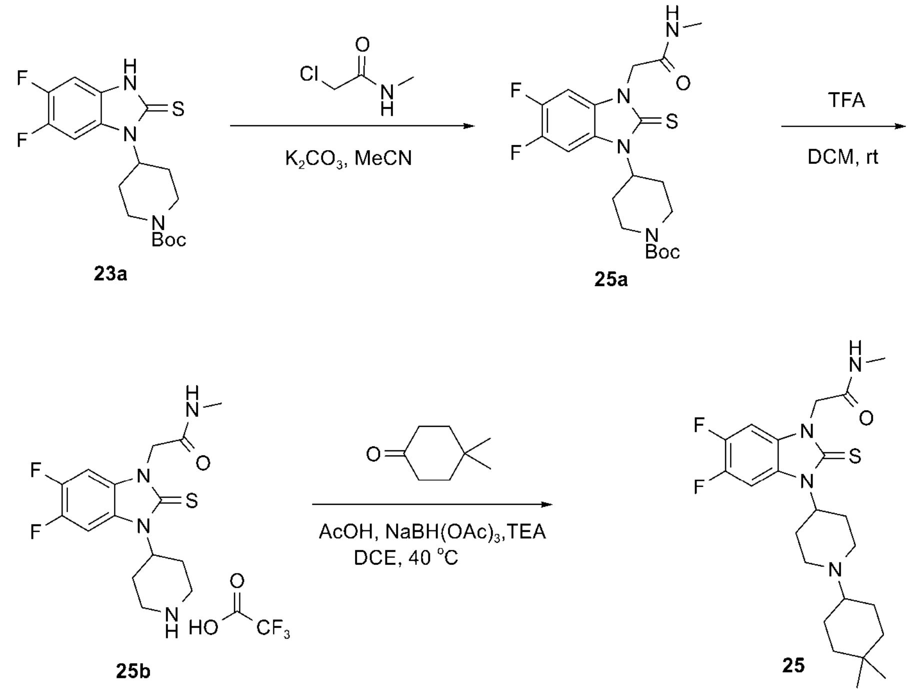


23a, 25a, 25b, 25

Synthesis of intermediate **25a**: To a clean, dry flask were added sequentially intermediate **23a** (1.00 g, 2.71 mmol), acetonitrile (10 mL), potassium carbonate (748 mg, 5.42 mmol) and 2-chloro-N-methylacetamide (379 mg, 3.52 mmol). The reaction was stirred at 70 °C for 12 hours and confirmed to be complete by TLC. After the reaction was complete, the reaction system was cooled to room temperature and filtered, and the filter cake was washed with a small amount of acetonitrile. The filtrate was concentrated via a rotary evaporator under reduced pressure. The crude product was purified by column chromatography (the mobile phase was dichloromethane and methanol, and the volume ratio was 40:1) to obtain 1.10 g of a yellow solid, with a yield of 92.13%.

Synthesis of intermediate **25b**: To a clean, dry flask was added intermediate **25a** (1.10 g, 2.50 mmol), and anhydrous dichloromethane (8 mL) was added thereto. At 0 °C, trifluoroacetic acid (2 mL) was added dropwise. After the addition was complete, the ice-water bath was removed, and the mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a light brown oily crude product. Methyl tert-butyl ether was added to the crude product, and the mixture was dispersed, stirred, filtered, and dried to obtain 1.14 g of a white solid, with a yield of 100%. The white solid was used directly in the next experiment without further purification.

Synthesis of compound **25**: To a clean, dry flask were added intermediate **25b** (1.14 g, 2.50 mmol) and anhydrous 1,2-dichloroethane (10 mL). Triethylamine was added dropwise to adjust the pH to approximately 8, with stirring for 10 min. 4,4-dimethylcyclohexanone (678 mg, 5.38 mmol) was added. Acetic acid was added to adjust the pH to approximately 5. After stirring at room temperature for 2 hours, sodium triacetoxyborohydride (1.59 g, 7.50 mmol) was added. The reaction was carried out at 40 °C for 16 hours, and the reaction was confirmed to be complete by TLC. To the reaction solution was added a saturated aqueous solution of sodium bicarbonate, and the mixture was layered. The organic phase was washed

with saturated brine and dried over anhydrous sodium sulfate and then filtered. The filtrate was concentrated via a rotary evaporator under reduced pressure. The crude product was purified by column chromatography (the mobile phase was dichloromethane and methanol, and the ratio was 40:1) to obtain 860 mg of a white solid, with a yield of 76.38%. LC-MS (ESI) m/z: 451.54 $(M + H)^+$; $^1H$ NMR (400 MHz, $CDCl_3$) δ 7.91 - 7.59 (m, 2H), 7.38 (dd, *J* = 10.2, 7.1 Hz, 1H), 4.52 - 4.22 (m, 1H), 3.90 (s, 2H), 3.81 - 3.46 (m, 2H), 3.31 - 2.84 (m, 3H), 2.80 (d, *J* = 4.8 Hz, 3H), 2.12 - 2.02 (m, 2H), 2.00 - 1.88 (m, 2H), 1.87 - 1.64 (m, 4H), 1.59 (d, *J* = 13.6 Hz, 2H), 1.33 (t, *J* = 13.3 Hz, 2H), 0.96 (s, 3H), 0.94 (s, 3H).

**Example 23:** Synthesis of 2-(6,7-difluoro-1-(1-(*cis*-4-isopropylcyclohexyl)piperidin-4-yl)-2-oxo-1,4-dihydroquinazoline-3 (2*H*)-yl)-N,N-dimethylacetamide (**26**)

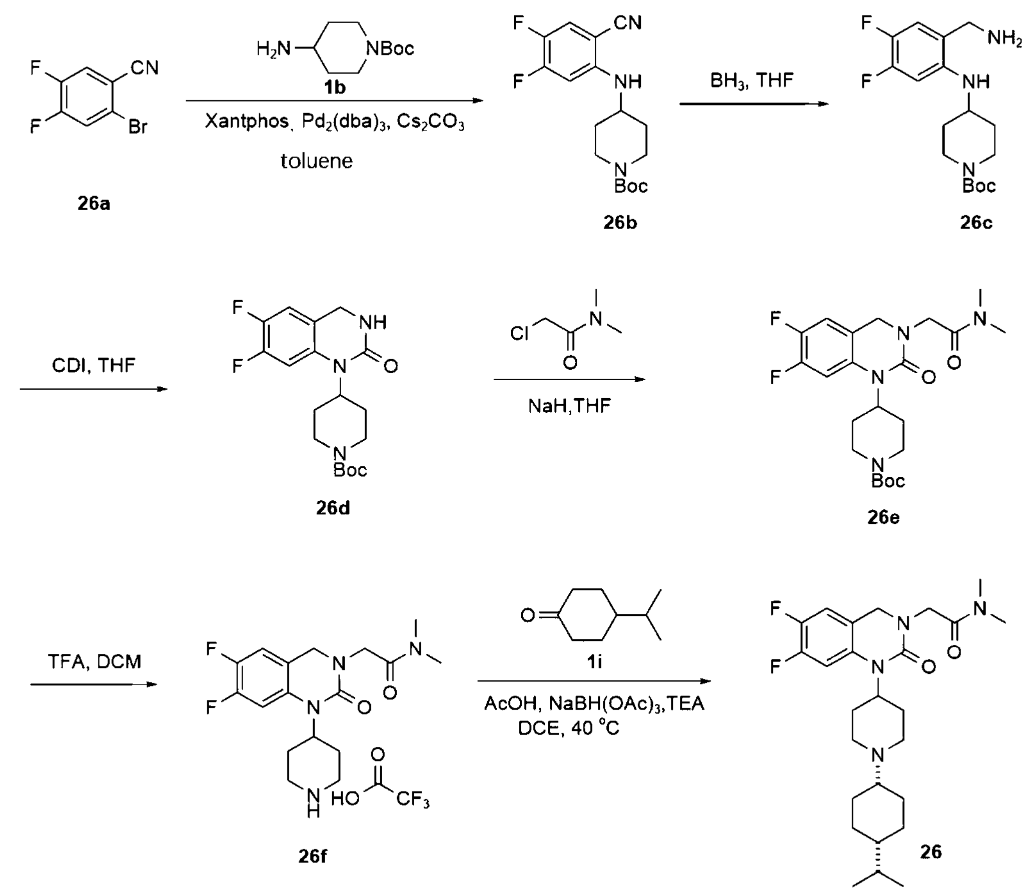


Synthesis of intermediate **26b:** To a clean, dry flask were added sequentially 4,5-difluoro-2-bromobenzonitrile (**26a**) (8.90 g, 40.83 mmol), Xantphos ligand (1.19 g, 2.06 mmol), and tris(dibenzylideneacetone)dipalladium (0.94 g, 1.03 mmol). After being purged with nitrogen, toluene (100 mL) was added. Under stirring at room temperature, anhydrous cesium carbonate (18.62 g, 57.16 mmol) and 4-amino-1-tert-butoxycarbonylpiperidine (**1b**) (9.81 g, 49.00 mmol) were added sequentially. The mixture was heated to 100 °C to react. The reaction was monitored by TLC until the starting material **26a** had reacted substantially to completion. After cooling to room temperature, the reaction solution was filtered, and the filter cake was washed with dichloromethane. The filtrate was concentrated, and the crude product was purified by column chromatography (the mobile phase was petroleum ether and ethyl acetate, and the volume ratio was 6:1) to obtain 13.10 g of a yellow solid, with a yield of 95.13%. LC-MS (ESI) m/z: 338.09 $(M + H)^+$.

Synthesis of intermediate **26c:** To a clean, dry flask were added sequentially intermediate 26b (13.10 g, 38.83 mmol) and THF (40 mL). After being purged with nitrogen, $BH_3$ in THF solution (1.0 mmol/mL, 117 mL) was added dropwise at 0 °C. The reaction solution was moved to room temperature and stirred for 16 hours, and the reaction was confirmed to be complete by TLC. The reaction was quenched by dropwise addition of methanol at 0 °C until no bubbling occurred. The mixture was distilled under reduced pressure to remove the solvent. Methanol (30 mL) and NaOH solution (1.0 mmol/mL, 78 mL) were added, and the mixture was stirred at room temperature for 1 hour. The mixture was evaporated to remove methanol, and then extracted with ethyl acetate, and layered. The organic phase was concentrated via a rotary evaporator under reduced pressure to obtain 12.72 g of a brown oily product, with a yield of 95.93%. LC-MS (ESI) m/z: 342.36 $(M + H)^+$.

Synthesis of intermediate **26d**: To a clean, dry flask were added sequentially intermediate **26c** (12.72 g, 37.26 mmol) and anhydrous tetrahydrofuran (60 mL). After being purged with nitrogen, N,N'-carbonyldiimidazole (8.46 g, 52.16 mmol) was added at 0 °C. The mixture was stirred at room temperature for 16 hours, and the reaction was quenched after the reaction was confirmed to be complete by TLC. The reaction solution was concentrated under reduced pressure, and the crude product was purified by column chromatography (the mobile phase was petroleum ether and ethyl acetate, and the volume ratio was 1:1) to obtain 10.27 g of a white solid, with a yield of 75.02%. LC-MS (ESI) m/z: 368.22 $(M + H)^+$. $^1H$ NMR (400 MHz, DMSO-$d_6$) δ 7.30 (dd, *J* = 10.5, 8.8 Hz, 1H), 7.21 (dd, *J* = 13.2, 6.9 Hz, 1H), 7.10 (s, 1H), 4.08 (s, 2H), 4.03 (q, *J* = 7.1 Hz, 2H), 3.93 - 3.82 (m, 1H), 2.85 (s, 2H), 2.40 (qd, *J* = 12.4, 4.3 Hz, 2H), 1.66 (d, *J* = 12.3 Hz, 2H), 1.41 (s, 9H).

Synthesis of intermediate **26e**: To a clean, dry flask were added intermediate **26d** (5.51 g, 15.00 mmol) and THF (50mL). After being purged with nitrogen, 60% NaH by mass (1.20 g, 30.00 mmol) was slowly added at 0 °C. The mixture was stirred for 0.5 hours. 2-chloro-N,N-dimethylacetamide (2.18 g, 18.00 mmol) was added. After stirring at room temperature for 16 hours, the reaction was confirmed to be complete by TLC. At 0 °C, water was added dropwise to quench the reaction. The mixture was extracted with ethyl acetate. The organic phase was dried over anhydrous $Na_2SO_4$ and then filtered. The filtrate was evaporated via a rotary evaporator under reduced pressure to remove the solvent and thus obtain 6.78 g of a yellow oily product, with a yield of 99.85%. LC-MS (ESI) m/z: 453.35 $(M + H)^+$.

Synthesis of intermediate **26f**: To a clean, dry flask were added intermediate **26e** (6.78 g, 14.98 mmol), and anhydrous dichloromethane (50 mL) was added thereto. At 0 °C, trifluoroacetic acid (10 mL) was added dropwise. After the addition was complete, the ice-water bath was removed, and the mixture was stirred at room temperature for 12 hours. The reaction was confirmed to be essentially complete by TLC. The mixture was evaporated via a rotary evaporator under reduced pressure to remove the solvent and thus obtain a light brown oily crude product. Methyl tert-butyl ether was added to the crude product, and the mixture was dispersed, stirred, filtered, and dried to obtain 6.90 g of a yellow solid, with a yield of 98.85%. The yellow solid was used directly in the next experiment without further purification.

Synthesis of compound **26**: To a clean, dry flask were added intermediate **26f** (2.50 g, 5.36 mmol) and anhydrous 1,2-dichloroethane (20 mL). Triethylamine was added dropwise to adjust the pH to approximately 8, with stirring for 10 min. 4-isopropylcyclohexanone (1.88 g, 13.40 mmol) was added. Acetic acid was added to adjust the pH to approximately 5. After stirring at room temperature for 2 h, sodium triacetoxyborohydride (3.41 g, 16.08 mmol) was added. The reaction was carried out at 40 °C for 16 hours, and the reaction was confirmed to be complete by TLC. The mixture was evaporated via a rotary evaporator under reduced pressure to remove the solvent. The crude product was purified by a preparative High Performance Liquid Chromatography (the separation conditions were the same as in Example 1) to obtain 650 mg of a white solid, with a yield of 25.45%. LC-MS (ESI) m/z: 477.38 $(M + H)^+$; $^1H$ NMR (600 MHz, $CDCl_3$) δ 6.98 (dd, *J* = 12.4, 6.7 Hz, 1H), 6.90 - 6.77 (m, 1H), 4.32 (s, 2H), 4.15 (s, 2H), 3.97 (tt, *J* = 12.1, 3.9 Hz, 1H), 3.21 - 3.07 (m, 2H), 3.02 (s, 3H), 2.97 (s, 3H), 2.52 (qd, *J* = 12.4, 3.9 Hz, 2H), 2.34 - 2.22 (m, 1H), 2.13 (t, *J* = 11.6 Hz, 2H), 1.85 - 1.70 (m, 2H), 1.70 - 1.52 (m, 5H), 1.51 - 1.40 (m, 2H), 1.40 - 1.29 (m, 2H), 1.15 - 1.05 (m, 1H), 0.87 (d, *J* = 6.6 Hz, 6H).

**Example 24:** Synthesis of N-(2-(6,7-difluoro-2-oxo-1-(l-(spiro[2.5]oct-6-yl)piperidin-4-yl)-1,4-dihydroquinazolin-3(2*H*)-yl)ethyl)acetamide (**30**)

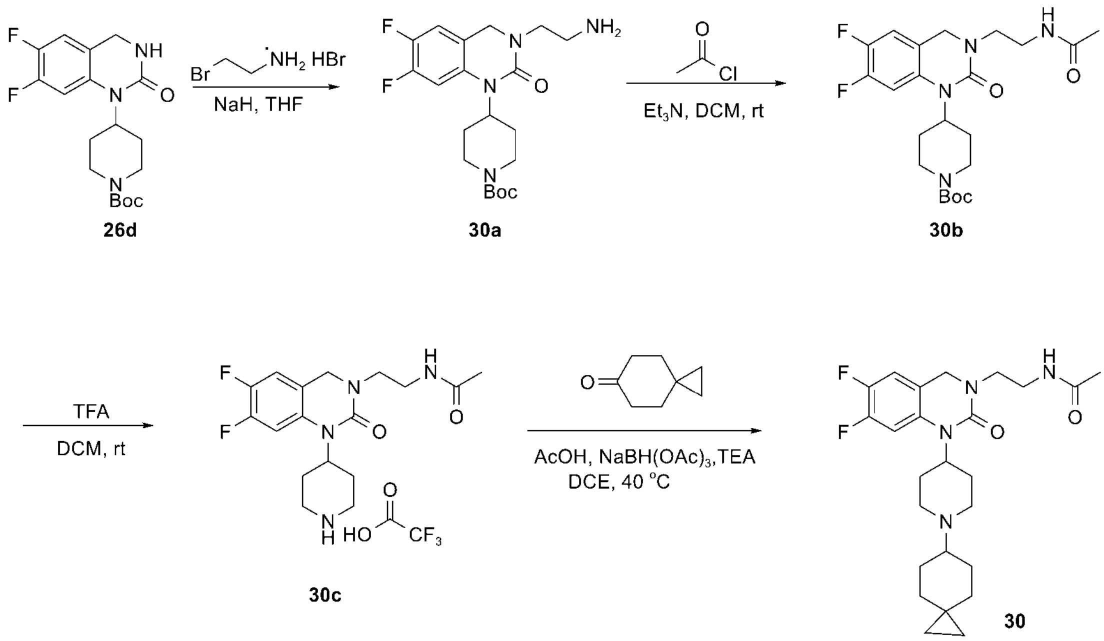


26d 30a 30b 30c 30

Synthesis of intermediate **30a**: To a clean, dry flask were added intermediate **26d** (3.00 g, 8.20 mmol) and THF (40 mL). After being purged with nitrogen, 60% NaH by mass(1.31 g, 32.80 mmol) was slowly added at 0 °C. The mixture was stirred for 0.5 hours. Triethylamine-pretreated 2-bromoethylamine hydrobromide (3.36 g, 16.40 mmol) was added. After stirring at room temperature for 16 hours, the reaction was confirmed to be complete by TLC. At 0 °C, water was added dropwise to quench the reaction. The mixture was extracted with dichloromethane. The organic phase was dried over anhydrous $Na_2SO_4$ and then filtered. The filtrate was evaporated via a rotary evaporator under reduced pressure to remove the solvent. The crude product was purified by column chromatography (the mobile phase was dichloromethane and methanol, and the volume ratio was 10:1) to obtain 2.36 g of a white solid, with a yield of 70.11%. LC-MS (ESI) m/z: 411.35 $(M + H)^+$.

Synthesis of intermediate **30b**: To a clean, dry flask were added intermediate **30a** (2.00 g, 4.87 mmol) and anhydrous dichloromethane (20 mL), and triethylamine (1.48 g, 14.62 mmol) was added thereto. Acetyl chloride (459 mg, 5.84 mmol) was slowly added under an ice-water bath. The mixture was stirred at room temperature for 3 hours. The reaction was confirmed to be essentially complete by TLC. The mixture was evaporated via a rotary evaporator under reduced pressure to remove the solvent. The crude product was purified by column chromatography (the mobile phase was dichloromethane and methanol, and the volume ratio was 20:1) to obtain 1.20 g of a white solid, with a yield of 54.54%. LC-MS (ESI) m/z: 453.36 $(M + H)^+$.

Synthesis of intermediate **30c**: To a clean, dry flask were added intermediate **30b** (1.20 g, 2.65 mmol), and anhydrous dichloromethane (10 mL) was added thereto. At 0 °C, trifluoroacetic acid (2 mL) was added dropwise. After the addition was complete, the ice-water bath was removed, and the mixture was stirred at room temperature for 12 hours. The reaction was confirmed to be essentially complete by TLC. The mixture was evaporated via a rotary evaporator under reduced pressure to remove the solvent and thus obtain a light brown oily crude product. Methyl tert-butyl ether was added to the crude product, and the mixture was dispersed, stirred, filtered, and dried to obtain 1.23 g of a white solid, with a yield of 99.51%. The white solid was used directly in the next experiment without further purification.

Synthesis of compound **30**: To a clean, dry flask were added intermediate **30c** (1.23 g, 2.64 mmol) and anhydrous 1,2-dichloroethane (10 mL). Triethylamine was added dropwise to adjust the pH to approximately 8, with stirring for 10 min. Spiro[2.5]octan-6-one (656 mg, 5.28 mmol) was added. Acetic acid was added to adjust the pH to approximately 5. After stirring at room temperature for 2 h, sodium triacetoxyborohydride (1.68 g, 7.92 mmol) was added. The reaction was carried out at 40 °C for 16 hours, and the reaction was confirmed to be complete by TLC. The mixture was evaporated via a rotary evaporator under reduced pressure to remove the solvent. The crude product was purified by column chromatography (the mobile phase was dichloromethane and methanol, and the volume ratio was 20:1) to obtain 800 mg of a yellow solid, with a yield of 65.79%. LC-MS (ESI) m/z: 461.45 $(M + H)^+$; $^1H$ NMR (400 MHz, $CDCl_3$) δ 7.14 (dd, *J* = 12.2, 6.7 Hz, 1H), 6.88 (dd, *J* = 9.6, 8.2 Hz, 1H), 6.59 (d, *J* = 6.1 Hz, 1H), 4.21 (s, 2H), 4.14 - 3.95 (m, 1H), 3.52 (dd, *J* = 6.9, 4.8 Hz, 2H), 3.44 (q, *J* = 5.6 Hz, 2H), 3.31 (d, *J* = 11.5 Hz, 2H), 3.00 - 2.75 (m, 3H), 2.68 (t, *J* = 11.8 Hz, 2H), 2.00 (d, *J* = 11.8 Hz, 2H), 1.94 (s, 3H), 1.91 - 1.71 (m, 4H), 1.54 (qd, *J* = 12.2, 3.5 Hz, 2H), 0.97 (d, *J* = 13.3 Hz, 2H), 0.33 (dd, *J* = 8.6, 5.7 Hz, 2H), 0.20 (dd, *J* = 8.8, 5.8 Hz, 2H).

**Example 25:** Synthesis of N-(2-(6,7-difluoro-1-(1-(4-isopropylcyclohexyl)piperidin-4-yl)-2-oxo-1,4-dihydroquinazoline-3 (2*H*)-yl)ethyl)methanesulfonamide (**31**)

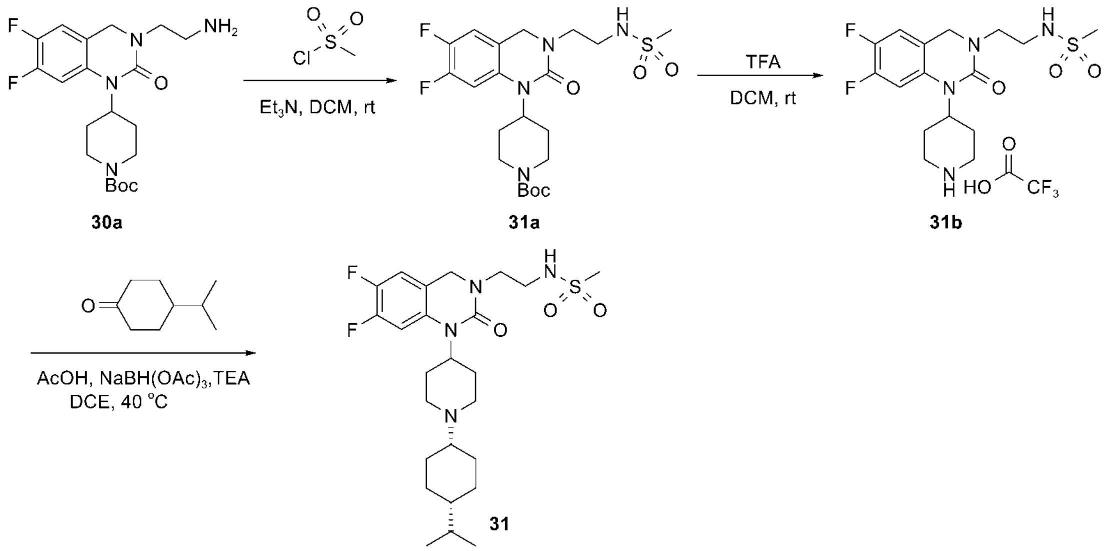


**30a** **31a** **31b** **31**

Synthesis of intermediate **31a**: To a clean, dry flask were added intermediate **30a** (1.18 g, 2.87 mmol) and anhydrous dichloromethane (10 mL), and triethylamine (580 mg, 5.75 mmol) was added thereto. Methylsulfonyl chloride (395 mg, 3.44 mmol) was slowly added under an ice-water bath. The mixture was stirred at room temperature for 4 hours. The reaction was confirmed to be essentially complete by TLC. Saturated solution of sodium bicarbonate was slowly added to quench the reaction. The mixture was extracted by adding dichloromethane. The organic phase was washed with saturated brine, and dried over anhydrous sodium sulfate. Then it was evaporated via a rotary evaporator under reduced pressure to remove the solvent. The crude product was purified by column chromatography (the mobile phase was dichloromethane and methanol, and the volume ratio was 20:1) to obtain 1.30 g of a yellow oil, with a yield of 92.85%. LC-MS (ESI) m/z: 489.27 $(M + H)^+$.

Synthesis of intermediate **31b**: To a clean, dry flask were added intermediate **31a** (1.30 g, 2.66 mmol), and anhydrous dichloromethane (10 mL) was added thereto. At 0 °C, trifluoroacetic acid (2 mL) was added dropwise. After the addition was complete, the ice-water bath was removed, and the mixture was stirred at room temperature for 12 hours. The reaction was confirmed to be essentially complete by TLC. The mixture was evaporated via a rotary evaporator under reduced pressure to remove the solvent and thus obtain an oily crude product. Methyl tert-butyl ether was added to the crude product, and the mixture was dispersed, stirred, filtered, and dried to obtain 1.30 g of a white solid, with a yield of 97.23%. The white solid was used directly in the next experiment without further purification.

Synthesis of compound **31**: To a clean, dry flask was added intermediate **31b** (1.30 g, 2.59 mmol) and anhydrous 1,2-dichloroethane (10 mL). Triethylamine was added dropwise to adjust the pH to approximately 8, with stirring for 10 min. 4-isopropylcyclohexanone (726 mg, 5.18 mmol) was added. Acetic acid was added to adjust the pH to approximately 5. After stirring at room temperature for 2 hours, sodium triacetoxyborohydride (1.65 g, 7.77 mmol) was added. The reaction was carried out at 40 °C for 16 hours, and the reaction was confirmed to be complete by TLC. The mixture was evaporated via a rotary evaporator under reduced pressure to remove the solvent. The crude product was purified by column chromatography (the mobile phase was dichloromethane and methanol, and the volume ratio was 20:1), and then further separated by a preparative High Performance Liquid Chromatography (the separation conditions were the same as in Example 1). 150 mg of a white solid was obtained, with a yield of 11.30%. LC-MS (ESI) m/z: 513.38 $(M + H)^+$; $^1H$ NMR (400 MHz, DMSO-$d_6$) $\delta$ 7.31 (t, $J$ = 9.5 Hz, 1H), 7.17 - 7.03 (m, 2H), 4.28 (s, 2H), 3.76 - 3.60 (m, 1H), 3.34 - 3.21 (m, 3H), 3.18 - 3.06 (m, 2H), 3.01 (d, $J$ = 11.1 Hz, 2H), 2.92 (d, $J$ = 1.4 Hz, 1H), 2.87 (s, 3H), 2.23 (s, 1H), 2.08 (t, $J$ = 11.5 Hz, 2H), 1.76 - 1.59 (m, 4H), 1.59 - 1.44 (m, 3H), 1.45 - 1.27 (m, 4H), 1.16 - 1.01 (m, 1H), 0.86 (d, $J$ = 6.6 Hz, 6H).

**Example 26:** Synthesis of N-(2-(6,7-difluoro-1-(1-(*cis*-4-isopropylcyclohexyl)piperidin-4-yl)-2-oxo-1,4-dihydroquinazoli n-3(2*H*)-yl)ethyl)ethanesulfonamide (**32**)

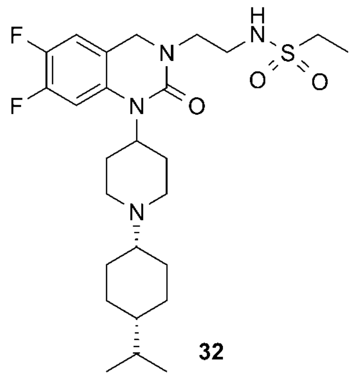

**32**

Intermediate **30a** and ethylsulfonyl chloride were used as starting materials. This compound was synthesized following the synthesis method in Example 25. In the last step, 160 mg of a white solid was obtained through separation and purification (the separation conditions were the same as in Example 1), with a yield of 20.50%. LC-MS (ESI) m/z: 527.34 (M + H)$^+$; $^1$H NMR (400 MHz, DMSO-$d_6$) δ 7.31 (t, *J* = 9.6 Hz, 1H), 7.18 - 7.05 (m, 2H), 4.28 (s, 2H), 3.79 - 3.58 (m, 1H), 3.33 - 3.21 (m, 3H), 3.09 (q, *J* = 6.3 Hz, 2H), 3.05 - 2.87 (m, 5H), 2.28 - 2.19 (m, 1H), 2.08 (t, *J* = 11.6 Hz, 2H), 1.76 - 1.60 (m, 4H), 1.60 - 1.47 (m, 3H), 1.44 - 1.27 (m, 4H), 1.13 (t, *J* = 7.3 Hz, 3H), 1.10 - 1.03 (m, 1H), 0.86 (d, *J* = 6.6 Hz, 6H).

**Example 27:** Synthesis of N-(2-(l-(l-(4,4-dimethylcyclohexyl)piperidin-4-yl)-6,7-difluoro-2-oxo-1,4-dihydroquinazolin-3( 2*H*)-yl)ethyl)acetamide (**33**)

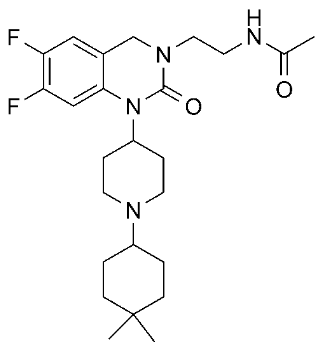

**33**

Intermediate **30c** (1.23 g, 2.64 mmol) and 4,4-dimethylcyclohexanone (833 mg, 6.60 mmol) were used as starting materials. This compound was synthesized following the synthesis method in Example 24 to obtain 820 mg of a yellow solid, with a yield of 67.16%. LC-MS (ESI) m/z: 463.57 (M + H)$^+$; $^1$H NMR (600 MHz, $CDCl_3$) δ 7.00 (s, 1H), 6.88 (dd, *J* = 9.5, 8.2 Hz, 1H), 6.49 (s, 1H), 4.19 (s, 2H), 4.02 - 3.76 (m, 1H), 3.54 (dd, *J* = 6.9, 4.6 Hz, 2H), 3.46 (dt, *J* = 7.3, 4.9 Hz, 2H), 3.24 - 2.94 (m, 2H), 2.79 - 2.48 (m, 2H), 2.48 - 2.17 (m, 3H), 1.95 (s, 3H), 1.89 - 1.72 (m, 4H), 1.46 (d, *J* = 12.6 Hz, 4H), 1.24 - 1.13 (m, 2H), 0.90 (d, *J* = 4.1 Hz, 6H).

**Example 28:** Synthesis of N-(2-(l-(l-(4,4-dimethylcyclohexyl)piperidin-4-yl)-6,7-difluoro-2-oxo-1,4-dihydroquinazolin-3( 2*H*)-yl)ethyl)ethanesulfonamide (**34**)

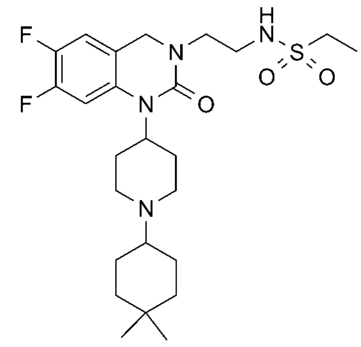

**34**

Intermediate **30a**, ethylsulfonyl chloride and 4,4-dimethylcyclohexanone were used as starting materials. This compound was synthesized following the synthesis method in Example 25. In the last step, 200 mg of a white solid was obtained, with a yield of 75.00%. LC-MS (ESI) m/z: 513.45 $(M + H)^+$; $^1H$ NMR (400 MHz, $CDCl_3$) δ 7.11 (s, 1H), 6.89 (t, *J* = 8.8 Hz, 1H), 5.21 (s, 1H), 4.26 (s, 2H), 4.14 (s, 1H), 3.57 (t, *J* = 5.7 Hz, 2H), 3.35 (q, *J* = 5.6 Hz, 2H), 3.32 - 3.20 (m, 1H), 3.01 (q, *J* = 7.4 Hz, 2H), 2.93 - 2.69 (m, 2H), 2.69 - 2.44 (m, 2H), 2.05 - 1.65 (m, 6H), 1.63 - 1.42 (m, 4H), 1.34 (t, *J* = 7.4 Hz, 3H), 1.30 - 1.12 (m, 2H), 0.92 (s, 3H), 0.91 (s, 3H).

**Example 29:** Synthesis of 1-(1-(4,4-dimethylcyclohexyl)piperidin-4-yl)-5,6-difluoro-3-((phenylsulfonyl)methyl)-1,3-dihy dro-2*H*-benzo[*d*]imidazol-2-one (**36**)

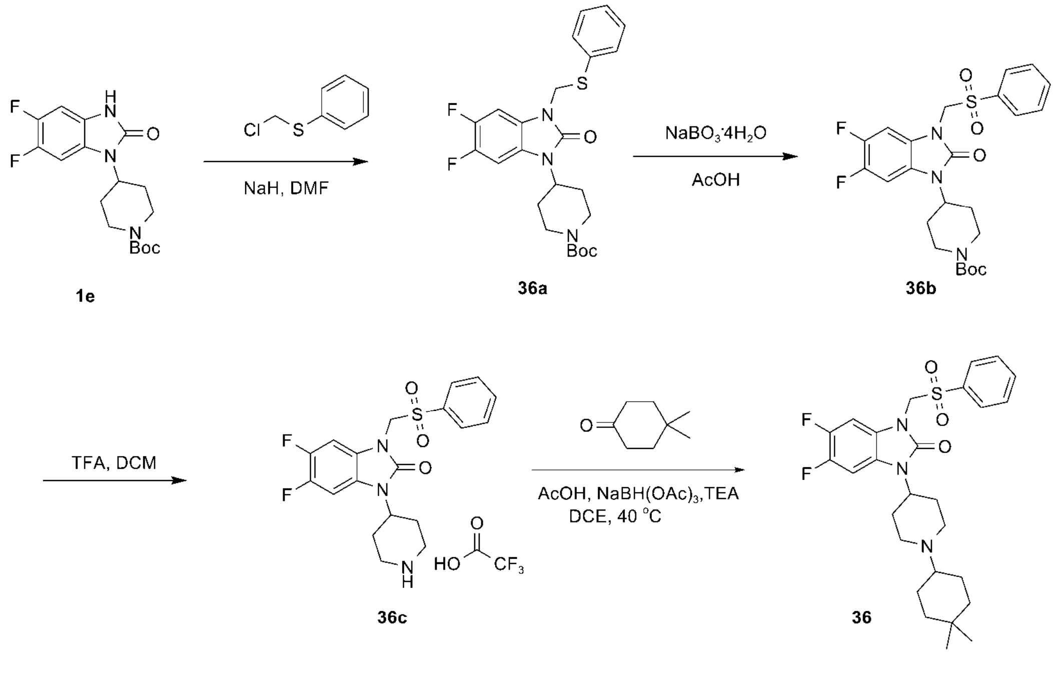


1e 36a 36b 36c 36

Synthesis of intermediate **36a**: To a clean, dry flask were added sequentially intermediate **1e**(500 mg, 1.41 mmol), anhydrous DMF (7 mL). After being purged with nitrogen three times, 60% NaH by mass (170 mg, 4.26 mmol) was slowly added under the condition of ice bath. The mixture was heated to room temperature and stirred for 30 min. Chloromethyl phenyl sulfide (125 mg, 2.13 mmol) was added. Stirring was continued at room temperature for 16 hours, and the reaction was confirmed to be essentially complete by TLC. To the reaction solution was added saturated aqueous solution of ammonium chloride. The mixture was extracted with ethyl acetate, and the organic phase was washed with saturated brine, dried over anhydrous sodium sulfate, and then evaporated via a rotary evaporator under reduced pressure to remove the solvent. The crude product was purified by column chromatography (the mobile phase was petroleum ether and ethyl acetate, and the volume ratio was 1:1) to obtain 300 mg of a white solid, with a yield of 44.78%. LC-MS (ESI) m/z: 420.22 $(M-tBu + H)^+$.

Synthesis of intermediate **36b**: To a clean, dry flask were added sequentially intermediate **36a** (300 mg, 0.61

mmol)and glacial acetic acid (3 mL). Sodium perborate tetrahydrate (235 mg, 1.53 mmol) was slowly added with stirring at room temperature. After stirring at room temperature for 16 hours, it was confirmed by TLC that the starting material had reacted substantially to completion. To the reaction solution was added the aqueous solution of potassium carbonate. The mixture was extracted with ethyl acetate. The organic phase was washed with saturated brine, and dried over anhydrous sodium sulfate, and then was evaporated via a rotary evaporator under reduced pressure to remove the solvent and thus obtain 281 mg of a light yellow solid, with a yield of 90.70%. LC-MS (ESI) m/z: 408.23 $(M\text{-}Boc + H)^+$.

Synthesis of intermediate **36c**: To a clean, dry flask was added intermediate **36b** (280 mg, 0.55 mmol), and anhydrous dichloromethane (5 mL) was added thereto. At 0 °C, trifluoroacetic acid (1 mL) was added dropwise. After the addition was complete, the ice-water bath was removed. The mixture was stirred at room temperature for 12 hours. It was confirmed by monitoring with TLC plates that the starting materials had reacted substantially to completion. The mixture was evaporated via a rotary evaporator under reduced pressure to remove the solvent and thus obtain a crude product. Methyl tert-butyl ether (5 mL) was added to the crude product, and the mixture was dispersed, stirred, filtered, and dried to obtain 287 mg of a yellow solid, with a yield of 99.99%. The yellow solid was used directly in the next experiment without further purification.

Synthesis of compound **36**: To a clean, dry flask were added intermediate **36c** (203 mg, 0.39 mmol) and anhydrous 1,2-dichloroethane (5 mL). Triethylamine was added dropwise to adjust the pH to approximately 8, with stirring for 10 minutes. 4,4-dimethylcyclohexanone (123 mg, 0.96 mmol) was added. Acetic acid was added to adjust the pH to approximately 5. After stirring at room temperature for 2 h, sodium triacetoxyborohydride (298 mg, 1.17 mmol) was added. The reaction was carried out at 40 °C for 24 hours, and the reaction was confirmed to be complete by TLC. To the reaction solution was added a saturated aqueous solution of sodium bicarbonate (10 mL) and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was purified by column chromatography (the mobile phase was dichloromethane and methanol, and the volume ratio was 40:1) to obtain 115 mg of a white solid, with a yield of 57.07%. LC-MS (ESI) m/z: 518.53 $(M + H)^+$; $^1H$ NMR (400 MHz, $CDCl_3$) δ 7.81 - 7.74 (m, 2H), 7.67 (t, *J* = 7.5 Hz, 1H), 7.58 (dd, *J* = 10.0, 6.7 Hz, 1H), 7.51 (t, *J* = 7.7 Hz, 2H), 7.04 (dd, *J* = 9.5, 6.6 Hz, 1H), 5.14 (s, 2H), 4.49 - 4.37 (m, 1H), 3.65 (d, *J* = 10.4 Hz, 2H), 3.14 (t, *J* = 12.3 Hz, 1H), 3.05 - 2.79 (m, 4H), 1.95 (d, *J* = 12.0 Hz, 2H), 1.89 - 1.79 (m, 2H), 1.68 - 1.63 (m, 2H), 1.59 (d, *J* = 13.8 Hz, 2H), 1.32 (td, *J* = 13.7, 3.5 Hz, 2H), 0.95 (d, *J* = 8.1 Hz, 6H).

**Example 30:** Synthesis of 5,6-difluoro-1-((methylsulfonyl)methyl)-3-(1-(spiro[3.5]non-7-yl)piperidin-4-yl)-1,3-dihydro-2*H*-benzo[*d*]imidazol-2-one (**37**)

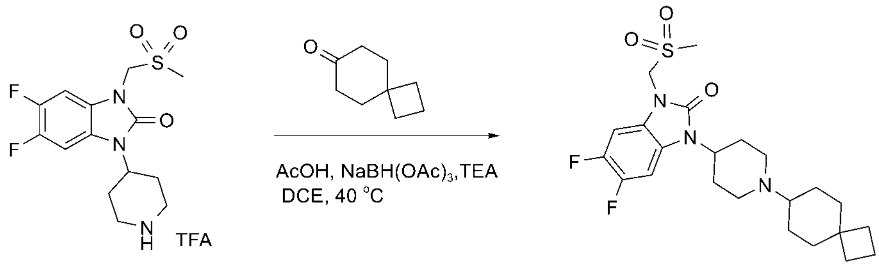


**18c** **37**

To a clean, dry flask were added intermediate **18c** (0.95 g, 2.12 mmol) and anhydrous 1,2-dichloroethane (4 mL). Triethylamine was added dropwise to adjust the pH to approximately 8, with stirring for 10 min. Spiro[3.5]nonan-7-one (0.44 g, 3.18 mmol) was added. Acetic acid was added to adjust the pH to approximately 5. After stirring at room temperature for 2 hours, sodium triacetoxyborohydride (1.12 g, 5.30 mmol) was added. The reaction was carried out at 40 °C for 24 hours, and the reaction was confirmed to be complete by TLC. To the reaction solution was added a saturated aqueous solution of sodium bicarbonate (5 mL), and the mixture was stirred and layered. After the organic phase was washed with saturated brine and dried over anhydrous sodium sulfate, it was evaporated via a rotary evaporator under reduced pressure to remove the solvent and thus obtain a crude product. The crude product was purified by column chromatography (the mobile phase was dichloromethane and methanol, and the volume ratio was 40:1) to obtain 220 mg of a white solid, with a yield of 21.30%. LC-MS (ESI) m/z: 468.33 $(M + H)^+$; $^1H$ NMR (400 MHz, $CDCl_3$) δ 7.71 (s, 1H), 7.15 (dd, *J* = 9.3, 6.6 Hz, 1H), 5.00 (s, 2H), 4.77 - 4.52 (m, 1H), 3.77 - 3.52 (m, 2H), 3.26 - 3.02 (m, 2H), 2.98 (s, 3H), 2.95 - 2.84 (m, 2H), 2.07 - 1.92 (m, 4H), 1.92 - 1.83 (m, 2H), 1.82 - 1.66 (m, 6H), 1.50 (q, *J* = 12.2, 11.5 Hz, 2H), 1.38 (q, *J* = 13.1, 11.3 Hz, 2H).

**Example 31:** Synthesis of 1-(1-(4,4-dimethylcyclohexyl)piperidin-4-yl)-5,6-difluoro-3-((methylsulfinyl)methyl)-1,3-dihydro-2H-benzo[d]imidazol-2-one (19) and 1-(1-(4,4-dimethylcyclohexyl)piperidin-4-yl)-5,6-difluoro-3-((S-methylsulfonimidoyl)methyl)-1,3-dihydro-2*H*-benzo[*d*]imidazol-2-one (42)

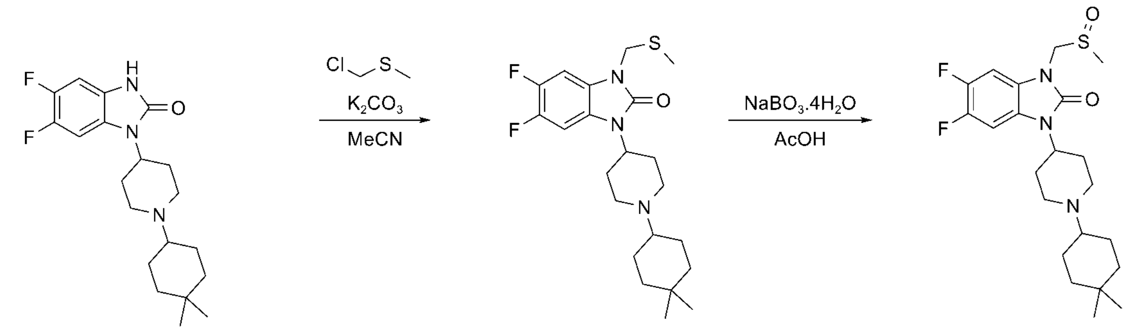


11b 19b 19

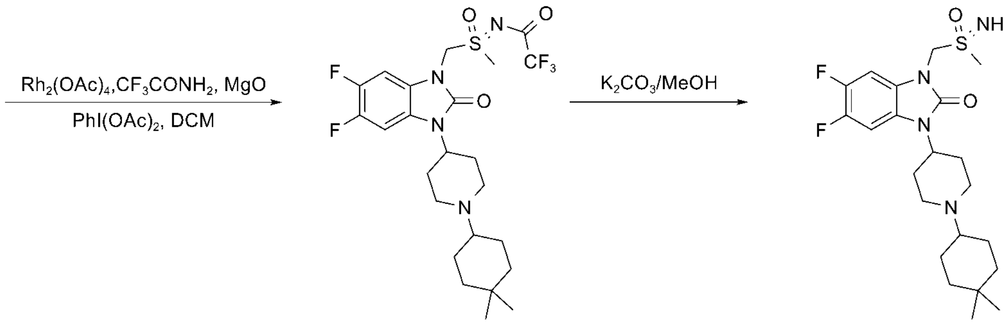


42a 42

Synthesis of intermediate **19b**: To a clean, dry round bottom flask were added sequentially intermediate **11b** (200 mg, 0.55 mmol), acetonitrile (2.0 mL), $K_2CO_3$ (114 mg, 0.83 mmol) and chloromethyl methyl sulfide (64 mg, 0.66 mmol). The reaction solution was stirred at 50 °C for 2 hours. Complete consumption of the starting material was detected by LCMS. Water (15 mL) and ethyl acetate (20 mL) were added to the reaction solution for dilution, and the mixture was extracted with ethyl acetate. The combined organic phases were dried over anhydrous $Na_2SO_4$ and then filtered. The filtrate was distilled via a rotary evaporator under reduced pressure to remove the solvent, and thus obtain 210 mg of a brown crude product, with a yield of 90.13%.

Synthesis of compound **19**: To a clean, dry flask were added sequentially intermediate **19b** (200 mg, 0.47 mmol) and glacial acetic acid (3 mL). Sodium perborate tetrahydrate (96 mg, 0.94 mmol) was slowly added with stirring at room temperature. After stirring at room temperature for 16 hours, it was confirmed by TLC that the starting material had reacted substantially to completion. An aqueous solution of potassium carbonate was added to the reaction solution. The mixture was extracted with ethyl acetate. After the organic phase was washed with saturated brine and dried over anhydrous sodium sulfate, it was evaporated via a rotary evaporator under reduced pressure to remove the solvent, and 150 mg of a yellow solid was obtained, with a yield of 72.60%. LC-MS (ESI) m/z: 440.28 $(M + H)^+$.

Synthesis of intermediate **42a**: To a clean, dry flask was added intermediate **19** (100 mg, 0.23 mmol). After being purged with nitrogen, anhydrous dichloromethane (1 mL), rhodium acetate dimer (2.50 mg, 0.0057 mmol), trifluoroacetamide (52 mg, 0.46 mmol), magnesium oxide (37 mg, 0.92 mmol) and diacetoxyiodobenzene (113 mg, 0.35 mmol) were added. After stirring at room temperature for 16 hours, it was monitored by TLC that the starting material had reacted substantially to completion. The reaction solution was evaporated via a rotary evaporator under reduced pressure to remove the solvent. The crude product was purified by column chromatography (the mobile phase was petroleum ether and ethyl acetate, and the volume ratio was 1:4) to obtain 100 mg of a white solid, with a yield of 79.37%. LC-MS (ESI) m/z: 551.32 $(M + H)^+$.

Synthesis of compound **42**: To a clean, dry flask were added intermediate **42a** (100 mg, 0.18 mmol), methanol (0.5 mL), and potassium carbonate (124 mg, 0.90 mmol). The mixture was stirred at room temperature for 0.5 hours. The reaction was confirmed to be complete by TLC. The reaction solution was filtered, and the filtrate was collected and then evaporated via a rotary evaporator under reduced pressure to remove the solvent and thus obtain a light brown oily crude

product, and the crude product was purified by column chromatography (the mobile phase was dichloromethane and methanol, and the volume ratio was 30:1) to obtain 20 mg of a white solid, with a yield of 24.39%. LC-MS (ESI) m/z: 455.39 $(M + H)^+$; $^1H$ NMR (400 MHz, DMSO-d6) δ 7.58 (ddd, *J* = 19.5, 10.8, 7.0 Hz, 2H), 5.29 - 5.07 (m, 2H), 4.21 - 4.07 (m, 1H), 3.92 (s, 1H), 3.07 - 2.94 (m, 2H), 2.89 (s, 3H), 2.43 - 2.22 (m, 4H), 1.76 - 1.66 (m, 2H), 1.65 - 1.55 (m, 2H), 1.51 - 1.34 (m, 4H), 1.21 - 1.10 (m, 2H), 0.89 (d, *J* = 2.6 Hz, 6H).

**Example 32:** Synthesis of 2-(3-(1-(4,4-dimethylcyclohexyl)piperidin-4-yl)-5,6-difluoro-2-oxo-2,3-dihydro-1*H*-benzo[*d*]imidazol-1-yl)ethane-1-sulfonamide (**43**)

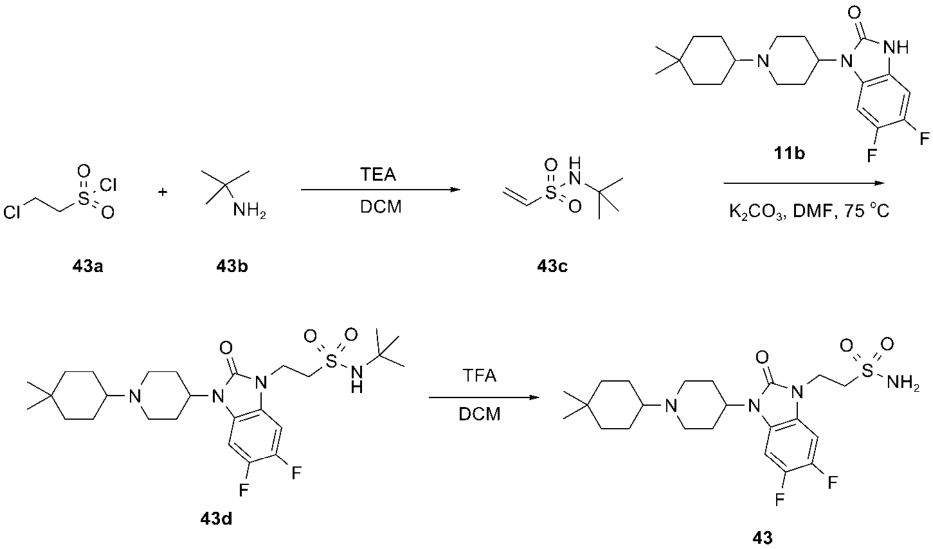


**43a** **43b** **43c** **11b** **43d** **43**

Synthesis of intermediate **43c**: To a clean, dry flask were added sequentially anhydrous dichloromethane (30 mL), tert-butylamine (730 mg, 10.0 mmol) and triethylamine (3.54 g, 35.0 mmol). The reaction system was cooled to 0 °C, and 2-chloroethanesulfonyl chloride (1.63 g, 10.0 mmol) was added. The mixture was continued stirring for 2 hours at 0 °C. It was monitored by TLC that the starting materials had reacted substantially to completion. To the reaction solution was added dichloromethane for dilution. The mixture was washed with saturated brine. The organic phase was dried over anhydrous $Na_2SO_4$ and then filtered. The filtrate was evaporated via a rotary evaporator under reduced pressure to remove the solvent, and thus obtain 1.20 g of a colorless transparent liquid, with a yield of 73.62%.

Synthesis of intermediate **43d**: To a clean, dry flask were added sequentially intermediate **11b** (363 mg, 1.0 mmol), DMF (5 mL), potassium carbonate (207 mg, 1.5 mmol) and intermediate **43c** (196 mg, 1.2 mmol). The reaction system was heated to 70 °C and stirred for 12 hours. It was monitored by TLC that the starting materials had reacted substantially to completion. The reaction solution was cooled to room temperature, diluted with the addition of water, and then extracted with ethyl acetate. The organic phase was washed with saturated brine, dried over anhydrous $Na_2SO_4$ and then filtered, and evaporated via a rotary evaporator under reduced pressure to obtain a crude product. The crude product was separated and purified by silica gel column chromatography (the mobile phase was dichloromethane and methanol, and the volume ratio was 30:1) to obtain 460 mg of a white solid, with a yield of 87.29%. LC-MS (ESI) m/z: 527.39 $(M + H)^+$.

Synthesis of compound **43**: To a clean, dry flask were added sequentially intermediate **43d** (460 mg, 0.87 mmol) and dichloromethane (4 mL). The reaction system was cooled to 0 °C, and trifluoroacetic acid (1 mL) was slowly added dropwise. After the addition was complete, the reaction solution was heated to room temperature, and the reaction was continued to be stirred for 6 hours. It was monitored by TLC that the starting materials had reacted substantially to completion. The mixture was evaporated via a rotary evaporator under reduced pressure to remove the solvent and thus obtain a crude product. To the crude product was added a saturated aqueous solution of sodium carbonate, and the mixture was extracted with dichloromethane. The combined organic phases were washed with saturated brine, and dried over anhydrous $Na_2SO_4$ and then filtered. The filtrate was evaporated via a rotary evaporator under reduced pressure to remove the solvent and thus obtain a crude product, which was separated and purified by column chromatography (the mobile phase was dichloromethane and methanol, and the volume ratio was 40:1 ~ 20:1) to obtain 280 mg of a white solid, with a yield of 68.16%. LC-MS (ESI) m/z: 471.38 $(M + H)^+$; $^1H$ NMR (400 MHz, DMSO-$d_6$) δ 7.47 (dd, *J* = 11.0, 7.0 Hz, 1H), 7.39 (dd, *J* = 10.7, 7.1 Hz, 1H), 6.96 (s, 2H), 4.18 (t, *J* = 7.0 Hz, 2H), 4.14 - 3.99 (m, 1H), 3.36 (t, *J* = 7.0 Hz, 2H), 3.17 (d, *J* = 5.2 Hz, 1H), 3.07 - 2.81 (m, 2H), 2.29 (t, *J* = 11.1 Hz, 4H), 1.76 - 1.51 (m, 4H), 1.48 - 1.35 (m, 4H), 1.28 - 1.13 (m, 2H), 0.88 (d,

*J* = 2.8 Hz, 6H).

**Example 33:** Synthesis of 1-(1-(4,4-dimethylcyclohexyl)piperidin-4-yl)-5,6-difluoro-3-(2-(S-methylsulfonimidoyl) ethyl)-1,3-dihydro-2*H*-benzo[*d*]imidazol-2-one (**44**)

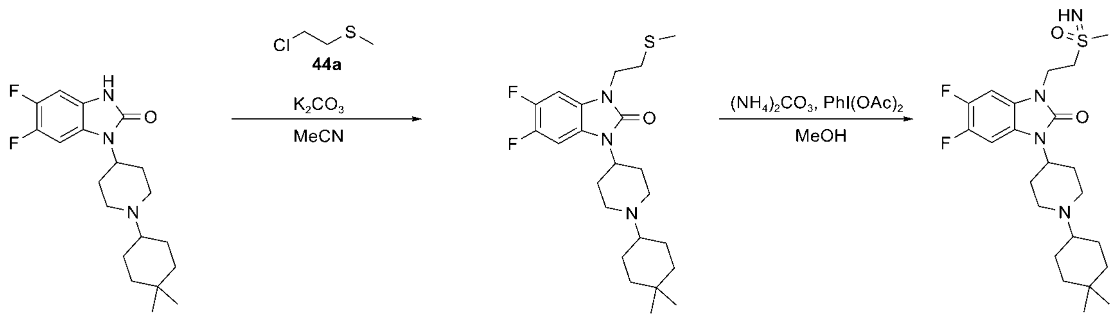


**11b** **44b** **44**

Synthesis of intermediate **44b**: To a clean, dry round bottom flask were added sequentially intermediate **11b** (200 mg, 0.55 mmol), acetonitrile (2.0 mL), $K_2CO_3$ (114 mg, 0.83 mmol) and 2-chloroethyl methyl sulfide (73 mg, 0.66 mmol). The reaction solution was stirred at 50 °C for 2 hours. Complete consumption of the starting material was detected by LCMS. Water (15 mL) and ethyl acetate (20 mL) were added to the reaction solution for dilution, and the mixture was extracted with ethyl acetate. The combined organic phases were dried over anhydrous $Na_2SO_4$ and then filtered. The filtrate was distilled via a rotary evaporator under reduced pressure to remove the solvent and thus obtain 240 mg of a brown crude product, with a yield of 100%. LC-MS (ESI) m/z: 438.34 $(M + H)^+$.

Synthesis of compound **44**: To a clean, dry round bottom flask were added sequentially intermediate **44b** (240 mg, 0.55 mmol), methanol (2.0 mL), $(NH_4)_2CO_3$ (79 mg, 0.82 mmol) and PhI(OAc)$_2$ (406 mg, 1.26 mmol). The reaction solution was stirred at 30 °C for 2 hours, and the complete consumption of the starting material was detected by LCMS. The reaction solution was evaporated via a rotary evaporator under reduced pressure to remove the solvent, and thus obtain a crude compound. The crude compound was purified by column chromatography (the mobile phase was dichloromethane and methanol, and the volume ratio was 50:1 to 10:1) to obtain 120 mg of a white solid, with a yield 46.69%. LC-MS (ESI) m/z: 469.34 $(M + H)^+$; $^1H$ NMR (400 MHz, Methanol-$d_4$) δ 7.44 (dd, *J* = 10.4, 7.1 Hz, 1 H), 7.37 (dd, *J* = 9.9, 7.2 Hz, 1 H), 4.59 - 4.52 (m, 1 H), 4.47 - 4.34 (m, 2 H), 3.71 (d, *J* = 12.7 Hz, 2 H), 3.67 - 3.55 (m, 2 H), 3.29 - 3.21 (m, 3 H), 3.10 (s, 3 H), 2.89 - 2.79 (m, 2 H), 2.15 - 2.10 (m, 2 H), 2.03 - 1.98 (m, 2 H), 1.82 - 1.72 (m, 2 H), 1.64 (d, *J* = 13.4 Hz, 2 H), 1.45 - 1.37 (m, 2 H), 1.02 (s, 3 H), 1.00 (s, 3 H).

**Example 34:** Synthesis of 2-(5-chloro-3-(1-(4,4-dimethylcyclohexyl)piperidin-4-yl)-6-fluoro-2-oxo-2,3-dihydro-1*H*-benz o[*d*]imidazol-1-yl)-N-methylacetamide (**47**)

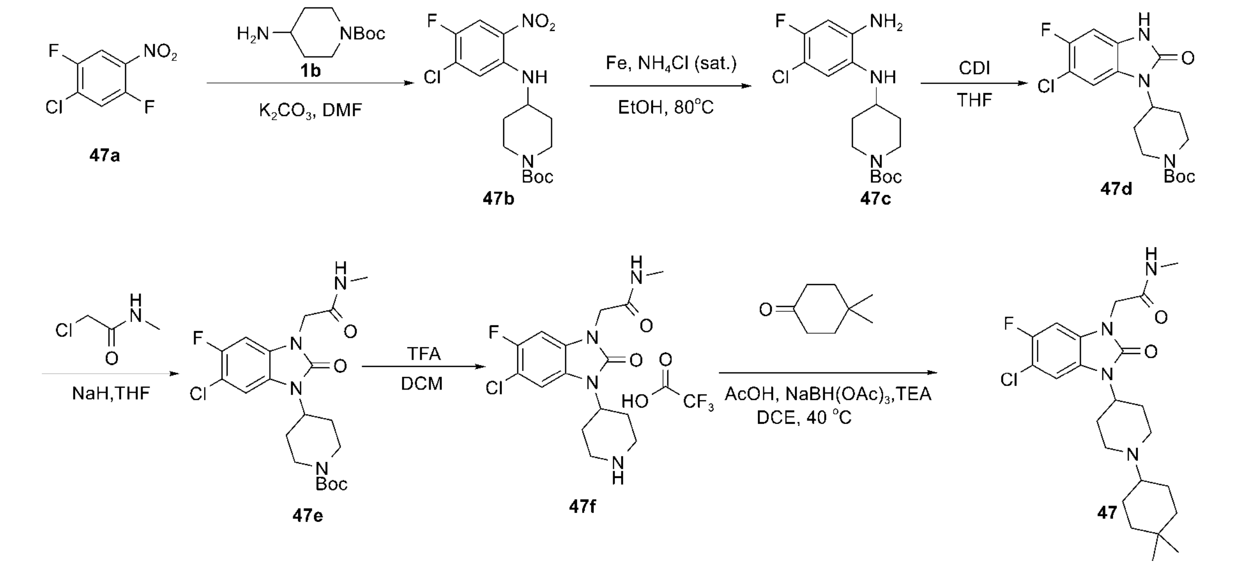


Synthesis of intermediate **47b**: To a clean, dry flask were added sequentially 1-chloro-2,4-difluoro-5-nitrobenzene (**47a**) (2.00 g, 10.33 mmol), acetonitrile (50 mL), N-Boc-4-aminopiperidine (2.07 g, 10.33 mmol), and potassium carbonate (2.14 g, 15.50 mmol). The reaction system was heated to 70 °C and stirred for 12 hours. It was monitored by TLC that the starting materials had reacted substantially to completion. The reaction solution was cooled to room temperature, diluted by adding ice water, and extracted with ethyl acetate. The combined organic phases were washed with saturated brine, dried over anhydrous $Na_2SO_4$ and then filtered, and evaporated via a rotary evaporator under reduced pressure to obtain a crude product. The crude product was purified by column chromatography (the mobile phase was petroleum ether and ethyl acetate, and the volume ratio was 5:1) to obtain 3.70 g of a yellow solid, with a yield of 95.81%. LC-MS (ESI) m/z: 374.25 $(M + H)^+$.

Synthesis of intermediate **47c**: Intermediate **47b** (3.00 g, 8.03 mmol) was placed in a clean flask. Ethanol (80 mL) was added to the reaction flask. Under stirring at room temperature, iron powder (2.69 g, 48.18 mmol) and saturated aqueous solution of $NH_4Cl$ (8 mL) were added to the reaction system. The reaction system was heated to 80 °C, and stirring was continued for 12 hours. It was monitored by TLC that the starting materials had reacted substantially to completion. The reaction solution was cooled to room temperature and filtered through celite, and the filter cake was washed with a small amount of anhydrous ethanol. The filtrate was concentrated, and the crude product was purified by column chromatography (the mobile phase was petroleum ether and ethyl acetate, and the volume ratio was 5:1) to obtain 2.10 g of a white solid, with a yield of 76.09%. LC-MS (ESI) m/z: 344.26 $(M + H)^+$.

Synthesis of intermediate **47d**: Intermediate **47c** (2.10 g, 6.11 mmol) and anhydrous tetrahydrofuran (30 mL) were placed in a clean, dry flask. After being purged with nitrogen, N,N'-carbonyldiimidazole (1.19 g, 7.33 mmol) was added at 0 °C. After stirring at room temperature for 12 hours, the reaction was confirmed to be complete by TLC. The reaction solution was concentrated under reduced pressure, and the crude product was purified by column chromatography (the mobile phase was petroleum ether and ethyl acetate, and the volume ratio was 1:1) to obtain 2.00 g of a white solid, with a yield of 88.50%. LC-MS (ESI) m/z: 370.22 $(M + H)^+$; $^1H$ NMR (400 MHz, $CDCl_3$) δ 9.40 (s, 1H), 7.11 (d, *J* = 6.0 Hz, 1H), 6.94 (d, *J* = 8.5 Hz, 1H), 4.41 (ddt, *J* = 12.6, 8.4, 4.1 Hz, 1H), 4.33 (d, *J* = 13.6 Hz, 2H), 3.00 - 2.73 (m, 2H), 2.26 (qd, *J* = 12.7, 4.6 Hz, 2H), 1.90 - 1.74 (m, 2H), 1.52 (s, 9H).

Synthesis of intermediate **47e**: To a clean, dry flask were added intermediate **47d** (2.00 g, 5.41 mmol) and THF (10 mL). After being purged with nitrogen, 60% NaH by mass (0.65 g, 16.23 mmol) was slowly added at 0 °C, and the mixture was stirred for 0.5 h. 2-chloro-N-methylacetamide (0.70 g, 6.49 mmol) was added. After stirring at room temperature for 16 hours, the reaction was confirmed to be complete by TLC. At 0 °C, water was added dropwise to quench the reaction. The mixture was extracted with ethyl acetate. The organic phase was dried over anhydrous $Na_2SO_4$ and then filtered. The filtrate was evaporated via a rotary evaporator under reduced pressure to remove the solvent and thus obtain 2.16 g of a white solid, with a yield of 90.38%. LC-MS (ESI) m/z: 441.14 $(M + H)^+$.

Synthesis of intermediate **47f**: To a clean, dry flask were added intermediate **47e** (2.16 g, 4.90 mmol), and anhydrous dichloromethane (20 mL) was added thereto. At 0 °C, trifluoroacetic acid (5 mL) was added dropwise. After the addition was complete, the ice-water bath was removed. The mixture was stirred at room temperature for 4 h. It was monitored by TLC plates that the starting materials had reacted substantially to completion. The mixture was evaporated via a rotary evaporator under reduced pressure to remove the solvent and thus obtain a light brown oily crude product. Diethyl ether (50 mL) was added to the crude product, and the mixture was dispersed, stirred, filtered, and dried to obtain 2.22 g of a yellow solid, with a yield of 100%. The yellow solid was used directly in the next experiment without further

purification.

Synthesis of compound **47**: To a clean, dry flask were added intermediate **47f** (2.22 g, 4.90 mmol) and anhydrous 1,2-dichloroethane (10 mL). Triethylamine was added dropwise to adjust the pH to approximately 8, with stirring for 10 minutes. 4,4-dimethylcyclohexanone (1.55 g, 12.25 mmol) was added. Acetic acid was added to adjust the pH to approximately 5. After stirring at room temperature for 2 h, sodium triacetoxyborohydride (3.12 g, 14.70 mmol) was added. The reaction was carried out at 40 °C for 16 hours, and the reaction was confirmed to be complete by TLC. The mixture was evaporated via a rotary evaporator under reduced pressure to remove the solvent. The crude product was purified by column chromatography (the mobile phase was dichloromethane and methanol, and the volume ratio was 20:1) to obtain 1.40 g of a white solid, with a yield of 63.35%. LC-MS (ESI) m/z: 451.32 $(M + H)^+$; $^1H$ NMR (400 MHz, Chloroform-*d*) δ 7.93 (s, 1H), 6.89 (d, *J* = 8.3 Hz, 1H), 6.07 (s, 1H), 4.76 - 4.59 (m, 1H), 4.45 (s, 2H), 3.69 (d, *J* = 11.8 Hz, 2H), 3.29 - 3.05 (m, 3H), 3.04 - 2.91 (m, 2H), 2.83 (d, *J* = 4.8 Hz, 3H), 2.08 - 1.96 (m, 4H), 1.72 (d, *J* = 12.6 Hz, 2H), 1.61 (d, *J* = 13.7 Hz, 2H), 1.41 - 1.28 (m, 2H), 0.96 (d, *J* = 5.4 Hz, 6H).

Biological Activity Assay

Experimental Example 1: Test of affinity of compounds of the present invention for MOR, NOPr (ORL-1 receptor) and KOR:

Experimental materials

(1) Main equipments: Electro-thermostatic Incubator (Shanghai YIHE), Microplate Counter (Universal Harvester, PerkinElmer), Microplate Shaker (VWR), 384-Well Microplate (Corning), and 96-Well Plate (Perkin Elmer).

(2) Main reagents: Cell membranes containing human MOR, KOR or NOPr (Perkin Elmer); [Tyrosyl-3,5-$^3H$(N)]-DAMGO (PerkinElmer) and DAMGO (MCE); [$^3H$]-U-69593 (PerkinElmer) and Naltrexone hydrochloride (Sigma); [$^3H$]-Nociceptin (PerkinElmer) and Nociceptin (1-13) amide (Sigma); polyethylenimine (PEI, Sigma); Bovine Serum Albumin (BSA, Sigma); HEPES buffer (Sigma); ULTIMA GOLD scintillation liquid (Perkin Elmer); Tris(hydroxymethyl)aminomethane (Tris-base, Sigma).

Experimental Methods

(1) Solution formulation

Standard buffer: 50 mM HEPES pH 7.4 + 0.025% BSA

Washing solution: 50 mM Tris pH 7.4

0.5% PEI solution: 0.5 mL PEI + 100 mL $ddH_2O$

Vehicle system: DMSO: $ddH_2O$ = 1: 99 (volume ratio)

Test samples: the compounds of the present invention, positive control (DAMGO, naltrexone hydrochloride, Nociceptin (1-13) amide), vehicle control

(2) Radioligand binding experiments:

a. 5 μL of test samples at various concentrations and 100 μL of standard buffer were added to a 96-well plate, and homogenized (500 rpm/min, 5 min).

b. A suspension of 1 μL of MOR- or KOR- or NOPr-containing membrane solution and 299 μl of standard buffer was added to each well, and homogenized (500 rpm/min, 5 min).

c. 100 μL of [$^3H$]-DAMGO was added to each well to a final concentration of 1 nM, (or 100 μL of [$^3H$]-U-69593 to a final concentration of 1.5 nM; or 100 μL of $^3H$-Nociceptin to a final concentration of 0.5 nM), homogenized by

centrifugation at 500 rpm/min for 5 min, followed by incubated at 27°C for 60 min.

d. UNIFILTER-96 GF/B filter plate was pre-incubated with 0.5% PEI at 4 °C for 1 hour, and the UNIFILTER-96 GF/B filter plate (0.5% PEI) was rinsed with 1 mL washing solution twice, then the mixing solution containing the cell membrane was transferred to the UNIFILTER-96 GF/B filter plate (0.5% PEI), and washed with washing buffer 4 times, with 50 mL each time.

e. Dry at 55 °C for 10 minutes.

f. 40 μL of ULTIMA GOLD scintillation liquid was added to each well and the scintillation counts were read by using a Universal Harvester to determine the radioactivity bound to the membrane.

Each test compound was tested at 10 concentrations for its binding rate (% inhibition), and its $IC_{50}$ (the concentration at which 50% of binding is inhibited) was determined from a plot with the logarithm of concentration on the X-axis and response counts on the Y-axis. Data were processed using Xl-fit 5.3.1 software. The nonlinear regression equation is:

$$Y = \text{Minimum} + (\text{Maximum} - \text{Minimum})/(1 + 10^\wedge(\log(IC_{50}\text{-}X)\times \text{Hill Coefficient}))$$

X = logarithm of compound concentration;

Y = percentage of inhibition (% inhibition rate);

Maximum and Minimum: Y values at the top and bottom plateaus of the curve;

$LogIC_{50}$: same as the logarithmic units of the X-axis; and

Hill coefficient: Slope factor or Hill coefficient.

The binding ability of representative compounds of the present application to receptor cell membranes was measured by using radiolabeled [$^3$H]-DAMGO (PerkinElmer), [$^3$H]-Nociceptin (PerkinElmer), [$^3$H]-U69593 (PerkinElmer) as displaceable ligand. Ki value was determined by the formula Ki = $IC_{50}$/(1 + L/Kd), where Kd is binding affinity of the [$^3$H]-radioligand, L is the concentration of the [$^3$H]-radioligand used. The results of the experiments are shown in Table 1 below, where "/" indicates no test data.

Table 1 $K_i$ Values of Affinity and Selectivity determined for the compounds of the present invention

| Compound | $K_i$ (nM) of Affinity of opioid receptor | | | Selectivity | |
|---|---|---|---|---|---|
| | *h*MOR | *h*NOPr | *h*KOR | *h*MOR/*h*NOPr | *h*KOR/*h*NOPr |
| DAMGO | 0.74 | / | / | | |
| Nociceptin | / | 0.15 | / | | |
| Naltrexone hydrochloride | / | / | 0.23 | | |
| **1** (Example 1) | 102.31 | 4.89 | 1368.83 | 20.92 | 279.92 |
| **2** (Example 2) | 55.60 | 0.30 | 1146.41 | 185.33 | 3821.37 |
| **4** (Example 3) | 374.78 | 268.54 | / | 1.40 | |
| **5** (Example 4) | 167.59 | 5.97 | 812.88 | 28.07 | 136.16 |
| **6** (Example 5) | 2647.63 | 181.88 | / | 14.56 | |
| **7** (Example 6) | 2618.10 | 1445.54 | / | 1.81 | |
| **8** (Example 7) | 108.19 | 32.69 | 224.30 | 3.31 | 6.86 |
| **9** (Example 8) | 141.46 | 72.60 | 1095.62 | 1.95 | 15.09 |
| **10** (Example 9) | 17.27 | 3.01 | 1162.04 | 5.74 | 386.06 |
| **11** (Example 10) | 94.16 | 36.61 | 365.55 | 2.57 | 9.98 |

(continued)

| Compound | $K_i$ (nM) of Affinity of opioid receptor | | | Selectivity | |
|---|---|---|---|---|---|
| | *h*MOR | *h*NOPr | *h*KOR | *h*MOR/*h*NOPr | *h*KOR/*h*NOPr |
| **12** (Example 11) | 178.43 | 73.89 | 157.70 | 2.41 | 2.13 |
| **13** (Example 12) | 66.86 | 56.53 | 593.55 | 1.18 | 10.50 |
| **14** (Example 13) | 289.90 | 19.97 | 434.10 | 14.52 | 21.74 |
| **15** (Example 14) | 237.59 | 4.78 | 345.14 | 49.71 | 72.21 |
| **16** (Example 15) | 223.87 | 32.56 | 490.77 | 6.88 | 15.07 |
| **17** (Example 16) | 170.82 | 104.33 | 207.07 | 1.64 | 1.98 |
| **18** (Example 17) | 161.96 | 13.69 | 306.54 | 11.83 | 22.39 |
| **20** (Example 18) | 223.30 | 2.55 | 226.04 | 87.57 | 88.64 |
| **22** (Example 20) | 180.82 | 64.01 | 983.98 | 2.82 | 15.37 |
| **23** (Example 21) | 75.07 | 3.81 | 9.41 | 19.70 | 2.47 |
| **25** (Example 22) | 69.54 | 6.77 | 29.43 | 10.27 | 4.35 |
| **31** (Example 25) | 4.16 | 1.75 | 135.79 | 2.38 | 77.59 |
| **32** (Example 26) | 7.88 | 0.83 | 112.58 | 9.49 | 135.64 |
| **34** (Example 28) | 98.92 | 52.94 | 296.78 | 1.87 | 5.61 |
| Conclusion: The above affinity test results indicate that the compounds of the present invention exhibit high affinity and high selectivity for NOPr. | | | | | |

Experimental Example 2: Measurement of the agonistic function of the compounds of the present invention on MOR, NOPr, and KOR (cAMP method)

Opioid receptors are G protein-coupled receptors, primarily coupled to $G_i$ proteins. Upon binding with a ligand and activation, they can inhibit the activity of adenylate cyclase via Gi proteins, thereby reducing intracellular cAMP levels. A cAMP kit (PerkinElmer) was used to detect the agonistic or inhibitory effects of compounds on three opioid receptors (MOR, KOR, NOPr). The cAMP assay was a competitive immunoassay used to detect the accumulation of intracellular cAMP. The signal value measured was negatively correlated with the concentration of cAMP.

Experimental materials

(1) Main equipments: EnVision Multifunctional Plate Reader (PerkinElmer), 384-Well Microplate (PerkinElmer)

(2) Main reagents: Fetal Bovine Serum (FBS, AUS Gene X), F12 medium (Hyclone), penicillin-streptomycin solution 100 × (Gibco), Hygromycin B Gold (HB, Invivogen), Forskolin (adenylate cyclase activator, Selleck), Bovine Serum Albumin (BSA, PerkinElmer), IBMX (Sigma), cAMP kit (PerkinElmer), HEPES buffer (Gibco), HBSS buffer (Sigma), Endomorphin 1 (MCE), Dynorphin A1-10 (MCE), Nociceptin (MCE)

(3) Cell line: Human embryonic kidney cells 293 stably expressing human MOR or KOR or NOPr (293-MOR, 293-KOR, 293-NOPr)

Experimental Methods

(1) Cell Culture and Reagent Preparation

Complete medium:

$$\text{DMEM} + 10\%\ \text{FBS} + 5\%\ \text{Dual-antibody solution} + 200\ \mu\text{g/mL HB}$$

Buffer:

$$1 \text{ x HBSS} + 20 \text{ mM HEPES} + 0.1\% \text{ BSA} + 500 \text{ }\mu\text{M IBMX}$$

Vehicle system: Dissolve the compounds to be tested with DMSO into stock solutions at various concentrations, and dilute with buffer before testing.

Compounds to be tested: Compounds of the present invention, positive control (Endomorphin 1, Dynorphin A1-10, Nociceptin), vehicle control (DMSO).

(2) cAMP Test

a. Re-suspend 293-MOR or 293-KOR or 293-NOPr cells in buffer, adjust the density to 20,000 cells/well, and seed them into 384-well test plates.

b. Prepare 8 × drug-containing solution with buffe.

c. Add 2.5 μL of 8 × drug-containing solution to the cell plate and incubate at 37 °C for 10 min.

d. Prepare 8 × forskolin solution (8 μM) with experimental buffer.

e. Add 2.5 μL of 8 × forskolin solution to the cell plate and incubate at 37 °C for 30 min.

f. Dilute the cAMP tracer (1:50) according to the operating instructions of the cAMP kit, and add 10 μL to each well of the cell plate.

g. Dilute Ulight-anti-cAMP (1:50) according to the operating instructions of the cAMP kit, and add 10 μL to each well of the cell plate.

h. Incubate for 1 h at room temperature.

j. Read the absorbance at wavelengths of 665 nm and 615 nm on an EnVision multifunction plate reader.

Data Analysis

(1) The calculation method of the relative activity of the compounds to be tested is as follows:

$$\%\text{activity} = (\text{Signal}_{\text{cmpd}} - \text{Signal}_{\text{ave_VC}})(\text{Signal}_{\text{ave_PC}} - \text{Signal}_{\text{ave_VC}}) \times 100$$

$\text{Signal}_{\text{cmpd}}$: Absorbance signal of the compounds to be tested
$\text{Signal}_{\text{ave_VC}}$: Absorbance signal of normal group, without drug, without Forskolin
$\text{Signal}_{\text{ave_PC}}$: Absorbance signal of control group, without drug, with Forskolin

(2) Calculate $EC_{50}$ and plot the effect-dose curve of the compounds to be tested:

$$Y = \text{Bottom} + (\text{Top-Bottom})/(1+10\text{^}((\text{LogEC}_{50}\text{-X})\text{*HillSlope})$$

X: logarithm of concentration of the agonists; Y: % activity.

According to the above method, the compounds of the present application were tested, "NA" means no test data, and the results are shown in Table 2 below:

Table 2 $EC_{50}$ values determined for compounds of the present invention

| Compound | hMOR | | hNOPr (ORL-1 receptor) | | hKOR | |
|---|---|---|---|---|---|---|
| | $EC_{50}$(nM) | $E_{max}$ (%) | $EC_{50}$(nM) | $E_{max}$ (%) | $EC_{50}$(nM) | $E_{max}$ (%) |
| Endomorphin 1 | 2.22 | 103 | / | / | / | / |
| Nociceptin | / | / | 0.66 | 96.94 | / | / |
| Dynorphin A1-10 | / | / | / | / | 0.65 | 102.50 |
| **1** (Example 1) | 539.70 | 75.80 | 8.77 | 92.25 | 2248.00 | 55.37 |
| **2** (Example 2) | 452.50 | 80.52 | 0.10 | 103.10 | 1715.00 | 103.00 |
| **5** (Example 4) | 1136.00 | 86.94 | 3.12 | 104.10 | 811.50 | 57.26 |
| **8** (Example 7) | 437.40 | 77.24 | 21.71 | 104.70 | 473.70 | 68.93 |
| **15** (Example 14) | 1620.00 | 89.92 | 7.35 | 100.00 | 5356.00 | 56.76 |
| **18** (Example 17) | 710.90 | 87.56 | 10.74 | 99.74 | 714.90 | 70.20 |
| **20** (Example 18) | 920.00 | 101.00 | 9.20 | 96.53 | 1154.00 | 82.79 |
| **31** (Example 25) | 22.81 | 90.71 | 6.47 | 96.77 | 560.60 | 67.29 |
| **32** (Example 26) | 29.99 | 92.52 | 5.85 | 98.55 | 601.40 | 84.77 |
| **37** (Example 30) | 257.6 | 92.2 | 3.5 | 100.3 | 477.5 | 49.84 |
| **42** (Example 31) | 416.6 | 92.2 | 48.6 | 99.5 | 491.3 | 84.67 |
| **43** (Example 32) | 158.7 | 101.0 | 24.5 | 98.3 | 2056.0 | 73.3 |
| **47** (Example 34) | 691.1 | 118.7 | 19.6 | 84.3 | 2264.2 | 56.4 |

Conclusion: The above cAMP agonist function test results show that the compounds of the present invention, which have high affinity for NOPr, and also exhibite good agonistic biological activity on NOPr ($E_{max}$ > 80%); they are full agonists of NOPr and possess good selectivity.

Experimental Example 3: Pharmacodynamic tests of the compounds of the present invention in formalin-induced inflammatory pain model

1. Experimental Principle

A continuous nociceptive stimulus can be produced by injecting 2% formalin solution subcutaneously into the dorsum of the left hind paw of rats using a micro-injection needle, which caused the animals to exhibit spontaneous pain behavioral responses (paw lifting, paw licking, etc.). In the experiment, an autonomous movement analyzer can be used to record the number of times of paw lifting and paw licking by the rats, so as to evaluate their degree of pain.

2. Experimental Instruments and Reagents

Morphine: Shenyang No. 1 Pharmaceutical Co., Ltd. of Northeast Pharmaceutical Group; DMSO: Hubei Xingfa Chemical Group Co., Ltd.; HS-15: Cencord; 0.9% NaCl Injection: Wuhan Binhu Shuanghe Pharmaceutical Co., Ltd.; Formaldehyde: Xilong Scientific Co., Ltd.; Electronic Balance: Shanghai Ranhao Electronics Co., Ltd. (JCS-51002C); Autonomous Movement Analyzer: Anhui Zhenghua Biological Instrument Equipment Co., Ltd. (ZH-PAN801).

3. Solution Formulation and Administration

The vehicle formulas for the test substances (compounds of the present invention) were 5% DMSO + 10% HS-15 + 85% 0.9% Sodium Chloride Injection; they were formulated into drug solutions of 0.2 mg/mL before administration, with a dosage volume of 5 mL/kg, administered via single subcutaneous (s.c.) injection. The vehicle for the control substance morphine was 0.9% Sodium Chloride Injection; it was formulated into a drug solution of 0.6 mg/mL before administration, with a dosage volume of 5 mL/kg, administered via single subcutaneous injection.

4. Experimental process

(1) Adaptive Feeding

SPF-grade male SD rats were purchased from Hunan SJA Laboratory Animal Co., Ltd. (Laboratory Animal Production License No.: SCXK (Xiang) 2019-0004). After 4 days of adaptive feeding, the rats were transferred from the breeding room to the laboratory every morning. A layer of training urine pad was placed on the experimental table, and a transparent white cylinder was placed on the training urine pad. Each rat was weighed individually and then placed into the testing transparent white cylinder to adapt to the testing environment for ≥1 h. This adaptation procedure was conducted for at least 3 consecutive days.

(2) Test of Analgesic Activity of Test Substances

After 3 consecutive days of adaptation to the experimental environment for the rats, rats weighing 220-240 g were numbered at the base of their tails; rats not meeting the weight criterion continued to be raised until they reached the standard weight. Thirty minutes before the formal test, a metal plate was attached to the left hind paw of each rat, and the rats were placed individually into the transparent white cylinders for adaptation. Fifteen minutes before the test, a single subcutaneous injection of either the control compound Morphine (3 mg/kg) or the representative compounds of the present invention was administered (test compounds, animal grouping, administration route, and dosage are shown in the table below). Subsequently, the model was established with formalin (the blank control group did not receive formalin injection). Immediately after formalin-induced modeling, the animals were placed into an automated motion analyzer, and the number of behaviors such as paw withdrawal, paw lifting, and paw licking in rats was automatically recorded. Data from 0 to 60 minutes post-formalin injection were statistically analyzed.

(3) Detection Indicators

After subcutaneous injection of formalin into the dorsum of the left hind paw of the rats, the rats were placed into the detection chamber. The number of movements (including lifting and licking of the injected paw) during the 0-60 minute period following formalin injection was recorded and analyzed. The Maximum Possible Effect percentage (MPE%) was calculated as follows.

$$\text{MPE\%} = [(\text{Vehicle-Sham})-(\text{Administration group-Sham})]/(\text{Vehicle-Sham}) \times 100\%$$

Notes: ① The MPE of the sham (model) group = 100%, and the MPE of the vehicle (blank control) group = 0%, serving as the standard for calculating MPE% for all groups. ② The movement counts of the sham group were subtracted to eliminate the influence of background noise on the experiment. ③ sham represents the mean value of the sham group; (vehicle - sham) represents (mean value of vehicle group - mean value of sham group).

(4) Data Collection and Statistical analysis

All measured and observed results and data were entered twice into Excel spreadsheets and subjected to statistical analysis. All indicators were expressed as mean ± standard deviation. One-way analysis of variance (One-way ANOVA) was used to compare whether there were statistical differences among groups. $p < 0.05$ indicates a statistical difference.

5. Test results

Table 3 Analgesic activity of compounds of the present invention in formalin-induced inflammatory pain model

| Compound No. | Number of animals (n) | Modes of Administration | Administered dose | MPE% |
|---|---|---|---|---|
| Morphine | 6 | s.c | 3 mg/kg | **75.9±28.9** **** |
| **1** (Example 1) | 6 | s.c | 3 mg/kg | **79.0±4.6** **** |
| **2** (Example 2) | 6 | s.c | 1 mg/kg | **100.5±5.7** **** |
| **18** (Example 17) | 6 | s.c | 3 mg/kg | **78.2±21.8**** |
| **20** (Example 18) | 6 | s.c | 1 mg/kg | **64.5±15.3** **** |

(continued)

| Compound No. | Number of animals (n) | Modes of Administration | Administered dose | MPE% |
|---|---|---|---|---|
| **37** (Example 30) | 6 | s.c | 1 mg/kg | **55.8±14.8 **** |
| Note: * *, *P*<0.01; ***, *P*<0.001; ****, *P*<0.0001; | | | | |

The above experimental results indicate that the representative compounds of the present invention have a better inhibitory effect on formalin-induced inflammatory pain.

Experimental Example 4: Effect of the compounds of the present invention on gastrointestinal peristalsis in SD rats

1. Experimental Principle

The movement speed of digestive tract contents is related to gastric emptying time, small intestine peristalsis, and the fluidity of digestive tract contents. Drugs that could promote or inhibit gastrointestinal peristalsis can all cause changes in gastrointestinal peristalsis function, thereby affecting the transit speed of contents in the intestine. Charcoal suspension is not absorbed in the intestine. Using charcoal as an indicator, the effect of the test drug on gastrointestinal peristalsis can be observed by measuring the transit distance of charcoal in the intestine within a certain period.

2. Experimental Instruments and Reagents

Morphine: Shenyang No. 1 Pharmaceutical Co., Ltd. of Northeast Pharmaceutical Group; DMSO: Hubei Xingfa Chemical Group Co., Ltd.; HS-15: Cencord; 0.9% NaCl Injection: Wuhan Binhu Shuanghe Pharmaceutical Co., Ltd.; Charcoal: Jiangsu Kanghong Carbon Industry Co., Ltd.; Gum Arabic: Taian Dingli Gum Industry Co., Ltd.; Electronic Balance: Shanghai Ranhao Electronics Co., Ltd. (JCS-51002C).

3. Solution Formulation and Administration

Formulation of 10% Charcoal Suspension: Weigh 1.5 g of gum arabic, add 88.5 mL of water, stir to dissolve, then add 10 g of charcoal powder, stir fully to prepare a uniform suspension for later use.

The vehicle formulas for the compounds of the examples of the present invention were 5% DMSO + 10% HS-15 + 85% 0.9% Sodium Chloride Injection. Before administration, drug solutions with concentrations of 0.2, 0.6, and 2 mg/mL were respectively formulated. The administration volume was 5 mL/kg, administered via a single subcutaneous injection.

The vehicle for the control substance Morphine was 0.9% Sodium Chloride Injection. Before administration, a drug solution with a concentration of 0.6 mg/mL was formulated. The administration volume was 5 mL/kg, administered via a single subcutaneous injection.

4. Experimental process

(1) Adaptive Feeding

SPF-grade male SD rats were purchased from Hunan SJA Laboratory Animal Co., Ltd. (Laboratory Animal Production License No.: SCXK (Xiang) 2019-0004). They were housed in cages with 6 rats per group. Before the experiment, all experimental animals were acclimatized in the breeding room for 5 days to adapt to the breeding environment. The breeding environmental temperature was maintained at 18~26 °C, humidity at 40~70%, with sufficient water and feed provided, and a 12-hour light-dark cycle was maintained daily.

(2) Experimental Operation

Grouping: All rats were fasted for 20 hours before the experiment day with free access to water. After random grouping, the rats were numbered. The Model Group, Positive Control Group, and Test Substance Low, Medium, and High Dose Groups were set up sequentially, with 6 rats per group.

Modeling and Administration: The Positive Control Group Morphine (3 mg/kg) and each dose group of the compounds of the examples of the present invention were subcutaneously injected with the corresponding drugs, respectively. The Model Group was subcutaneously administered with an equal volume of vehicle (administration volume for all groups was 5 mL/kg). After 15 minutes, each group was gavaged with 10% charcoal suspension (volume 10 mL/kg). After 60 minutes, the rats were sacrificed, the abdominal cavity was opened via dissection, and the intestinal tract from the

pylorus to the ileocecal part was excised. The intestine was placed on a tray and stretched into a straight line. A ruler was used to precisely measure the transit distance of the contents (charcoal) and the total length of the small intestine, and the charcoal propulsion rate was calculated.

(3) Detection Indicators

The charcoal propulsion rate was calculated using the charcoal transit distance and the total length of the small intestine for each group, respectively. The differences in charcoal propulsion rates among groups were compared to determine the effect of the drugs on gastrointestinal peristalsis in rats.

Charcoal Propulsion Rate (%) = [Charcoal Transit Distance (cm)/Total Small Intestine Length (cm)] $\times$ 100%.

(4) Data Collection and Statistical Analysis

The measured data were entered into Excel spreadsheets and subjected to statistical analysis. Each indicator is expressed as mean $\pm$ standard deviation. One-way Analysis of Variance (One-way ANOVA) was used to compare whether there were statistical differences among groups. $p < 0.05$ indicates a statistical difference.

5. Conclusion

The positive control morphine (3 mg/kg) has a significant inhibitory effect on the gastrointestinal peristalsis in rats. At a dose equipotent to 3 mg/kg morphine in analgesic activity, the compounds in the examples of the present invention have no significant effect on the gastrointestinal peristalsis function in rats.

Although the embodiments disclosed in the present application are as above, the contents described are only the embodiments adopted for the convenience of understanding the present application, and are not used to limit the present application. Any person skilled in the art to which this application belongs may make any modifications and changes in the form and details of implementation without departing from the idea and scope disclosed in this application, but the scope of protection of this application shall still be subject to the scope defined in the appended claims.

## Claims

**1.** A compound having general formula (I), a stereoisomer thereof, or a pharmaceutically acceptable salt thereof;

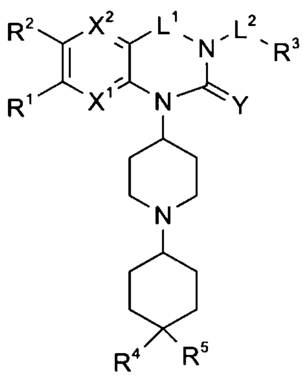

(I)

wherein,

$R^1$ and $R^2$ are each independently selected from hydrogen, halogen, $C_{1-6}$ alkyl, halogenated $C_{1-6}$ alkyl, $C_{1-6}$ alkoxy, halogenated $C_{1-6}$ alkoxy, and provided that: $R^1$ and $R^2$ are not simultaneously hydrogen;
$R^3$ is selected from $-C(O)NR^aR^b$, $-S(O)_2NR^aR^b$, $-S(O)(NR^a)R^b$, $-S(O)R^c$, $-S(O)_2R^c$, $-NHS(O)_2R^c$, $-NHC(O)R^d$, $-C(O)OR^d$, 5- to 10-membered heteroaryl, and said 5- to 10-membered heteroaryl is optionally substituted with one or more $R^e$;
$R^4$ and $R^5$ are each independently selected from hydrogen, halogen, $C_{1-6}$ alkyl, $C_{1-6}$ alkoxy; or $R^4$ and $R^5$ together with a carbon atom to which they are attached form $C_{3-8}$ cycloalkyl; or $R^4$ and $R^5$ together with a carbon atom to

which they are attached form a 3- to 8-membered heterocyclyl, $R^4$ and $R^5$ can be the same or different, and provided that: $R^4$ and $R^5$ are not simultaneously hydrogen, wherein said $C_{1-6}$ alkyl, $C_{1-6}$ alkoxy, $C_{3-8}$ cycloalkyl and 3- to 8-membered heterocyclyl are each optionally substituted with one or more R';

$X^1$ and $X^2$ are each independently selected from N and $CR^f$;

$L^1$ is selected from a single bond and $-CR^gR^h-$;

$L^2$ is selected from $C_{1-6}$ alkylene group, and said $C_{1-6}$ alkylene group is optionally substituted with one or more $R^m$;

Y is selected from O and S;

$R^a$ and $R^b$ are each independently selected from hydrogen, hydroxyl, $C_{1-6}$ alkyl, $C_{3-6}$ cycloalkyl, $-(CH_2)_n-C_{3-6}$ cycloalkane, $-(CH_2)_n-OH$, wherein said $C_{1-6}$ alkyl, $C_{3-6}$ cycloalkyl, $-(CH_2)_n-C_{3-6}$ cycloalkane, $-(CH_2)_n-OH$ are each optionally substituted with one or more R', and $R^a$ and $R^b$ may be the same or different;

each $R^c$ is independently selected from $C_{1-6}$ alkyl, $C_{3-6}$ cycloalkyl, $C_{6-10}$ aryl, $-(CH_2)_n-C_{3-6}$ cycloalkane, $-(CH_2)_n-C_{6-10}$ aromatic hydrocarbon, wherein said $C_{1-6}$ alkyl, $C_{3-6}$ cycloalkyl, $C_{6-10}$ aryl, $-(CH_2)_n-C_{3-6}$ cycloalkane, $-(CH_2)_n-C_{6-10}$ aromatic hydrocarbon are each optionally substituted with one or more R';

each $R^d$ is independently selected from $C_{1-6}$ alkyl, $C_{3-6}$ cycloalkyl, $-(CH_2)_n-C_{3-6}$ cycloalkane, wherein said $C_{1-6}$ alkyl, $C_{3-6}$ cycloalkyl, $-(CH_2)_n-C_{3-6}$ cycloalkane are each optionally substituted with one or more R';

$R^e$, $R^f$ are each independently selected from hydrogen, halogen, $C_{1-3}$ alkyl, $-O-C_{1-3}$ alkane, wherein said $C_{1-3}$ alkyl, $-O-C_{1-3}$ alkane are each optionally substituted with one or more R';

$R^g$, $R^h$ and $R^m$ are each independently selected from hydrogen, halogen, $C_{1-3}$ alkyl, wherein said $C_{1-3}$ alkyl is optionally substituted with one or more R';

each R' is independently selected from H, F, Cl, Br, and I; and

n is an integer of 1, 2 or 3.

2. The compound according to claim 1, a stereoisomer thereof, or a pharmaceutically acceptable salt thereof, wherein $R^1$ and $R^2$ are each independently selected from hydrogen, halogen, $C_{1-3}$ alkyl, halogenated $C_{1-3}$ alkyl, $C_{1-3}$ alkoxy, halogenated $C_{1-3}$ alkoxy, and provided that: $R^1$ and $R^2$ are not simultaneously hydrogen; or $R^1$ and $R^2$ are each independently selected from hydrogen, halogen, $C_{1-3}$ alkyl, and provided that: $R^1$ and $R^2$ are not simultaneously hydrogen; or $R^1$ and $R^2$ are each independently halogen; or $R^1$ and $R^2$ are both fluorine or chlorine; or $R^1$ is chlorine and $R^2$ is fluorine; or $R^1$ is fluorine and $R^2$ is chlorine; or $R^1$ is chlorine and $R^2$ is methoxyl.

3. The compound according to claim 1 or 2, a stereoisomer thereof, or a pharmaceutically acceptable salt thereof, wherein $R^3$ is selected from $-C(O)NR^aR^b$, $-S(O)_2NR^aR^b$, $-S(O)R^c$, $-S(O)_2R^c$, $-NHS(O)_2R^c$, $-NHC(O)R^d$, $-C(O)OR^d$, and 5- to 6-membered heteroaryl, and said 5-to 6-membered heteroaryl is optionally substituted with one or more $R^e$; or $R^3$ is $-C(O)NR^aR^b$; or $R^3$ is $-S(O)_2NR^aR^b$; or $R^3$ is $-S(O)(NR^a)R^b$; or $R^3$ is $-S(O)R^c$; or $R^3$ is $-S(O)_2R^c$; or $R^3$ is $-NHS(O)_2R^c$; or $R^3$ is $-NHC(O)R^d$; or $R^3$ is-$C(O)OR^d$; or $R^3$ is 5- to 6-membered heteroaryl, and said 5- to 6-membered heteroaryl is optionally substituted with one or more $R^e$.

4. The compound according to claim 3, a stereoisomer thereof, or a pharmaceutically acceptable salt thereof, wherein $R^3$ is selected from $-C(O)N(CH_3)_2$, $-C(O)NH_2$, $-C(O)NHOH$, $-S(O)_2NH_2$, $-S(O)_2N(CH_3)_2$, $-S(O)(NH)CH_3$, $-S(O)CH_3$, $-S(O)CH_2CH_3$, $-S(O)_2CH_3$, $-S(O)_2CH_2CH_3$, $-S(O)_2CH(CH_3)_2$, $-C(O)NHCH_2CH_2OH$, $-C(O)NHCH_3$, $-C(O)NHCH_2CH_3$, $-NHC(O)CH_3$, $-NHC(O)CH_2CH_3$, $-C(O)OCH_2CH_3$, $-S(O)_2-Ph$, $-S(O)_2CH_2-Ph$,

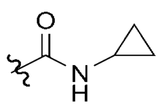,

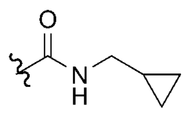, 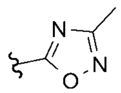,

$-NHS(O)_2CH_3$, $-NHS(O)_2CH_2CH_3$; or, $R^3$ is selected from $C(O)NHCH_3$, $-S(O)_2N(CH_3)_2$, $-S(O)CH_3$, $-S(O)_2CH_3$, $-S(O)_2CH_2CH_3$, $-NHS(O)_2CH_3$, $-NHS(O)_2CH_2CH_3$, $-NHC(O)CH_3$, $-NHC(O)CH_2CH_3$.

5. The compound according to any one of claims 1 to 4, a stereoisomer thereof, or a pharmaceutically acceptable salt thereof, wherein $R^4$ and $R^5$ are each independently selected from hydrogen, halogen, $C_{1-6}$ alkyl, $C_{1-6}$ alkoxy; or $R^4$ and $R^5$ together with the carbon atom to which they are attached form $C_{3-6}$ cycloalkyl; or $R^4$ and $R^5$ together with the carbon atom to which they are attached form a 3- to 6-membered heterocyclyl, $R^4$ and $R^5$ can be the same or different, and

provided that: $R^4$ and $R^5$ are not simultaneously hydrogen, wherein said $C_{1-6}$ alkyl, $C_{1-6}$ alkoxy, $C_{3-6}$ cycloalkyl and 3- to 6-membered heterocyclyl are each optionally substituted with one or more R'; or $R^4$ is hydrogen, $R^5$ is selected from halogen, $C_{1-6}$ alkyl, $C_{1-6}$ alkoxy, wherein said $C_{1-6}$ alkyl, $C_{1-6}$ alkoxy are each optionally substituted with one or more R'; or $R^5$ is hydrogen, $R^4$ is selected from halogen, $C_{1-6}$ alkyl and $C_{1-6}$ alkoxy, wherein said $C_{1-6}$ alkyl, $C_{1-6}$ alkoxy are each optionally substituted with one or more R'; or $R^4$ and $R^5$ are each independently halogen; or $R^4$ and $R^5$ are each independently $C_{1-6}$ alkyl, wherein said $C_{1-6}$ alkyl is optionally substituted with one or more R'; or $R^4$, $R^5$ are each independently $C_{1-6}$ alkoxy, wherein said $C_{1-6}$ alkoxy is each optionally substituted with one or more R'; or $R^4$ and $R^5$ together with the carbon atom to which they are attached form $C_{3-6}$ cycloalkyl, wherein said $C_{3-6}$ cycloalkyl is optionally substituted with one or more R'; or $R^4$ and $R^5$ together with the carbon atom to which they are attached form a 3- to 6-membered heterocyclyl, wherein said 3- to 6-membered heterocyclyl is optionally substituted with one or more R'.

**6.** The compound according to claim 5, a stereoisomer thereof, or a pharmaceutically acceptable salt thereof, wherein $R^4$ and $R^5$ are each independently selected from hydrogen, halogen, $C_{1-6}$ alkyl, or $R^4$ and $R^5$ together with the carbon atom to which they are attached form $C_{3-6}$ cycloalkyl, or $R^4$ and $R^5$ together with the carbon atom to which they are attached form a 3-to 6-membered heterocyclyl, $R^4$ and $R^5$ can be the same or different, and provided that $R^4$ and $R^5$ are not simultaneously hydrogen; or $R^4$ and $R^5$ are each independently selected from hydrogen, halogen, $C_{1-3}$ alkyl, or $R^4$ and $R^5$ together with the carbon atom to which they are attached form $C_{3-4}$ cycloalkyl, or $R^4$ and $R^5$ together with the carbon atom to which they are attached form a 3-4 membered heterocyclyl, $R^4$ and $R^5$ can be the same or different, and provided that $R^4$ and $R^5$ are not simultaneously hydrogen; or both $R^4$ and $R^5$ are methyl, or one of $R^4$ and $R^5$ is isopropyl and the other is hydrogen, or $R^4$ and $R^5$ together with the carbon atom to which they are attached form cyclopropyl, or $R^4$ and $R^5$ together with the carbon atom to which they are attached form cyclobutyl, or $R^4$ and $R^5$ together with the carbon atom to which they are attached form oxetanyl.

**7.** The compound according to any one of claims 1 to 6, a stereoisomer thereof, or a pharmaceutically acceptable salt thereof, wherein $X^1$ and $X^2$ are each independently selected from N and CH; or both of $X^1$ and $X^2$ are N; or $X^1$ is N, $X^2$ is CH; or $X^2$ is N, $X^1$ is CH; or both of $X^1$ and $X^2$ are CH.

**8.** The compound according to any one of claims 1 to 7, a stereoisomer thereof, or a pharmaceutically acceptable salt thereof, wherein $L^1$ is a single bond; or $L^1$ is $-CR^gR^h-$; or $L^1$ is selected from $-CH_2-$.

**9.** The compound according to any one of claims 1 to 8, a stereoisomer thereof, or a pharmaceutically acceptable salt thereof, wherein $L^2$ is selected from $C_{1-3}$ alkylene group, and said $C_{1-3}$ alkylene group is optionally substituted with one or more $R^m$; or $L^2$ is selected from $-CH_2-$, $-CH_2CH_2-$, and $-CH(CH_3)-$.

**10.** The compound according to any one of claims 1 to 9, a stereoisomer thereof, or a pharmaceutically acceptable salt thereof, wherein Y is O; or Y is S; and/or

one of $R^a$ and $R^b$ is selected from hydrogen and the other is selected from hydroxyl, $C_{1-3}$ alkyl, $C_{3-6}$ cycloalkyl, $-(CH_2)_n-C_{3-6}$ cycloalkane, $-(CH_2)_n-OH$, wherein said $C_{1-3}$ alkyl, $C_{3-6}$ cycloalkyl, $-(CH_2)_n-C_{3-6}$ cycloalkane, $-(CH_2)_n-OH$ are each optionally substituted with one or more R'; or one of $R^a$ and $R^b$ is selected from hydrogen and the other is selected from methyl, ethyl, cyclopropyl, cyclopropylmethyl, hydroxyl and hydroxyethyl; or both of $R^a$ and $R^b$ are $C_{1-3}$ alkyl; or both of $R^a$ and $R^b$ are hydrogen; and/or

each $R^c$ is independently selected from methyl, ethyl, propyl, isopropyl, cyclopropyl, cyclopropylmethyl, benzyl and phenyl; and/or

each $R^d$ is independently selected from methyl, ethyl, propyl, isopropyl, cyclopropyl and cyclopropylmethyl; and/or

$R^e$ and $R^f$ are each independently hydrogen; or $R^e$ and $R^f$ are each independently halogen; or $R^e$ and $R^f$ are each independently $C_{1-3}$ alkyl, and said $C_{1-3}$ alkyl is optionally substituted with one or more R'; or $R^e$ and $R^f$ are each independently $-O-C_{1-3}$ alkane, and said $-O-C_{1-3}$ alkane is optionally substituted with one or more R'; or $R^e$ and $R^f$ are each independently selected from hydrogen, fluorine, chlorine, bromine, methyl, methoxyl, trifluoromethyl and trifluoromethoxyl; and/or

$R^g$, $R^h$ and $R^m$ are each independently selected from hydrogen, fluorine and methyl; or $R^g$, $R^h$ and $R^m$ are each independently hydrogen; or $R^g$, $R^h$ and $R^m$ are each independently fluorine; or $R^g$, $R^h$ and $R^m$ are each independently methyl; and/or

each R' is independently selected from H, F, Cl, and Br; or each R' is independently selected from H, F and Cl; and/or

n is 1; or n is 2; or n is 3.

**11.** The compound according to any one of claims 1 to 10, a stereoisomer thereof, or a pharmaceutically acceptable salt thereof, wherein the compound is selected from one of the following chemical structural formula:

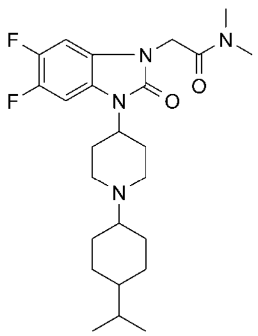

**A-1**

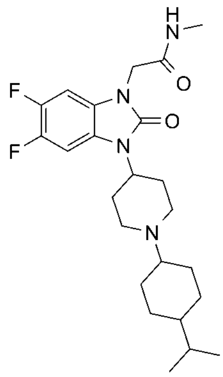

**A-2**

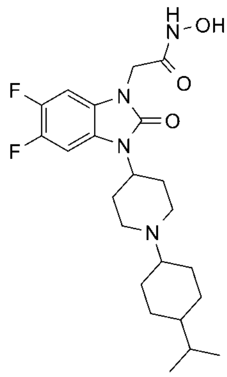

**A-3**

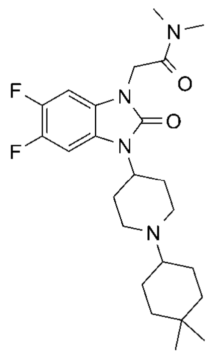

**4**

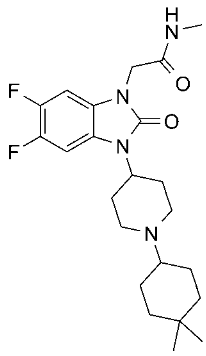

**5**

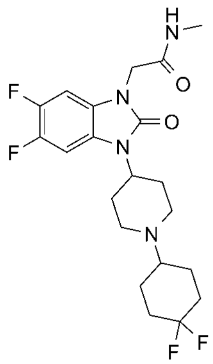

**6**

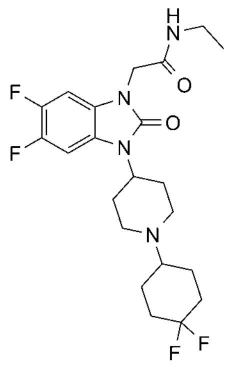

**7**

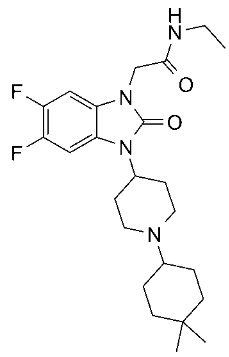

**8**

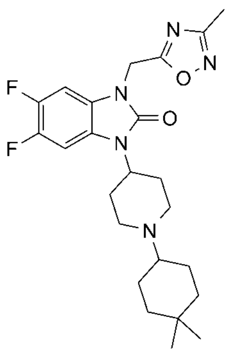

**9**

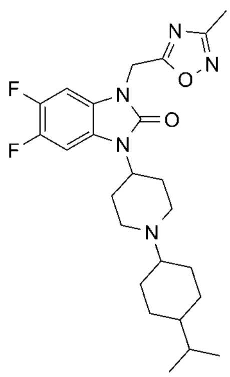

**A-10**

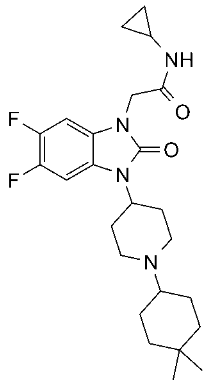

**11**

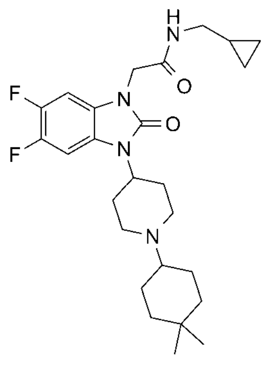

**12**

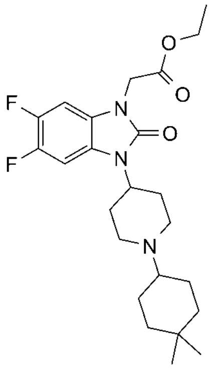

**13**

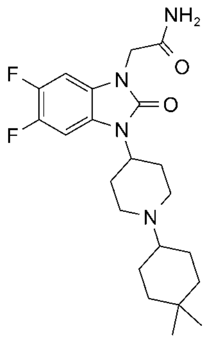

**14**

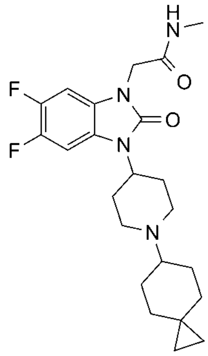

**15**

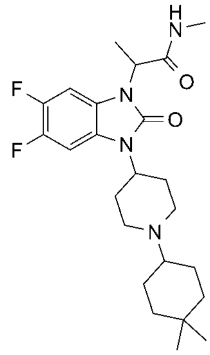

**16**

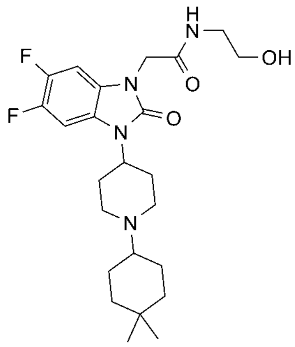

**17**

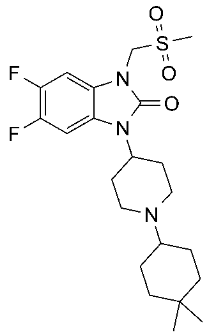

**18**

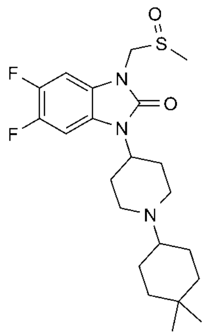

**19**

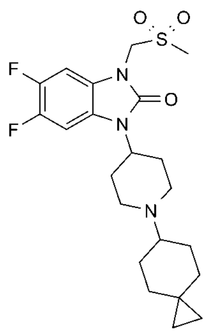

**20**

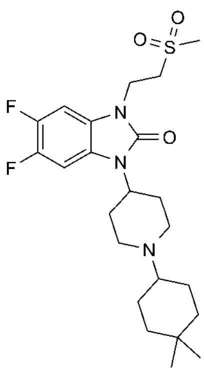

**21**

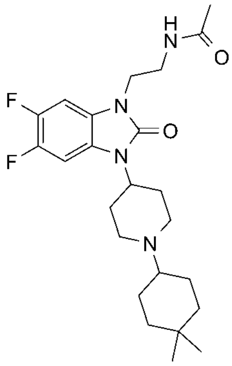

**22**

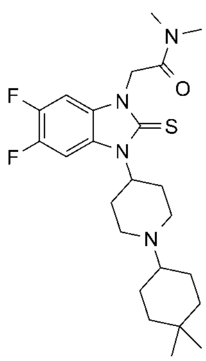

**23**

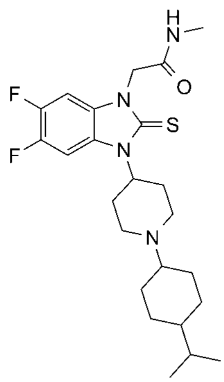

**A-24**

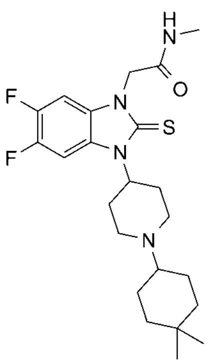

**25**

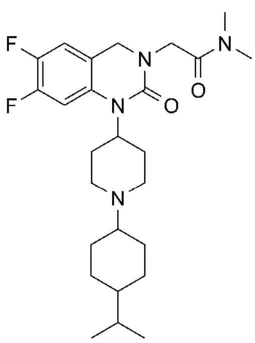

**A-26**

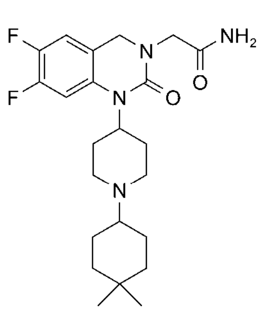

**27**

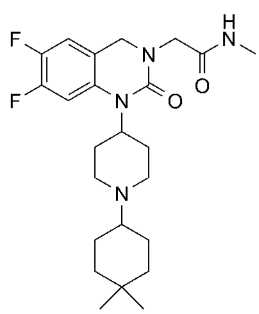

**28**

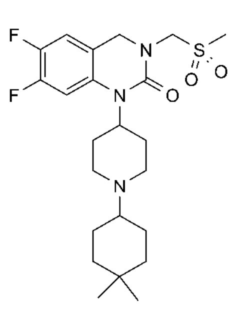
29

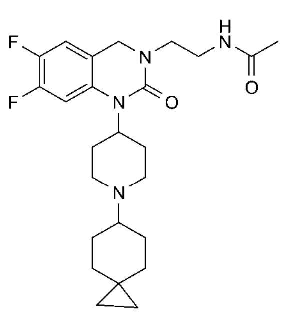
30

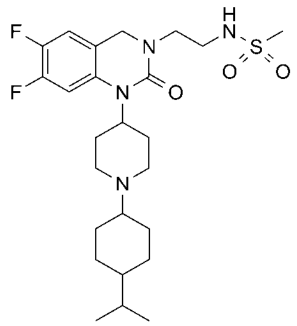
A-31

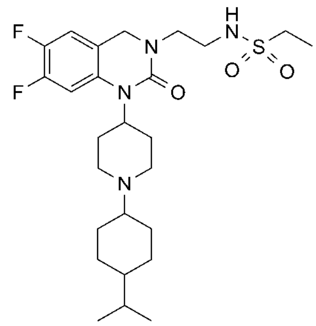
A-32

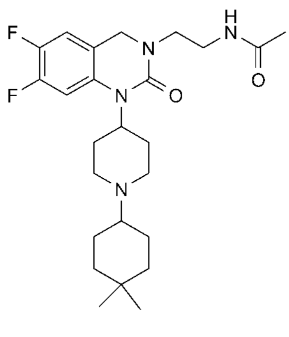
33

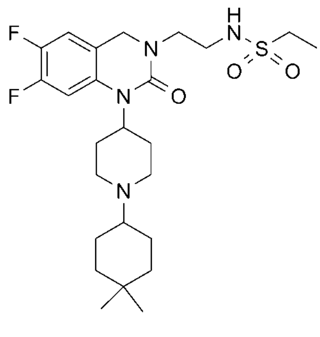
34

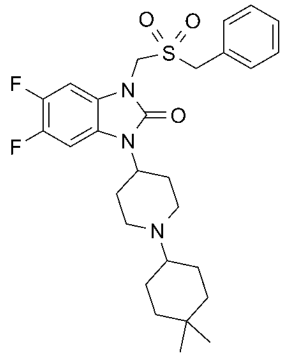
35

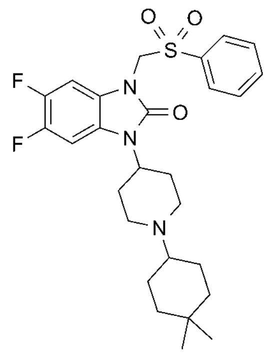
36

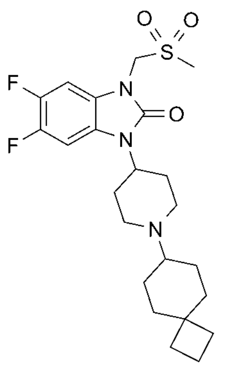
37

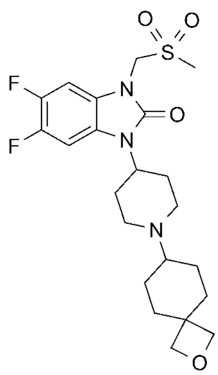
38

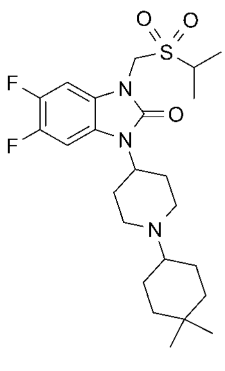
39

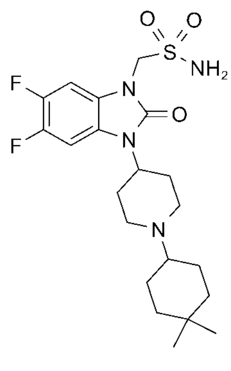
40

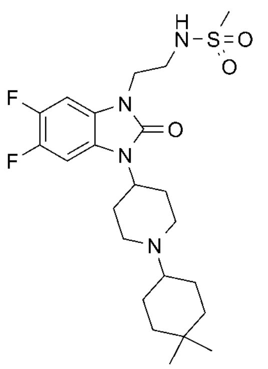
41

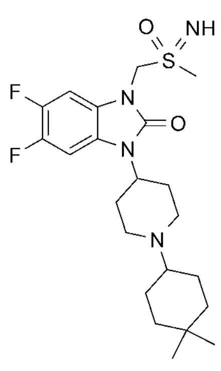
42

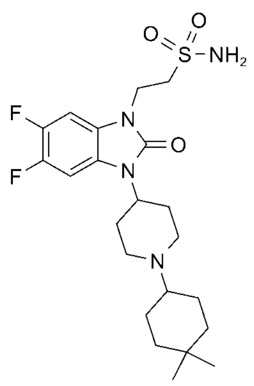
43

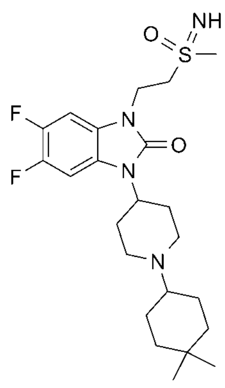
44

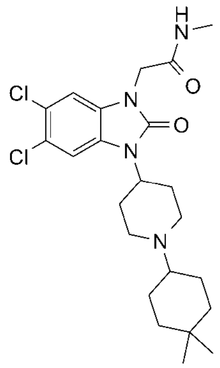 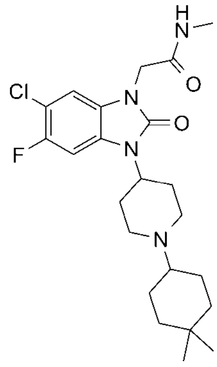 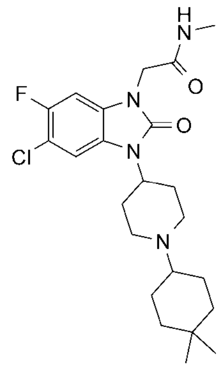 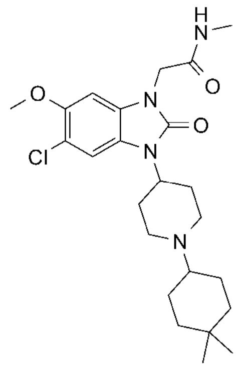

45 46 47 48 ;

and optionally, selected from one of the following chemical structural formula:

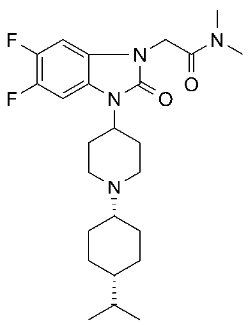 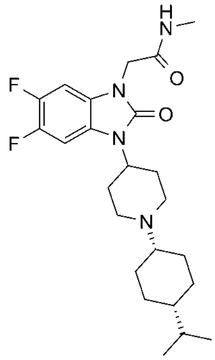 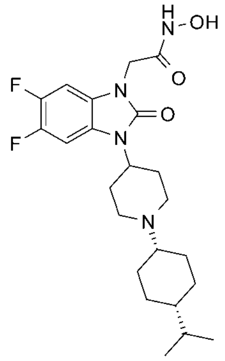 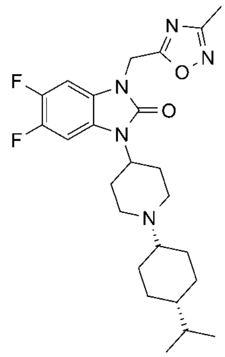

1 2 3 10

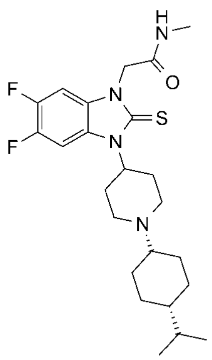 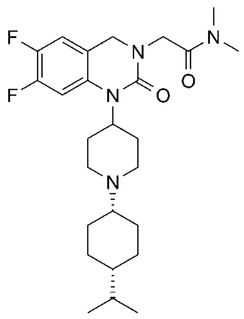 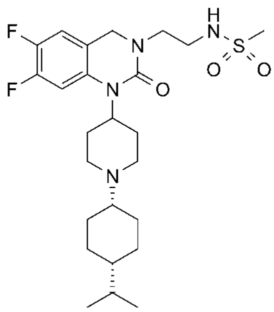 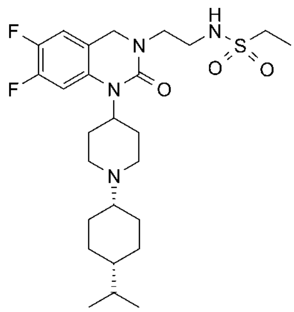

24 26 31 32 .

**12.** A method for preparing the compound according to claim 1, a stereoisomer thereof, or a pharmaceutically acceptable salt thereof, comprising the following steps:

(1) a compound of formula (I-1) undergoes a substitution reaction with a compound of formula (I-2) to yield a compound of formula (I-3);

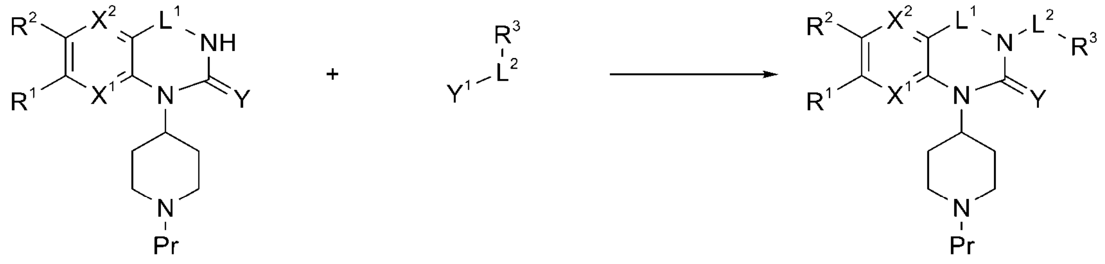

(I-1) (I-2) (I-3)

(2) the compound of formula (I-3) undergoes a deprotection reaction to yield a compound of formula (I-4), and then undergoes a reductive amination reaction with a compound of formula (I-5) to obtain the compound of formula (I);

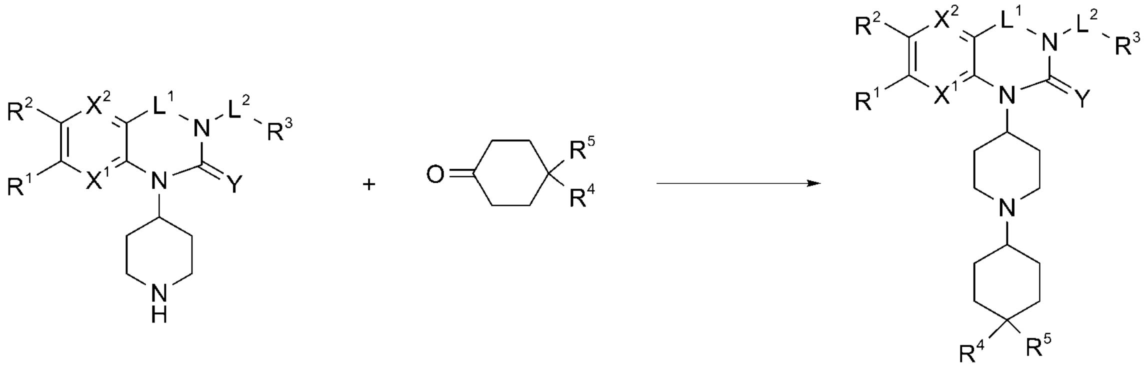

(I-4) (I-5) (I) ;

or,

(1') a compound of formula (I-6) undergoes a reductive amination reaction with the compound of formula (I-5) to yield a compound of formula (I-7);

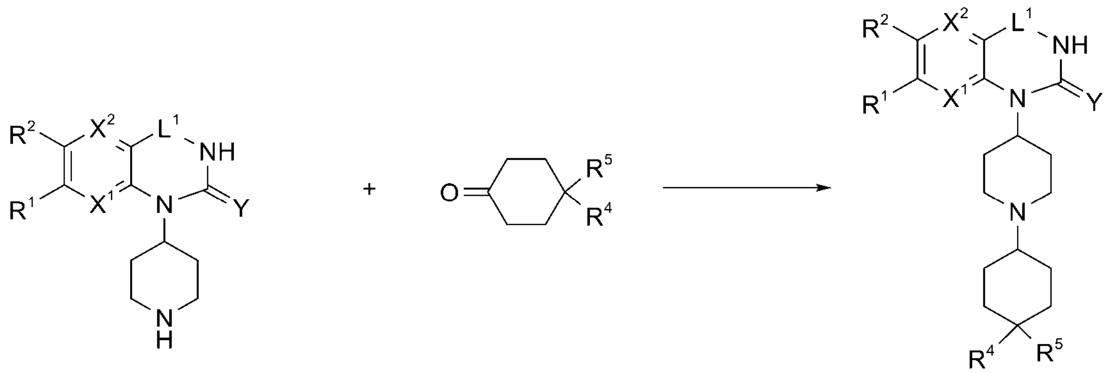

(I-6) (I-5) (I-7)

(2') the compound of formula (I-7) undergoes a substitution reaction with the compound of formula (I-2) to yield the compound of formula (I);

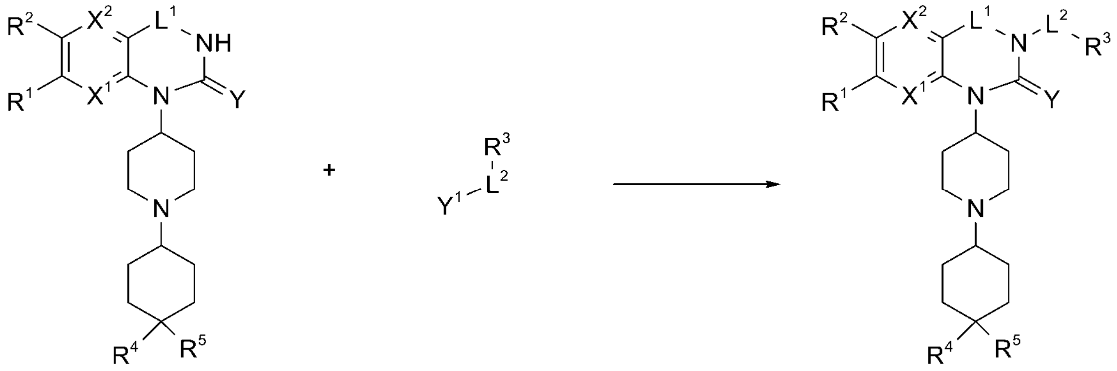

(I-7) (I-2) (I) ;

in the above preparation method, $Y^1$ in formula (I-2) represents a leaving group; Pr in formula (I-1) and formula (I-3) represent an amino protecting group; the definitions of other groups in formulas (I-1) to (I-7) and formula (I) are as defined in claim 1.

**13.** A pharmaceutical composition comprising the compound according to any one of claims 1 to 11, or a stereoisomer thereof, or a pharmaceutically acceptable salt thereof, and a pharmaceutically acceptable excipient.

**14.** The compound according to of any one of claims 1 to 11, a stereoisomer thereof, or a pharmaceutically acceptable salt thereof, or the pharmaceutical composition of claim 13, for use as a medicament.

**15.** The compound according to of any one of claims 1 to 11, a stereoisomer thereof, or a pharmaceutically acceptable salt thereof, or the pharmaceutical composition of claim 13, for use in the treatment and/or prevention of a disease associated with the NOP receptor.

**16.** Use of the compound according to of any one of claims 1 to 11, a stereoisomer thereof, or a pharmaceutically acceptable salt thereof, or the pharmaceutical composition of claim 13, in the manufacture of a medicament for the treatment and/or prevention of diseases associated with NOP receptors.

**17.** A method for the treatment and/or prevention of diseases associated with NOP receptors, comprising administering to a subject in need thereof a therapeutically effective amount of the compound according to of any one of claims 1 to 11, a stereoisomer thereof, or a pharmaceutically acceptable salt thereof, or the pharmaceutical composition of claim 13.

**18.** The compound, a stereoisomer thereof, or a pharmaceutically acceptable salt thereof, or the pharmaceutical composition for use in the treatment and/or prevention of a disease associated with the NOP receptor of claim 15, or the use of claim 16, or the method of claim 17, wherein the disease associated with the NOP receptor is selected from pain, cough, sleep disorders, hypertension, urinary incontinence, epilepsy, traumatic injury, eating disorders, anxiety, depression, alcohol addiction, drug withdrawal syndrome, memory loss caused by Alzheimer's disease or other dementias, preferably pain; optionally, the pain is selected from postoperative pain, cancer-induced pain, neuropathic pain, traumatic pain and inflammation-induced pain.

**INTERNATIONAL SEARCH REPORT**

International application No.
**PCT/CN2025/070679**

**A. CLASSIFICATION OF SUBJECT MATTER**

C07D401/04(2006.01)i； A61K31/454(2006.01)i； A61P25/04(2006.01)i

According to International Patent Classification (IPC) or to both national classification and IPC

**B. FIELDS SEARCHED**

Minimum documentation searched (classification system followed by classification symbols)

IPC： C07D/-， A61K/-， A61P/-

Documentation searched other than minimum documentation to the extent that such documents are included in the fields searched

Electronic data base consulted during the international search (name of data base and, where practicable, search terms used)

CNTXT, ENTXT, CNKI, STN (CAPLUS, REGISTRY, MARPAT): 检索结构式, search for structural formula, 环己基, 哌啶, 苯并咪唑, 二氢喹唑啉, 痛敏肽, 孤啡肽, 阿片受体样受体1, structure, cyclohexyl, piperidine, benzimidazole, dihydroquinazoline, nociceptin, orphanin FQ peptide, opioid receptor-like receptor 1, NOP, ORL-1

**C. DOCUMENTS CONSIDERED TO BE RELEVANT**

| Category* | Citation of document, with indication, where appropriate, of the relevant passages | Relevant to claim No. |
|---|---|---|
| X | CN 115611856 A (YICHANG HUMANWELL PHARMACEUTICAL CO., LTD. et al.) 17 January 2023 (2023-01-17)<br>description, paragraphs [0011], [0021]-[0032] and [0075], and embodiment 48B | 1-18 |
| A | US 2006264638 A1 (EURO-CELTIQUE S.A.) 23 November 2006 (2006-11-23)<br>description, paragraph [0009], [0018] and [0081], and embodiment 1 | 1-18 |
| A | CN 1288464 A (PFIZER PHARMACEUTICALS INC.) 21 March 2001 (2001-03-21)<br>entire document | 1-18 |
| A | US 2004067950 A1 (SCHERING-PLOUGH CORPORATION) 08 April 2004 (2004-04-08)<br>entire document | 1-18 |
| A | CN 101622241 A (MITSUBISHI TANABE PHARMA CORP.) 06 January 2010 (2010-01-06)<br>entire document | 1-18 |
| A | US 2014187535 A1 (SHIONOGI & CO., LTD. et al.) 03 July 2014 (2014-07-03)<br>entire document | 1-18 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 March 2025** | **25 March 2025** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2025/070679**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17-18**
because they relate to subject matter not required to be searched by this Authority, namely:
Claims 17-18 relate to a disease treatment method. The present search report is provided on the basis that the subject matter of said claims is the corresponding pharmaceutical use of the compound of the present application.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2025/070679**

| Patent document cited in search report | | | Publication date (day/month/year) | Patent family member(s) | | | Publication date (day/month/year) |
|---|---|---|---|---|---|---|---|
| CN | 115611856 | A | 17 January 2023 | EP | 4345096 | A1 | 03 April 2024 |
| | | | | EP | 4345096 | A4 | 13 November 2024 |
| | | | | WO | 2023284834 | A1 | 19 January 2023 |
| | | | | US | 2024327383 | A1 | 03 October 2024 |
| | | | | JP | 2024523683 | A | 28 June 2024 |
| | | | | CA | 3224323 | A1 | 19 January 2023 |
| | | | | KR | 20240022630 | A | 20 February 2024 |
| US | 2006264638 | A1 | 23 November 2006 | JP | 2007277257 | A | 25 October 2007 |
| | | | | ATE | 302770 | T1 | 15 September 2005 |
| | | | | EP | 1242085 | A1 | 25 September 2002 |
| | | | | EP | 1242085 | A4 | 12 February 2003 |
| | | | | EP | 1242085 | B1 | 24 August 2005 |
| | | | | AU | 1816901 | A | 12 June 2001 |
| | | | | US | 2002128288 | A1 | 12 September 2002 |
| | | | | US | 7456198 | B2 | 25 November 2008 |
| | | | | US | 7678809 | B2 | 16 March 2010 |
| | | | | WO | 0139775 | A1 | 07 June 2001 |
| | | | | DE | 60022226 | D1 | 29 September 2005 |
| | | | | JP | 2003524634 | A | 19 August 2003 |
| | | | | HK | 1049284 | A1 | 09 May 2003 |
| | | | | HK | 1049284 | B | 24 February 2006 |
| CN | 1288464 | A | 21 March 2001 | AR | 014421 | A1 | 28 February 2001 |
| | | | | ES | 2175925 | T3 | 16 November 2002 |
| | | | | EP | 1049689 | A1 | 08 November 2000 |
| | | | | EP | 1049689 | B1 | 26 June 2002 |
| | | | | DE | 69901934 | D1 | 01 August 2002 |
| | | | | DE | 69901934 | T2 | 07 November 2002 |
| | | | | JP | 2002509148 | A | 26 March 2002 |
| | | | | JP | 3342478 | B2 | 11 November 2002 |
| | | | | CO | 4990962 | A1 | 26 December 2000 |
| | | | | NO | 20003671 | D0 | 18 July 2000 |
| | | | | NO | 20003671 | L | 18 September 2000 |
| | | | | CA | 2317462 | A1 | 22 July 1999 |
| | | | | CA | 2317462 | C | 13 September 2005 |
| | | | | ATE | 219772 | T1 | 15 July 2002 |
| | | | | WO | 9936421 | A1 | 22 July 1999 |
| | | | | TR | 200002090 | T2 | 21 November 2000 |
| | | | | PA | 8467101 | A1 | 29 September 2000 |
| | | | | OA | 11445 | A | 30 April 2004 |
| | | | | BG | 104670 | A | 31 May 2001 |
| | | | | EA | 200000683 | A1 | 25 December 2000 |
| | | | | PE | 20000166 | A1 | 07 March 2000 |
| | | | | IS | 5539 | A | 16 June 2000 |
| | | | | MA | 26598 | A1 | 20 December 2004 |
| | | | | IL | 137149 | A0 | 24 July 2001 |
| | | | | PT | 1049689 | E | 30 September 2002 |
| | | | | GT | 199900008 | A | 12 July 2000 |
| | | | | ZA | 99314 | B | 18 July 2000 |
| | | | | BR | 9907104 | A | 24 October 2000 |
| | | | | AU | 1679099 | A | 02 August 1999 |

Form PCT/ISA/210 (patent family annex) (July 2022)

**INTERNATIONAL SEARCH REPORT**
**Information on patent family members**

International application No.
**PCT/CN2025/070679**

| Patent document cited in search report | | | Publication date (day/month/year) | Patent family member(s) | | | Publication date (day/month/year) |
|---|---|---|---|---|---|---|---|
| | | | | US | 6423725 | B1 | 23 July 2002 |
| | | | | TNSN | 99006 | A1 | 10 November 2005 |
| | | | | DK | 1049689 | T3 | 22 July 2002 |
| | | | | AP | 9901442 | A0 | 31 March 1999 |
| | | | | PL | 342046 | A1 | 21 May 2001 |
| US | 2004067950 | A1 | 08 April 2004 | None | | | |
| CN | 101622241 | A | 06 January 2010 | EP | 2128154 | A1 | 02 December 2009 |
| | | | | EP | 2128154 | A4 | 26 January 2011 |
| | | | | EP | 2128154 | B1 | 08 April 2015 |
| | | | | MX | 2009009355 | A | 11 September 2009 |
| | | | | CA | 2679530 | A1 | 04 September 2008 |
| | | | | CA | 2679530 | C | 25 September 2012 |
| | | | | KR | 20090115976 | A | 10 November 2009 |
| | | | | KR | 101165504 | B1 | 13 July 2012 |
| | | | | JPWO | 2008105497 | A1 | 03 June 2010 |
| | | | | JP | 5313865 | B2 | 09 October 2013 |
| | | | | US | 2010120841 | A1 | 13 May 2010 |
| | | | | US | 8877779 | B2 | 04 November 2014 |
| | | | | WO | 2008105497 | A1 | 04 September 2008 |
| | | | | AU | 2008219980 | A1 | 04 September 2008 |
| | | | | AU | 2008219980 | B2 | 12 January 2012 |
| | | | | JP | 2013107910 | A | 06 June 2013 |
| | | | | ES | 2541133 | T3 | 16 July 2015 |
| | | | | BRPI | 0808025 | A2 | 24 June 2014 |
| US | 2014187535 | A1 | 03 July 2014 | WO | 2014102594 | A2 | 03 July 2014 |
| | | | | WO | 2014102594 | A3 | 16 October 2014 |
| | | | | US | 9090618 | B2 | 28 July 2015 |
| | | | | US | 2015322066 | A1 | 12 November 2015 |
| | | | | US | 9598411 | B2 | 21 March 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202410019096X
- WO 2003082333 A1
- WO 2001007050 A1


### Non-patent literature cited in the description


- *ACS Chem Neurosci.*, 2013, vol. 4 (2), 214-224